# FASCICULE DE BREVET EUROPEEN

(11) **EP 1 845 978 B1**
(45) Date de publication et mention de la délivrance du brevet: **18.08.2010**
(21) Numéro de dépôt: 06709121.5
(22) Date de dépôt: 18.01.2006
(51) Int. Cl.: A61K 31/435, C07D 471/04

(54) **PYRAZOLO PYRIDINES SUBSTITUEES, COMPOSITIONS LES CONTENANT, PROCEDE DE FABRICATION ET UTILISATION**
SUBSTITUIERTE PYRAZOLOPYRIDINE, DIESE ENTHALTENDE ZUSAMMENSETZUNGEN, VERFAHREN ZUR HERSTELLUNG DAVON SOWIE DEREN VERWENDUNG
SUBSTITUTED PYRAZOLO-PYRIDINES, COMPOSITIONS CONTAINING THEM, METHOD FOR THE PRODUCTION THEREOF, AND THEIR USE

(30) Priorité: 19.01.2005 FR 0500555; 13.07.2005 FR 0507505
(43) Date de publication de la demande: 24.10.2007
(73) Titulaire: Aventis Pharma S.A., 92160 Antony (FR)
(72) Inventeur: RONAN, Baptiste, F-92140 Clamart (FR); TABART, Michel, F-91290 La Norville (FR); HALLEY, Frank, F-92370 Chaville (FR); BACQUE, Eric, F-91190 Gif sur Yvette (FR); SOUAILLE, Catherine, F-94600 Choisy Le Roi (FR); UGOLINI, Antonio, F-91300 Massy (FR); VIVIANI, Fabrice, F-95380 Louvres (FR)
(74) Mandataire: Neyret, Daniel Jean Marie
(86) Numéro de dépôt international: PCT/FR2006/000114
(87) Numéro de publication internationale: WO 2006/077319

(56) Documents cités:
- WO-A-02/12242
- WO-A-03/045949
- WO-A-2004/009596
- WO-A-2004/076450
- WO-A-2005/110410
- US-A1- 2004 092 546
- US-B1- 6 562 811

## Description

La présente invention concerne notamment de nouveaux composés chimiques, particulièrement de nouvelles pyrazolo pyridines substituées, les compositions les contenant, et leur utilisation comme médicaments.

Plus particulièrement, et selon un premier aspect, l'invention concerne de nouvelles pyrazolo pyridines spécifiques présentant une activité anticancéreuse, via la modulation de l'activité de protéines, en particulier des kinases.

A ce jour, la plupart des composés commerciaux utilisés en chimiothérapie posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinases sont une famille d'enzyme qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, FAK, KDR et Tie2 sont préférées.

Ces produits répondent à la formule (I) suivante : 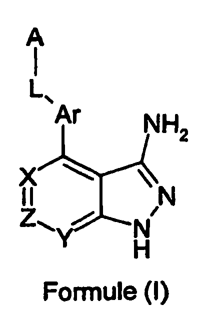 dans laquelle :
3) L'un de X, Y et Z est choisi parmi N et O, et deux autres de Z, Y et X sont C(R5) et C(R6); 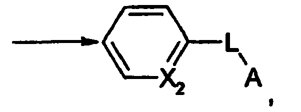
dans lequel X2 est choisi parmi N , C-CH₃, CF et CH.

Des substituants R5 et R6 préférés sont indépendamment sélectionnés parmi H, halogène, OMe et méthyle.

R5 et R6 sont avantageusement choisis parmi H et F.

R5 et R6 sont préférentiellement H.

Des substituants L-A préférés sont avantageusement choisis parmi NH-CO-NH-A et NH-SO₂-A.

Une combinaison L-A particulièrement efficace est obtenue lorsque L-A est NHCONH-A.

A est choisi parmi phényle, pyrazolyle et isoxazolyle ; éventuellement substitué.

Le substituant A est très avantageusement substitué par un substituant sélectionné dans un premier groupe constitué par (C1-C6)alkyle, (C1-C6)alkyle halogéné, (C2-C6)alkylène, (C2-C6)alkynyte, aryle, halogène, hétéroaryle, O-(C1-C6)alkyle, O-Aryle, O-hétéroaryle, S-(C1-C6)alkyle, S-Aryle, S-hétéroaryle, chacun étant éventuellement substitué par un ou plusieurs substituants choisis parmi (C1-C3)alkyle, halogène, O-(C1-C3)alkyle.

Le substituant A est préférentiellement substitué par un substituant sélectionné dans un deuxième groupe constitué par F, Cl, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9) ; dans lequel R8 et R9 sont indépendamment choisis parmi H, (C1-C3)alkyle, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 0 à 3 hétéroatomes choisis parmi O, N et S ; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Des substituants A particulièrement préférés sont choisis parmi phényle et isoxazolyle ; lesdits substituants A pouvant être substitués par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné. Lorsque A est disubstitué, les deux substituants de A peuvent former un cycle de 5 à 7 chaînons contenant de 0 à 3 hétéroatomes.

A est avantageusement substitué par un ou plusieurs substituants, identiques ou différents, indépendemment sélectionnés dans le groupe constitué par F, Cl, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9), (C1-C6)alkyle, (C2-C6)alkylène, (C2-C6)alkynyle, aryle, hétéroaryle, O-(C1-C6)alkyle, O-Aryle, O-hétéroaryle, S-(C1-C6)alkyle, S-Aryle, S-hétéroaryle, chacun étant éventuellement substitué par un ou plusieurs substituants choisis parmi (C1-C3)alkyle, halogène, O-(C1-C3)alkyle; dans lequel R8 et R9 sont indépendamment choisis parmi H, (C1-C3)alkyle, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Selon un mode de réalisation préféré, A est avantageusement 2-fluoro-5-trifluorométhyl-phényle ou 2-méthoxy-5-trifluorométhyl-phényle.

Des produits conforme à l'invention peuvent être choisis parmi :
1-[4-(3-Amino-1H-pyrazolo[3,4-b]pyridin-4-yl)-phényl]-3-(2-fluoro-5-trifluorométhyl-phényl)-urée,
1-[5-(3-Amino-1H-pyrazolo[3,4-b]pyridin-4-yl)-pyridin-2-yl]-3-(2-fluoro-5-trifluorométhyl-phényl)-urée,
1-[4-(3-Amino-1H-pyrazolo[3,4-b]pyridin-4-yl)-phényl]-3-(2-méthoxy-5-trifluorométhyl-phényl)-urée,
1-[5-(3-Amino-1H-pyrazolo[3,4-b]pyridin-4-yl)-pyridin-2-yl]-3-(2-méthoxy-5-trifluorométhyl-phényl)-urée,
1-[4-(3-Amino-1H-pyrazolo[3,4-b]pyridin-4-yl)-phényl]-3-(2-méthylcarbonylamino-5-trifluorométhyl-phényl)-urée,
1-[5-(3-Amino-1H-pyrazolo[3,4-b]pyridin-4-yl)-pyridin-2-yl]-3-(2-méthylcarbonylamino-5-trifluorométhyl-phényl)-urée,
1-[4-(3-Amino-1H-pyrazolo[3,4-b]pyridin-4-yl)-phényl]-3-(2-éthoxy-5-trifluorométhyl-phényl)-urée,
1-[4-(3-Amino-1H-pyrazolo[3,4-b]pyridin-4-yl)-phényl]-3-(2-fluoro-5-méthyl-phényl)-urée,
Acide 3-{3-[4-(3-amino-1*H*-pyrazolo[3,4-b]pyridin-4-yl)-phényl]-uréido}-4-méthoxy-benzoique,
1-[4-(3-Amino-1H-pyrazolo[3,4-b]pyridin-4-yl)-phényl]-3-(2-hydroxy-5-trifluorométhyl-phényl)-urée,
1-[4-(3-Amino-1H-pyrazolo[3,4-b]pyridin-4-yi)-phényl]-3-(3,4-diméthyl-phényl)-urée,
1-[4-(3-Amino-1H-pyrazolo[3,4-b]pyridin-4-yl)-phényl]-3-(2-méthoxy-5-terbutyl-phényl)-urée,
1-[4-(3-Amino-1H-pyrazolo[3,4-b]pyridin-4-yl)-phényl]-3-(3-trifluorométhyl-4-méthyl-phényl)-urée,
1-[4-(3-Amino-1H-pyrazolo[3,4-b]pyridin-4-yl)-phényl]-3-(3-chloro-4-méthyl-phényl)-urée,
1-[4-(3-Amino-1H-pyrazolo[3,4-b]pyridin-4-yl)-phényl]-3-(3-éthyl-phényl)-urée, 1-[4-(3-Amino-1H-pyrazolo[3,4-b]pyridin-4-yl)-phényil-3-1,3-benzodioxol-5-yl-urée,
1-[4-(3-Amino-1H-pyrazolo[3,4-b]pyridin-4-yl)-phényl]-3-(3-chloro-4-méthoxy-phényl)-urée,
1-[4-(3-Amino-1H-pyrazolo[3,4-b]pyridin-4-yl)-phényl]-3-(2-méthoxy-5-chloro-phényl)-urée,
1-[4-(3-Amino-1H-pyrazolo[3,4-b]pyridin-4-yl)-phényl]-3-(2-éthoxy-phényl)-urée,
1-[4-(3-Amino-1H-pyrazolo[3,4-b]pyridin-4-yl)-phényl]-3-(2-méthoxy-5-méthyl-phényl)-urée,
N-[4-(3-Amino-1H-pyrazolo[3,4-b]pyridin-4-yl)-phenyl]-2,3-dichloro-benzenesulfonamide.

D'autres produits conforme à l'invention peuvent être choisis parmi :
1-[4-(3-Amino-1H-pyrazolo[4,3-c]pyridin-4-yl)-phényl]-3-(2-fluoro-5-trifluorométhyl-phényl)-urée,
1-[5-(3-Amino-1H-pyrazolo[4,3-c]pyridin-4-yl)-pyridin-2-yl]-3-(2-fluoro-5-trifluorométhyl-phényl)-urée,
1-[4-(3-Amino-1H-pyrazolo[4,3-c]pyridin-4-yl)-phényl]-3-(phényl)-urée,
1-[5-(3-Amino-1H-pyrazolo[4,3-c]pyridin-4-yl)-pyridin-2-yl]-3-(2-méthoxy-5-trifluorométhyl-phényl)-urée,
1-[4-(3-Amino-1H-pyrazolo[4,3-c]pyridin-4-yl)-phényl]-3-(2-méthoxy-5-trifluorométhyl-phényl)-urée,
1-[4-(3-Amino-1H-pyrazolo[4,3-c]pyridin-4-yl)-phényl]-3-(2-méthylcarbonylamino-5-trifluorométhyl-phényl)-urée,
1-[4-(3-Amino-1H-pyrazolo[4,3-c]pyridin-4-yl)-phényl]-3-(2-fluoro-5-méthyl-phényl)-urée,
1-[4-(3-Amino-1H-pyrazolo[4,3-c]pyridin-4-yl)-phényo-3-(2,4-diméthoxyphényl)-urée,
N-[4-(3-Amino-1H-pyrazolo[4,3-c]pyridin-4-yl)-phényl]-2,3-dichloro-benzenesulfonamide,
1-[4-(3-Amino-1H-pyrazolo[4,3-c]pyridin-4-yl)-phényl]-3-(2-méthoxy-5-terbutyl-phényl)-urée,
1-[4-(3-Amino-1H-pyrazolo[4,3-c]pyridin-4-yl)-phényl]-3-(3,4-diméthyl-phényl)-urée,
1-[4-(3-Amino-1H-pyrazolo[4,3-c]pyridin-4-yl)-phényl]-3-(3-trifluorométhyl-4-méthyl-phényl)-urée,
1-[4-(3-Amino-1H-pyrazolo[4,3-c]pyridin-4-yl)-phényl]-3-(2,5-difluoro-phényl)-urée,
1-[4-(3-Amino-1H-pyrazolo[4,3-c]pyridin-4-yl)-phényl]-3-(3-chloro-4-méthyl-phény)-urée,
1-[4-(3-Amino-1H-pyrazolo[4,3-c]pyridin-4-yl)-phényl]-3-(2-méthyl-5-fluoro-phényl)-urée,
1-[4-(3-Amino-1H-pyrazolo[4,3-c]pyridin-4-yl)-phényl]-3-(2,4-diméthoxy-5-chloro-phényl)-urée,
1-[4-(3-Amino-1H-pyrazolo[4,3-c]pyridin-4-yl)-phényl]-3-(3-trifluorométhylsulfanyl-phényl)-urée.

D'autres produits conforme à l'invention peuvent être choisis parmi :
1-[4-(3-Amino-1H-pyrazolo[3,4-c]pyridin-4-yl)-phényl]-3-(2-fluoro-5-trifluorométhyl-phényl)-urée,
1-[4-(3-Amino-1H-pyrazolo[3,4-c]pyridin-4-yl)-phényl]-3-(2-méthoxy-5-trifluorométhyl-phényl)-urée,
N-[4-(3-Amino-1H-pyrazolo[3,4-c]pyridin-4-yl)-phényl]-2,3-dichloro-benzenesulfonamide.

Un produit conforme à l'invention pourra se présenter sous forme :
non chirale, ou
racémique, ou
enrichie en un stéréoisomère, ou
enrichie en un énantiomère ;
pourra être éventuellement salifié, être éventuellement hydraté, et être éventuellement solvaté.

Un produit conforme à l'invention pourra être utilisé pour la fabrication d'un médicament utile pour traiter un état pathologique, en particulier un cancer.

Selon un second aspect, l'invention concerne un médicament, comprenant un produit de formule (I) selon son premier aspect, ou un sel d'addition de ce composé à un acide pharmaceutiquement acceptable, ou encore un hydrate ou un solvate du produit de formule (I).

Selon un troisième aspect, invention concerne une composition pharmaceutique comprenant un produit selon son premier ou son second aspect, en combinaison avec un excipient pharmaceutiquement acceptable.

Selon un quatrième aspect, l'invention concerne l'utilisation d'un produit selon l'un des autres aspects de l'invention, comme agent inhibiteur d'une réaction catalysée par une kinase. Parmi les kinases, FAK, KDR et Tie2 sont préférées.

La présente invention concerne aussi les compositions thérapeutiques comprenant un produit selon l'invention, en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intra-péritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Les composés de la présente invention peuvent être administrés seuls ou en mélange avec d'autres anticancéreux. Parmi les associations possibles on peut citer:
les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
les dérivés du platine comme notamment le cisplatine, le carboplatine ou l'oxaliplatine
les agents antibiotiques comme notamment la bléomycine, la mitomycine, la dactinomycine
les agents antimicrotubules comme notamment la vinblastine, la vincristine, la vindésine, la vinorelbine, les taxoïdes (paclitaxel et docétaxel)
les anthracyclines comme notamment la doxorubicine, la daunorubicine, l'idarubicine, l'épirubicine, la mitoxantrone, la losoxantrone
les inhibiteurs de topoisomérases des groupes I est II telles que l'étoposide, le teniposide, l'amsacrine, l'irinotecan, le topotecan et le tomudex
les fluoropyrimidines telles que la 5-fluorouracile, l'UFT, la floxuridine
les analogues de cytidine telles que la 5-azacytidine, la cytarabine, la gemcitabine, la 6-mercaptomurine, la 6-thloguanine les analogues d'adénosine tels que la pentostatine, la cytarabine ou le phosphate de fludarabine
le méthotrexate et l'acide folinique
les enzymes et composés divers tels que la L-asparaginase, l'hydroxyurée, l'acide trans-rétinoique, la suramine, la dexrazoxane, l'amifostine, l'herceptine ainsi que les hormones oestrogéniques, androgéniques
les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Les produits de l'invention sont utiles comme agents inhibiteurs d'une réaction catalysée par une kinase. FAK, KDR et Tie2 sont des kinases pour lesquelles les produits de l'invention seront particulièrement utiles en tant qu'inhibiteurs.

Les raisons pour lesquelles ces kinases sont choisies sont données ci-après :

### FAK

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. II a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol-3-OH kinase (P13-kinase) se lie aussi à FAK sur la tyrosine 397 et cette interaction pourrait être nécessaire à l'activation de P13-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].
Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaires *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucleotides anti-sens de FAK perdent leur adhésion et entrent en apoptose (Xu et al, Cell Growth Differ. 4:413-418. 1996). II a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

### KDR

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.
En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 161-1169] et l'antagoniste (angiopoïetine 2 ou Ang2) [P.C. Maisonpierre et al. (1997) Science 277, 55-60]. L'angiopoïetine 1 peut synergiser avec le VEGF dans les derniers stades de la néo-angiogénèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (9996) Cell 87, 1171-1180]. La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]. Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénographes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénérescence maculaire, l'arthrite rhumatoïde, l'hémoangiome infantile et les cancers).

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 1-éthylbutyle, 2-éthylbutyle, 3,3-diméthylbutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-l-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle; napht-2-yle ; anthracen-9-yl ; 1,2,3,4-tétrahydronapht 5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle ; adamantyle; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituant différent de H, par exemple halogène; alkyle; aryle; hétéroaryle, cycloalkyle ; hétérocyclyle; alkylène; alkynyle; OH ; O-alkyle; O-alkylène ; O-aryle; O-hétéroaryle; NH₂ ; NH-alkyle; NH-aryle; NH-hétéroaryle; N-alkyle-alkyle' ; SH ; S-alkyle; S-aryle; S(O₂)H ; S(O₂)-alkyle; S(O₂)-aryle; SO₃H ; SO₃-alkyle; SO₃-aryle; CHO ; C(O)-alkyle; C(O)-aryle ; C(O)OH ; C(O)O-alkyle; C(O)O-aryle ; OC(O)-alkyle; OC(O)-aryle ; C(O)NH₂; C(O)NH-alkyle; C(O)NH-aryle ; NHCHO ; NHC(O)-alkyle; NHC(O)-aryle ; NH-cycloalkyle ; NH-hétérocyclyle, CONH-Hétérocyclyle, CO-Hétéroaryte, CO-Hétérocyclyle NHCO-Héteroaryle, NHCO-Hétérocyclyle, NHCONH-alkyle.

La présente invention a encore pour objet le procédé de préparation des produits de formule (I).
Les produits selon l'invention peuvent être préparés à partir de méthodes conventionnelles de chimie organique. Le schéma 1 ci-dessous est illustratif de la méthode utilisée pour la préparation de l'exemple 1 concernant les pyrazolo[3,4-b]pyridines. A ce titre, elle ne saurait constituer une limitation de la portée de l'invention, en ce qui concerne les méthodes de préparation des composés revendiqués.

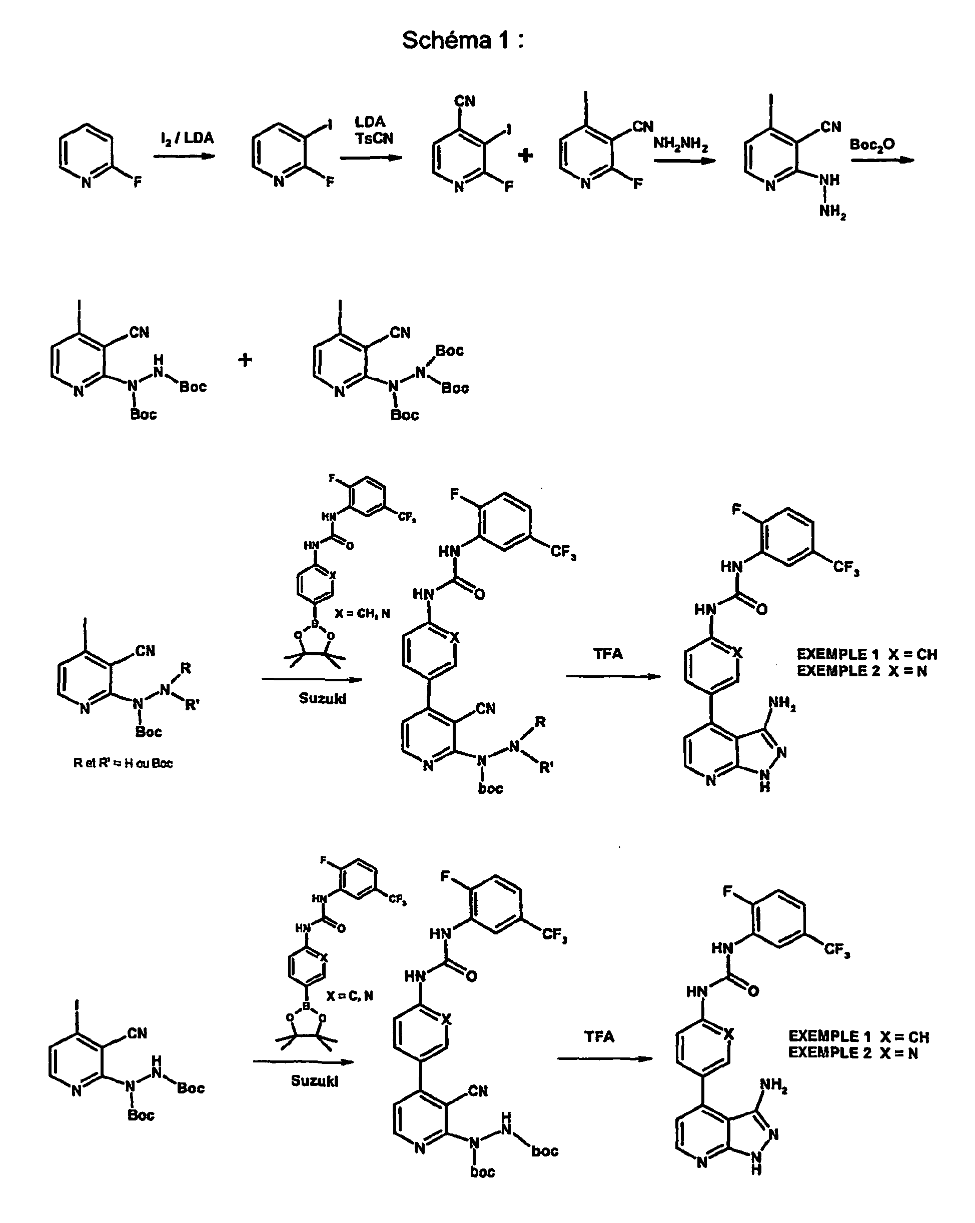

Le schéma 2 ci-dessous est illustratif de la méthode utilisée pour la préparation des exemples concernant les pyrazolo[4,3-c]pyridines. A ce titre, elle ne saurait constituer une limitation de la portée de l'invention, en ce qui concerne les méthodes de préparation des composés revendiqués.

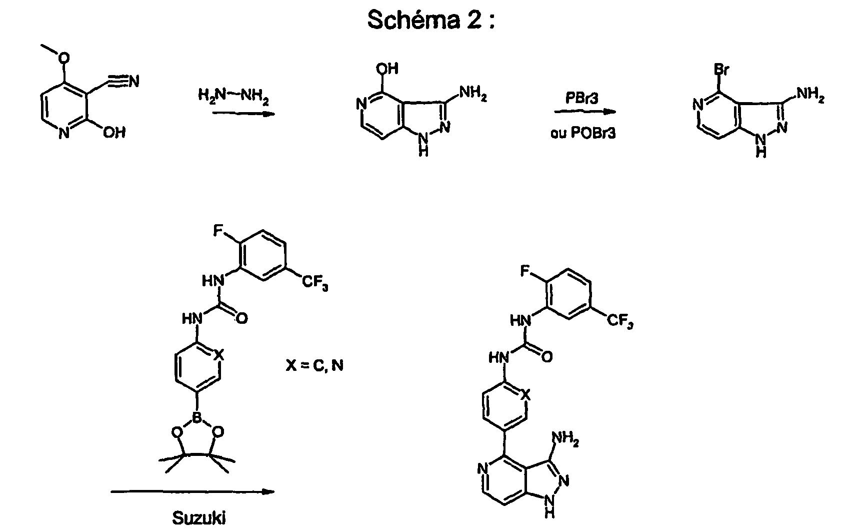

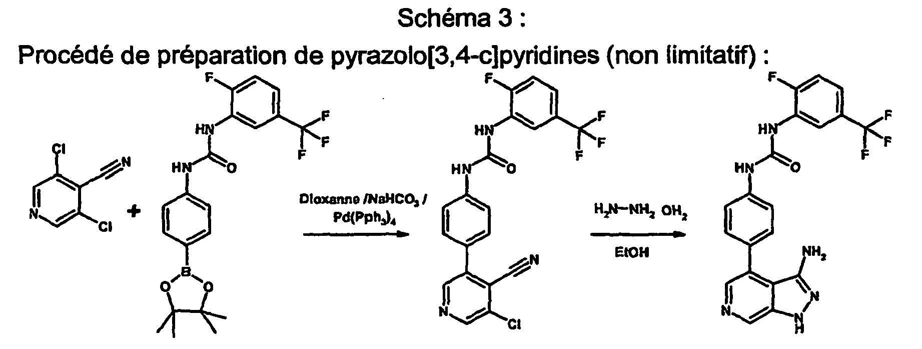

Le schéma 4 ci-dessous est illustratif de la méthode utilisée pour la préparation des exemples concernant les chaînes de dérivés boronates. 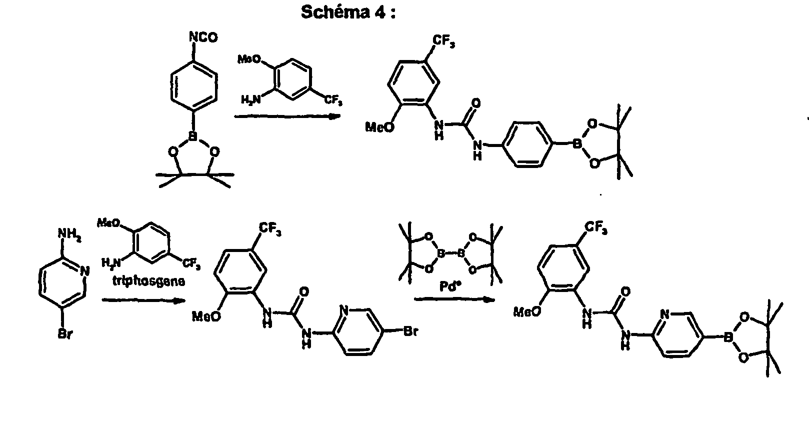 Le schéma 5 ci-dessous est illustratif de la méthode utilisée pour la préparation du 4-iodo-1H-pyrazolo[3,4-b]pyridin-3-ylamine.

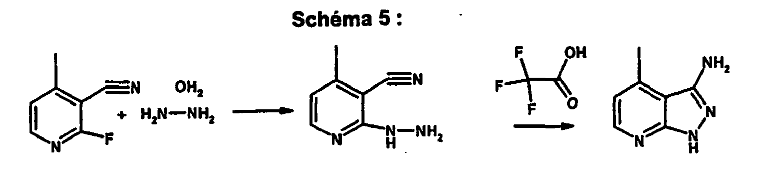

Le schéma 6 ci-dessous est illustratif de la méthode utilisée pour la préparation de dérivés urées du 4-bromo-1H-pyrazolo[4,3-c]pyridin-3-ylamine.

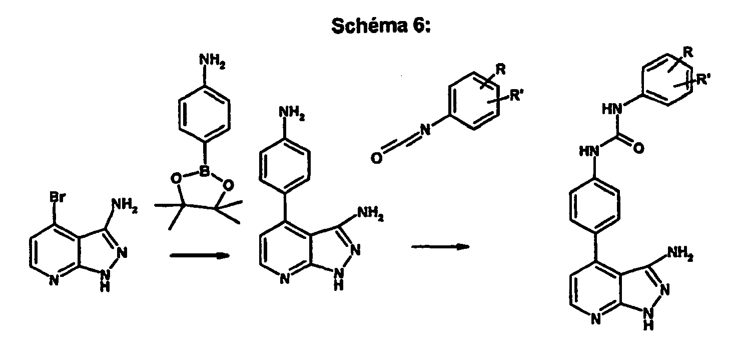

Le schéma 7 ci-dessous est illustratif de la méthode alternative utilisée pour la préparation des exemples concernant les pyrazolo[4,3-c]pyridines

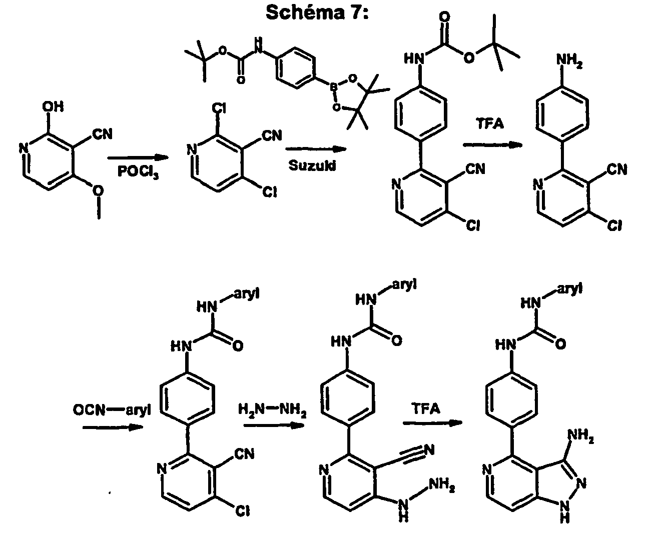

Le schéma 8 ci-dessous est illustratif de la méthode altrenative utilisée pour la préparation de pyrazolo[3,4-c]pyridines.

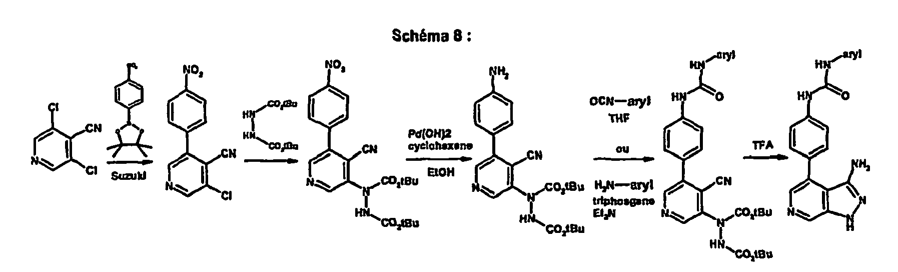

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires. Ces groupes sont ceux qui permettent d'être éliminés sans toucher au reste de la molécule. Comme exemples de groupes protecteurs de la fonction amino, on peut citer le carbamate de *tert*-butyle qui peut être régénéré au moyen d'acide trifluoroacétique ou d'iodotriméthylsilane, l'acétyle qui peut être régénéré en milieu acide (acide chlorhydrique par exemple). Comme groupes protecteurs de la fonction carboxyle, on peut citer les esters (méthoxyméthylester, benzylester par exemple). Comme groupes protecteurs de la fonction alcool, on peut citer les esters (benzoylester par exemple) qui peuvent être régénérés en milieu acide ou par hydrogénation catalytique. D'autres groupes protecteurs utilisables sont décrits par T. W. GREENE et coll. dans Protective Groups in Organic Synthesis, third edition, 1999, Wiley-Interscience.
Un autre objet de la présente invention se rapporte aux composés de formule générale (II) : 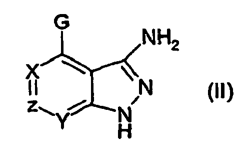 dans laquelle X, Y, Z sont tels que définis précédemment et G est un atome d'halogène approprié dans la réaction de couplage de Suzuki, à tire de produits intermédiaires pour la préparation des produits de formule générale (I) tels que définis à la revendication 1.

Un autre objet de la présente invention se rapporte aux composés de formule générale (III) : 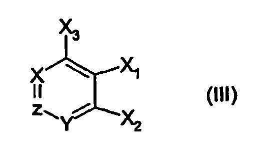 dans laquelle :
X, Y, Z sont tels que définis précédemment,
X₃ est Ar-L-A où Ar, L et A sont tels que définis précédemment, ou Ar-L où Ar est tel que défini précédemment et L est NH₂ ou NO₂,
X₁ et X₂ sont différents et indépendemment choisis parmi CN, Cl, -NH-NH₂, - N(Boc)-NH(Boc), -N(Boc)-N(Boc)₂, à titre de produits intermédiaires pour la préparation des produits de formule générale (I) tels que définis à la revendication 1.

Les composés de formule (I) sont isolés et peuvent être purifiés par les méthodes connues habituelles, par exemple par cristallisation, chromatographie ou extraction.
Les énantiomères, diastéréoisomères des composés de formule (I) font également partie de l'invention.
Les composés de formule (I) comportant un reste basique peuvent être éventuellement transformés en sels d'addition avec un acide minéral ou organique, par action d'un tel acide au sein d'un solvant, par exemple organique tel un alcool, une cétone, un éther ou un solvant chloré.
Les composés de formule (I) comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles.
Ces sels font également partie de l'invention.
Lorsqu'un produit selon l'invention présente au moins une fonction basique libre, des sels pharmaceutiquement acceptables peuvent être préparés par réaction entre ledit produit et un acide minéral ou organique. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, p-toluène sulfonate, propanesulfonates, xylènesulfonates, salicylates, cinnamates, glutamates, aspartates, glucuronates, galacturonates.
Lorsqu'un produit selon l'invention présente au moins une fonction acide libre, des sels pharmaceutiquement acceptables peuvent être préparés par réaction entre ledit produit et une base minérale ou organique. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, histidine, pipéridine, morpholine, pipérazine, triéthylamine.

L'invention est également décrite par les exemples suivants, donnés à titre d'illustration de l'invention.

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 mn.
Les spectres MS ont été réalisés en électrospray (ES⁺) sur un appareil Platform II (Micromass). Les principaux ions observés sont décrits.
Les points de fusion ont été mesurés en capillaire, sur un appareil Mettler FP62, gamme 30°C à 300°C, montée de 2°C par minute.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était controlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitriie 95/5 (v/v) contenant 0,07% (v/v) d'acide trifluoroacétique, pendant que l'autre colonne était en cours de séparation. L'élution des colonnes a été effectuée en utilisant un gradient linéaire de 5 à 95% d'acétonitrile contenant 0,07% (v/v) d'acide trifluoroacétique dans l'eau contenant 0,07% (v/v) d'acide trifluoroacétique, à un débit de 10 mUmn. A la sortie de la colonne de séparation, un millième de l'effluent est séparé par un LC Packing Accurate, dilué à l'alcool méthylique à un débit de 0,5 mUmn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

### Exemple 1

### 1-[4-(3-Amino-1H-pyrazolo[3,4-b]pyridin-4-yl)-phényl]-3-(2-fluoro-5-trifluorométhyl-phényl)-urée

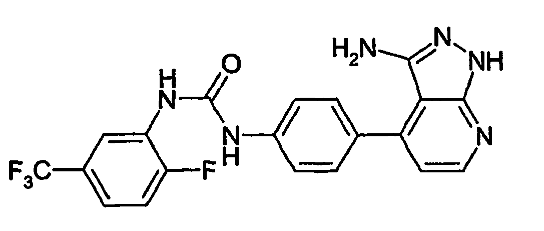

### 2-fluom-3-iodo-pyridine

A une solution de 0,103mol de LDA dans 200mL de THF à -75°C sont ajoutés 10g de 2-fluoropyridine dans 50mL de THF. La solution jaune est agitée pendant 3 heures à -75°C, puis sont ajoutés 26,2g d'iode dans 80mL de THF. Le mélange réactionnel est agité pendant 1,5 heures à -75°C, puis sont ajoutés 50mL d'eau à cette température. On laisse remonter la température, puis à 0°C sont ajoutés 100mL d'eau supplémentaire. La suspension est décolorée par ajout de thiosulfate de sodium. Le mélange est extrait avec du diéthyléther. La phase organique est séchée sur sulfate de magnésium puis concentrée sous pression réduite. Le résidu huileux est purifié sur silice Si60 (40-63µm) (cyclohexane / diéthyléther, 95:5). Le produit obtenu est repris dans le diéthyléther et un insoluble beige est éliminé par essorage. Après séchage sous vide, 10,21 g de poudre blanche de 2-fluoro-3-iodopyridine sont obtenus.
Spectre MS (ES+) : m/z= 224 [MH+]

### 2-Fluoro-4-iodo-nicotinonitrile

A une solution de 21,5mmol de LDA dans 20mL de THF à -75°C sont ajoutés 4,8g de 2-fluoro-3-iodopyridine dans 20mL de THF. La solution jaune est agitée pendant 1h10 à -75°C, puis, à cette température sont ajoutés 3,9g de cyanure de para-toluène sulfonyle dans 20mL de THF. Le mélange réactionnel est agité pendant 2 heures à -75°C, puis sont ajoutés 20mL d'eau à cette température. On laisse remonter la température, puis à 0°C sont ajoutés 40mL d'eau supplémentaire. Le mélange est extrait par le diéthyléther. La phase organique est séchée sur sulfate de magnésium puis concentrée sous pression réduite. La résine marron obtenu est purifié sur silice Si60 (40.63µm) (cyclohexane / diéthyléther, 8:2). On obtient une fraction de 667mg d'une poudre jaune de 2-Fluoro-4-iodo-nicotinonitrile et une deuxième fraction de 2,1 g d'une poudre jaune pâle de 2-Fluoro-3-iodo-isonicotinonitrile.
Spectre MS (ES+) : m/z= 249 [MH+]

### 2-Hydrazino-4-iodo-nicotinonitrile

A une solution de 1,2g de 2-fluoro-4-iodo-nicotinonitrile dans 20mL de MeOH à 20°C sont ajoutés 2,4mL d'hydrate d'hydrazine. La suspension jaune est agitée 20min à 20°C puis est filtrée. Le précipité est lavé au méthanol pour donner après séchage sous vide 947mg de poudre blanche de 2-Hydrazino-4-iodo-nicotinonitrile.
Spectre MS (ES+) : m/z= 261 [MH+]

### (3-Cyano-4-iodo-pyridin-2-yl)-hydrazine dicarboxylate de di-tert-butyle et N,N',N'-(3-Cyano-4-iodo-pyridin-2-yl)-hydrazine tricarboxylate de tri-tert-butyle

A un mélange de 234mg de 2-Hydrazino-4-iodo-nicotinonitrile, 27,5mg de N,N-diméthyaminopyridine et 0,316mL de triéthylamine dans 11mL de dichlorométhane à 4°C sont ajoutés 492mg de dicarbonate de di-terbutyle dans 5mL de dichlorométhane. La réaction est agitée 30min à 4°C. puis on laisse remonter la température à 20°C et on agite pendant 5h. De l'eau est ajoutée et le mélange est extrait avec de l'acétate d'éthyle. La phase organique est séchée sur sulfate de magnésium puis concentrée sous pression réduite. Le résidu huileux jaune est purifié sur colonne Biotage KP-Sil de 60Å SiO₂ 32-63µm pré-remplie (éluant : dichlorométhane / méthanol, 99,5:0,5 puis 99:1) donnant 2 fractions majoritaires, une première fraction de 110mg d'une poudre jaune de N,N',N'-(3-Cyano-4-iodo-pyridin-2-yl)-hydrazine-tricarboxylate de tri-tert-butyle (Spectre MS (ES+): m/z= 561 [MH+]) et une deuxième fraction de 202mg d'une huile jaune pâle contenant du (3-Cyano-4-iodo-pyridin-2-yl)-hydrazine-dicarboxylate de di-tert-butyle. Cette huile jaune est re-purifié sur colonne Biotage KP-Sil de 60Å SiO₂ 32-63µm pré-remplie (éluant : cyclohexane / acétate d'éthyle, 95:5 puis 9:1) donnant 114mg d'une poudre beige de (3-Cyano-4-iodo-pyridin-2-yl)-hydrazine-dicarboxylate de di-tert-butyle, Spectre MS (ES+) : m/z= 461 [MH+]

### N,N',N'-(3-cyano-4-iodo-pyridin-2-yl)-hydrazine-tricarboxylate de tri-tert-butyle

A un mélange de 925mg de 2-Hydrazino-4-iodo-nicotinonitrile, 109mg de N,N-diméthyaminopyridine et 2,6mL de triéthylamine dans 45mL de dichlorométhane à 4°C sont ajoutés 3,9g de di-terbutyldicarbonate (Boc₂O) dans 20mL de dichlorométhane. La réaction est agitée 30min à 4°C puis on laisse remonter la température à 20°C. De l'eau est ajoutée et le mélange extrait avec de l'acétate d'éthyle puis séché sur sulfate de magnésium et concentré sous pression réduite. Le résidu huileux brun est purifié sur silice Si60 (40-63µm) (dichlorométhane / méthanol, 98 :2) pour obtenir 1,2g d'une poudre jaune de N,N',N'-(3-Cyano-4-iodo-pyridin-2-yl)-hydrazine-tricarboxylate de tri-tert-butyle.
PF = 138°C (Köfler).
Spectre MS (ES+) : m/z= 561 [MH+]

### Couplage de Suzuki (X = C) avec un dérivé di-boc

A une solution de 110mg de (3-cyano-4-iodo-pyridin-2-yl)-hydrazine dicarboxylate de di-tert-butyle et de 122mg de 1-(2-Fluoro-5-trifluorométhyl-phényl)-3-[4-(4,4,5,5-tétraméthyl-1,3,2-dioxaborolan-2-yl)-phényl]-urée dans 5,5mL de dioxane sont ajoutés 57mg de NaHCO₃ dans 1,7mL d'eau. Puis, 26mg de tétrakis(triphénylphosphine)palladium sont ajoutés et la réaction est chauffée au reflux à 100°C. Après 2,5h la solution jaune-clair est refroidie à 20°C et 10mL d'acétate d'éthyle sont ajoutés. La phase organique est lavée 2 fois avec 8mL d'eau puis 8mL de saumure. Après séchage sur sulfate de magnésium et concentration sous pression réduite, l'huile jaune résiduelle est purifiée sur colonne Biotage KP-Sil de 60Å SiO₂ 32-63µm pré-remplie (cyclohexane / acétate d'éthyle, 9:1). On obtient 114mg de (3-Cyano-4-{4-[3-(2-fluoro-5-trifluorométhyl-phényl)-uréido]-phényl}-pyridin-2-yl)-hydrazine dicarboxylate de di-tert-butyle.
Spectre MS (ES+) : m/z= 631 [MH+]

### Couplage de Suzuki (X = C) avec un dérivé tri-boc

A une solution de 108mg de N,N',N'-(3-cyano-4-iodo-pyridin-2-yl)-hydrazine-tricarboxylate de tri-tert-butyle et de 98mg de 1-(2-Fluoro-5-tilfluorométhyl-phényl)-3-[4-(4,4,5,5-tétraméthyl-1,3,2-dioxaborolan-2-yl)-phényl]-urée dans 4,5mL de dioxane sont ajoutés 45,4mg de NaHCO₃ dans 1,4mL d'eau. Puis, 20,3mg de tétrakis(triphénylphosphine)palladium sont ajoutés et la réaction est chauffée au reflux à 100°C. Après 2,5h la solution jaune-clair est refroidie à 20°C et 10mL d'acétate d'éthyle sont ajoutés. La phase organique est lavée 2 fois avec 8mL d'eau puis 8mL de saumure. Après séchage sur sulfate de magnésium et concentration sous pression réduite, l'huile jaune résiduelle est purifiée sur colonne Biotage KP-Sil de 60Å SiO₂ 32-63µm pré-remplie (cyclohexane / acétate d'éthyle, 9:1 puis 7:3). On obtient une fraction de 86mg de N,N',N'-(3-Cyano-4-{4-[3-(2-fluoro-5-trifluorométhyl-phényl)-uréido]-phényl}-pyridin-2-yl)-hydrazinetricarboxylate de tri-tert-butyle et une fraction de 31 mg de (3-Cyano-4-{4-[3-(2-fluoro-5-trifluorométhyl-phényl)-uréido]-phényl}-pyridin-2-yl)-hydrazinedicarboxylate de di-tert-butyle.
Spectre MS (ES+) : m/z= 731 [MH+]

### 1-[4-(3-Amino-1H-pyrazolo[3,4-b]pyridin-4-yl)-phényl]-3-(2-fluoro-5-trifluorométhyl-phényl)-urée (à partir du dérivé diboqué)

A une solution de 111mg de (3-Cyano-4-{4-[3-(2-fluoro-5-trifluorométhyl-phényl)-uréido]-phényl}-pyridin-2-yl)-hydrazinedicarboxylate de di-tert-butyle dans 4mL de dichlorométhane à 20°C sont ajoutés 0,3mL d'acide trifluoroacétique contenant 10% d'anisole et la réaction est agitée 4h. Le milieu réactionnel est concentré sous pression réduite donnant un solide rouge-orangé. Ce résidu est purifié sur colonne Biotage KP-Sil de 60Å SiO₂ 32.63µm pré-remplie (gradient dichlorométhane / solution A, 95:5 à 90:10; solution A = dichlorométhane / méthanol / ammoniaque, 38:17:2). On obtient 56mg d'une poudre beige de 1-[4-(3-Amino-1H-pyrazolo[3,4-b]pyridin-4-yl)-phényl]-3-(2-fluoro-5-trifluorométhyl-phényl)-urée, dont les caractéristiques sont les suivantes :
Spectre IR (KBr): 3370; 3300; 1717; 1604; 1541; 1443; 1317; 1310; 1205; 1184; 1122; 1114; 1069 et 818 cm⁻¹
Spectre de R.M.N. ¹H (300 MHz, (CD₃)₂SO d6, δ en ppm) : 4,58 (s large, 2H) ; 6,91 (d, J = 5,0 Hz, 1H) ; 7,41 (dm, J = 9,0 Hz, 1H) ; 7,52 (t large, J = 9,0 Hz, 1H) ; 7,56 (d large, J = 8,5 Hz, 2H) ; 7,68 (d large, J = 8,5 Hz, 2H) ; 8,37 (d, J = 5,0 Hz, 1H) ; 8,62 (dd large, J = 2,5 et 7,5 Hz, 1H) ; 9,13 (m étalé, 1H) ; 9,57 (m étalé, 1H) ; 12,25 (s large, 1H)
PF =175°C déc. (Köfler).

De façon analogue, le 1-[4-(3-Amino-1H-pyrazolo[3,4-b]pyridin-4-yl)-phényl]-3-(2-fluoro-5-trifluorométhyl-phényl)-urée peut être obtenu à partir d'un dérivé triboqué comme suit :
A une solution de 81mg de dérivé N,N',N'-(3-cyano-4-{4-[3-(2-fluoro-5-trifluorométhyl-phényl)-uréido]-phényl}-pyridin-2-yl)-hydrazinetricarboxylate de tri-tert-butyle dans 3mL de dichlorométhane à 20°C sont ajoutés 0,2mL d'acide trifluoroacétique contenant 10% d'anisole et la réaction est agitée une nuit. Le milieu réactionnel est concentré sous pression réduite donnant un solide rouge-orangé. Ce résidu est purifié sur colonne Biotage KP-Sil de 60Å SiO₂ 32-63µm pré-remplie (gradient dichlorométhane / solution A, 95:5 puis 90:10; solution A = dichlorométhane / méthanol / ammoniaque, 38:17:2). On obtient 27mg d'une poudre beige de 1-[4-(3-amino-1H-pyrazolo[3,4-b]pyridin-4-yl)-phényl]-3-(2-fluoro-5-trifluorométhyl-phényl)-urée

### Exemple 2

### 1-[5-(3-Amino-1H-pyrazolo[3,4-b]pyridin-4-yl)-pyridin-2-yl]-3-(2-fluoro-5-trifluorométhyl-phényl)-urée

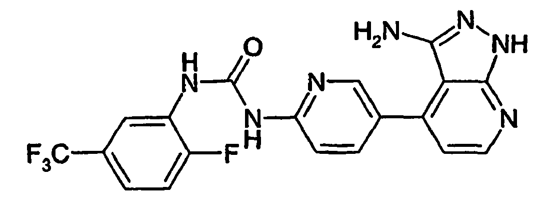

### Couplage de Suzuki (X = N)

A une solution de 150mg de N,N',N'-(3-cyano-4-iodo-pyridin-2-yl)-hydrazine-tricarboxylate de tri-tert-butyle et de 136mg de 1-(2-Fluoro-5-trifluorométhyl-phényl)-3-[5-(4,4,5,5-tétraméthyl-1,3,2-dioxaborolan-2-yl)-pyridin-2-yl]-urée dans 6,2mL de dioxane sont ajoutés 63mg de NaHCO₃ dans 1,9mL d'eau. Puis, 29mg de tétrakis(triphénylphosphine)palladium sont ajoutés et la réaction est chauffée au reflux à 100°C. Après 2,5h la solution jaune est refroidie à 20°C et 10mL d'acétate d'éthyle sont ajoutés. La phase organique est lavée 2 fois avec 8mL d'eau puis 8mL de saumure. Après séchage sur sulfate de magnésium et concentration sous pression réduite l'huile jaune résiduelle est purifiée sur colonne Biotage KP-Sil de 60Å SiO₂ 32-63µm pré-remplie (gradient dichlorométhane / solution A, 98:2 à 95:5 ; solution A = dichlorométhane / méthanol / ammoniaque, 38 :17 :2). On obtient 67mg de N,N',N'-{3'-cyano-6-[3-(2-fluoro-5-trifluorométhyl-phényl)-uréido]-[3,4']bipyridinyl-2'-yl}-hydrazine tricarboxylate de tri-tert-butyle (Spectre MS (ES+) : m/z= 732 [MH+] ) et 55mg de {3'-cyano-6-[3-(2-fluoro-5-trifluorométhyl-phényl}-uréido]-[3,4']bipyridinyl-2'-yl}-hydrazine dicarboxylate de di-tert-butyle (Spectre MS (ES+) : m/z= 632 [MH+] ).

### 1-[5-(3-Amino-1H-pyrazolo[3,4-b]pyridin-4-yl)-pyridin-2-yl]-3-(2-fluoro-5-trifluorométhyl-phényl)-urée

A une solution de 55mg de {3'-cyano-6-[3-(2-fluoro-5-trifluorométhyl-phényl)-uréido]-[3,4']bipyridinyl-2'-yl}-hydrazine dicarboxylate de di-tert-butyle dans 2mL de dichlorométhane à 20°C sont ajoutés 0,15mL d'acide trifluoroacétique contenant 10% d'anisole et la réaction est agitée 2h.

Séparément, à une solution de 67mg de dérivé N,N',N'-{3'-cyano-6-[3-(2-fluoro-5-trifluorométhyl-phényl)-uréido]-[3,4']bipyridinyl-2'-yl}-hydrazine tricarboxylate de tri-tert-butyle dans 3mL de dichlorométhane à 20°C sont ajoutés 0,16mL d'acide trifluoroacétique contenant 10% d'anisole et la réaction est agitée 2h.

Les milieux réactionnels rouge-brun sont réunis et concentrés sous pression réduite donnant un solide rouge-orangé. Ce résidu est purifié sur colonne Biotage KP-Sil de 60Å SiO₂ 32-63µm pré-remplie (gradient dichlorométhane / solution A, 95:5 à 90:10 puis 70:30; solution A = dichlorométhane / méthanol / ammoniaque, 38 :17 :2). On obtient un produit que l'on reprend dans de l'acétate d'éthyle et qu'on lave à l'eau pour éliminer du trifluoroacétate d'ammonium. Après séchage sur sulfate de magnésium et concentration sous pression réduite, on obtient 58mg d'une poudre jaune beige de 1-[5-(3-amino-1H-pyrazolo[3,4-b]pyridin-4-yl)-pyridin-2-yl]-3-(2-fluoro-5-trifluorométhyl-phényl)-urée, dont les caractéristiques sont les suivantes :
Spectre IR (KBr) : 3209; 1708; 1610; 1571; 1441; 1376; 1302; 1250; 1168; 1119; 1071; 822 et 616 cm⁻¹
Spectre de R.M.N. ¹H (300 MHz, (CD₃)₂SO d6, δ en ppm) : 4,70 (s large, 2H) ; 7,00 (d, J = 5,0 Hz, 1H) ; 7,45 (dm, J = 9,0 Hz, 1H) ; 7,55 (t large, J = 9,0 Hz, 1H) ; 7,65 (d large, J = 8,5 Hz, 1H) ; 8,06 (dd, J = 2,5 et 8,5 Hz, 1H) ; 8,41 (d, J = 5,0 Hz, 1H) ; 8,54 (d, J = 2,5 Hz, 1H) ; 8,67 (dd, J = 2,5 et 7,5 Hz, 1H) ; 10,15 (s large, 1H) ; 11,15 (m très étalé, 1H) ; 12,35 (m étalé, 1H) PF = >260°C (Köfler).

### Exemple 3

### 1-[4-(3-Amino-1H-pyrazolo[4,3-c]pyridin-4-yl)-phényl]-3-(2-fluoro-5-trifluorométhyl-phényl)-urée

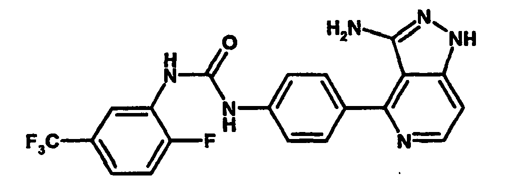

### 3-Amino-1H-pyrazolo[4,3-c]pyridin-4-ol

Un mélange de 1,2g de 2-hydroxy-4-méthoxy-nicotinonitrile et de 30mL d'hydrate d'hydrazine est porté à 140°C pendant 18h dans une bombe. Le milieu réactionnel est ensuite évaporé à sec. Le résidu est repris dans l'éther éthylique, et la suspension résultante est filtrée. On obtient 1,1g de 3-amino-1H-pyrazolo[4,3-c]pyridin-4-ol sous l'aspect d'un solide gris.

SM : 150⁺ = M⁺°; PF = 240°C déc. (Köfler).

### 4-Bromo-1H-pyrazolo[4,3-c]pyridin-3-ylamine

A 3,5g de POBr₃ fondu à 45°C, on ajoute 0,4g de 3-amino-1H-pyrazolo[4,3-c]pyridin-4-ol, puis la suspension est portée à 70°C pendant 4h. On laisse refroidir et on hydrolyse, avec précaution, avec une solution de bicarbonate de sodium. Le mélange est extrait avec de l'acétate d'éthyle. La phase organique est isolée et séchée sur sulfate de magnésium, filtrée, puis concentrée sous pression réduite. On obtient 0,33g de 4-bromo-1H-pyrazolo[4,3-c]pyridin-3-ylamine sous l'aspect d'un solide crème.

SM : 212⁺ = M⁺° ; PF = 230°C déc. (Köfler).

### 1-[4-(3-Amino-1H-pyrazolo[4,3-c]pyridin-4-yl)-phényl]-3-(2-fluoro-5-trifluorométhyl-phényle)-urée

A une solution de 53mg de 4-bromo-1H-pyrazolo[4,3-c]pyridin-3-ylamine dans 6mL de dioxane, on ajoute 127mg de 1-(2-fluoro-5-trifluorométhyl-phényl)-3-[4-(4,4,5,5-tétraméthyl-1,3,2-dioxaborolan-2-yl)-phényl]-urée, 60mg de bicarbonate de sodium dans 1,8mL d'eau et 30mg de tétrakis(triphénylphosphine)palladium. La réaction est chauffée dans un bain à 100°C pendant 2h. On laisse refroidir puis on ajoute de l'acétate d'éthyle. Le mélange est lavé avec de l'eau. La phase organique est séchée sur sulfate de magnésium, puis concentrée sous pression réduite. Le résidu huileux est purifié sur colonne Biotage KP-Sil de 60Å SiO₂ 32-63µm pré-remplie (gradient dichlorométhane / solution A, 90:10 à 70:30; solution A = dichlorométhane / méthanol / ammoniaque, 38:17:2). On obtient 34mg de 1-[4-(3-amino-1H-pyrazolo[4,3-c]pyridin-4-yl)-phényle]-3-(2-fluoro-5-trifluorométhyl-phényl)-urée solide de couleur crème, dont les caractéristiques sont les suivantes :
SM : 431⁺ = MH⁺
Spectre IR (KBr) : 3354; 1717; 1605; 1542; 1442; 1339; 1313; 1181; 1119; 816 et 615 cm⁻¹
Spectre de R.M.N. ¹H (300 MHz, (CD₃)₂SO d6, δ en ppm) : 4,72 (s large, 2H) ; 7,20 (d, J = 5,5 Hz, 1H) ; 7,41 (m large, 1H) ; 7,51 (t large, J = 9,5 Hz, 1H) ; de 7,59 à 7,72 (m, 4H) ; 8,22 (d, J = 5,5 Hz, 1H) ; 8,65 (d large, J = 7,5 Hz, 1H) ; 8,96 (s large, 1H) ; 9,37 (s, 1H) ; 12,1 (s large, 1H).

### Exemple 4

### 1-[5-(3-Amino-1H-pyrazolo[4,3-c]pyridin-4--yl)-pyridin-2-yl]-3-(2-fluoro-5-trifluorométhyl-phényl)-urée

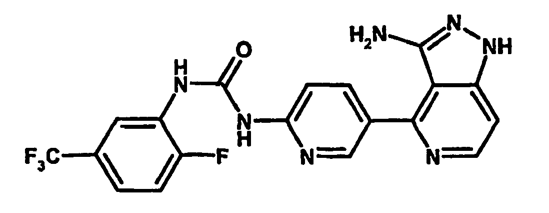

### 1-[5-(3-Amino-1h-pyrazolo[4,3-c]pyridin-4-yl)-pyridin-2-yl]-3-(2-fluoro-5-trifluorométhyl-phényl)-urée

A une solution de 106mg de 4-bromo-1H-pyrazolo[4,3-c]pyridin-3-ylamine dans 10mL de dioxane, on ajoute 252mg de 1-(2-fluoro-5-trifluorométhylphényl)-3-[5-(4,4,5,5-tétraméthyl-1,3,2-dioxaborolan-2-yl)-pyridin-2-yl]-urée, 120mg de bicarbonate de sodium dans 3mL d'eau, et 60mg de tétrakis(triphénylphosphine)palladium. La réaction est chauffée dans un bain à 100°C pendant 2h. On laisse refroidir puis on ajoute de l'acétate d'éthyle et le mélange est lavé avec de l'eau. On sèche la phase organique sur sulfate de magnésium, puis on concentre sous pression réduite. Le résidu huileux est purifié sur colonne Biotage KP-Sil de 60Å SiO₂ 32-63µ pré-remplie (gradient dichlorométhane / solution A, 90:10 à 70:30; solution A = dichlorométhane / méthanol / ammoniaque, 38:17:2). On obtient 35mg d'un solide jaune de 1-[5-(3-amino-1H-pyrazolo[4,3-c]pyridin-4-yl)-pyridin-2-yl]-3-(2-fluoro-5-trifluorométhyl-phényl)-urée, dont les caractéristiques sont les suivantes :
SM : 432⁺ = MH⁺
Spectre IR (KBr): 3404; 3224; 1693; 1609; 1572; 1441; 1340; 1300; 1246; 1119; 819 et 615 cm⁻¹
Spectre de R.M.N. ¹H (300 MHz, (CD₃)₂SO d6, δ en ppm) : 4,88 (s large, 2H) ; 7,25 (d, J = 6,0 Hz, 1H) ; 7,44 (m large, 1H) ; de 7,50 à 7,53 (m, 2H) ; 8,15 (dd, J = 2,5 et 8,5 Hz, 1H) ; 8,26 (d, J = 6,0 Hz, 1H) ; 8,63 (d, J = 2,5 Hz, 1H) ; 8,70 (d large, J = 7,5 Hz, 1H) ; 10,15 (s large, 1H) ; 11,3 (m très étalé, 1H) ; 12,2 (s large, 1H).

### Exemple 5

### 1-[4-(3-Amino-1h-pyrazolo[3,4-b]pyridin-4-yl)-phényl]-3-(2-méthoxy-5-trifluorométhyl-phényl)-urée

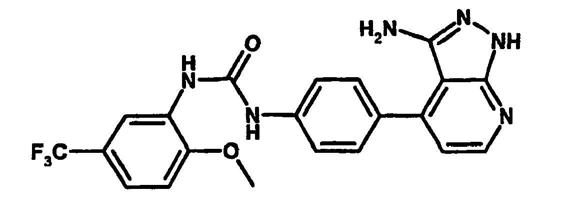

### 1-(2-Méthoxy-4-trifluorométhyl-phényl)-3-[4-(4,4,5,5-tétraméthyl-1,3,2-dioxaborolan-2-yl)-phényl]-urée

A une solution de 1g de 2-méthoxy-4-(trifluorométhyl)aniline et de 128mg de 4-diméthylaminopyridine dans 150mL de tétrahydrofurane et 1,5mL de triéthylamine est ajouté, a 20°C, 1,28g de 2-(4-isocyanato-phényl)-4,4,5,5-tétraméthyl-1,3,2-dioxaborolane. Apres 4h, la réaction est évaporée à sec sous pression réduite. Le résidu est repris par un mélange d'acétate d'éthyle et d'eau. La phase organique est séchée sur sulfate de magnésium, puis concentrée sous pression réduite. l'huile jaune résiduelle est purifiée sur colonne biotage kp-sil de 60å sio₂ 32-63µm pré-remplie (éluant : cyclohexane / acétate d'éthyle, 8: 2). On obtient une poudre jaune pale qui est reprise dans du méthanol. II se forme un insoluble blanc qui est séparé par filtration, puis le filtrat est évaporé à sec sous pression réduite pour donner 0,47g de poudre jaune pale de 1-(2-méthoxy-4-tifluorométhyl-phényl)-3-[4-(4,4,5,5-tétraméthyl-1,3,2-dioxaborolan-2-yl)-phényl]-urée.

SM : 437⁺ = MH⁺

Spectre de R.M.N. ¹H (300 MHz, (CD₃)₂SO d6, δ en ppm): 1,30 (s : 12H) ; 3,99 (s : 3H) ; 7,22 (d, J = 9 Hz : 1H) ; 7,34 (dd, J = 9 et 1 Hz : 1H) ; 7,50 (d , J = 9 : 2H) ; 7,63 (d , J = 9 Hz : 2H) ; 8,56 (mt : 2H) ; 9,58 (s : 1H).

### Dérivés di-boc et tri-boc du 1-[4-(3-cyano-2-hydrazino-pyridin-4-yl)-phényl]-3-(2-méthoxy-5-triftuorométhyl-phényl)-urée

A une solution de 0,27g de N, N', N'-(3-cyano-4-iodo-pyridin-2-yl)-hydrazine-tricarboxylate de tri-tert-butyle dans 14mL de dioxane, à 20°C, sont ajoutés 273mg de 1-(2-méthoxy-4-trifluorométhyl-phényl)-3-[4-(4,4,5,5-tétraméthyl-1,3,2-dioxaborolan-2-yl)-phényl]-urée. A cette solution, on ajoute une solution de 114mg de bicarbonate de sodium dans 4mL d'eau, puis 51 mg de tétrakis(triphénylphosphine)palladium. La réaction est chauffée au reflux à 100°C pendant 2,5h. La solution jaune-clair est refroidie, puis on ajoute de l'acétate d'éthyle. Le mélange est lavé avec de l'eau puis de la saumure. La phase organique est séchée sur sulfate de magnésium, puis concentrée sous pression réduite. La résine jaune orange obtenue est purifiée sur colonne biotage kp-sil de 60å SiO₂ 32-63µm pré-remplie (éluant : cyclohexane / acétate d'éthyle, 7: 3, puis dichlorométhane / solution a, 9:1; solution a = dichlorométhane / méthanol / ammoniaque, 38:17:2). On obtient 0,33g de poudre jaune d'un mélange de dérivés di-boc et tri-boc du 1-[4-(3-cyano-2-hydrazino-pyridin-4-yl)-phényl]-3-(2-méthoxy-5-trifluorométhyl-phényl)-urée.

### 1-[4-(3-Amino-1h-pyrazolo[3.4-b]pyridin-4-yl)-phényl]-3-(2-méthoxy-5-trifluorométhyl-phényl)-urée

A une solution de 272mg d'un mélange de dérivés di-boc et tri-boc du 1-[4-(3-cyano-2-hydrazino-pyridin-4-yl)-phényl]-3-(2-méthoxy-5-trifluorométhyl-phényl)-urée dans 7,7mL de dichlorométhane à 20°C, on ajoute 0,62mL d'acide trifluoroacétique contenant 10% d'anisole. Après 3h d'agitation, le milieu rouge-orange est concentré à sec sous pression réduite. L'huile rouge-orange résiduelle est purifiée sur colonne Biotage KP-Sil de 60Å SiO₂ 32-63µm pré-remplie (gradient dichlorométhane / solution A, 90:10 à 70:30; solution A = dichlorométhane / méthanol / ammoniaque, 38:17:2). On obtient une poudre jaune beige qui est reprise dans l'acétate d'éthyle. La solution est lavée à l'eau. La phase organique est séchée sur sulfate de magnésium, puis concentrée sous pression réduite pour donner 61 mg d'une poudre jaune de 1-[4-(3-amino-1H-pyrazolo[3,4-b]pyridin-4-yl)-phényl]-3-(2-méthoxy-5-trifluorométhyl-phényl)-urée, dont les caractéristiques sont les suivantes :
SM : 443⁺ = MH⁺
Spectre IR (KBr): 3380; 1706; 1675; 1600; 1539; 1314; 1269; 1134; 1117; 822 et 622 cm⁻¹
Spectre de R.M.N. ¹H (400 MHz, (CD₃)₂SO d6, δ en ppm) : 3,99 (s, 3H) ; 4,59 (s large, 2H) ; 6,91 (d, J = 5,0 Hz, 1H) ; 7,22 (d, J = 8,5 Hz, 1H) ; 7,34 (dd large, J = 2,0 et 8,5 Hz, 1H) ; 7,55 (d large, J = 8,5 Hz, 2H) ; 7,66 (d large, J = 8,5 Hz, 2H) ; 8,37 (d, J = 5,0 Hz, 1H) ; de 8,56 à 8,61 (m, 2H) ; 9,66 (s, 1H) ; 12,25 (s large, 1H).
PF = >260°C. (Köfler).

### Exemple 6

### 1-[5-(3-Amino-1h-pyrazolo[3,4-b]pyridin-4-yl)-pyridin-2-yl]-3-(2-méthoxy-5-trifluorométhyl-phényl)-urée

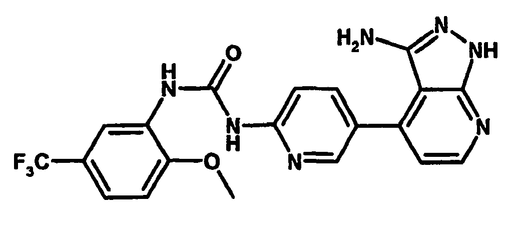

### 1-(5-Bromo-pyridin-2-yl)-3-(2-méthoxy-5-trifluorométhyl-phényl)-urée

3,32g de 2-méthoxy-5-trifluorométhyl-phenylamine dans 30mL de tétrahydrofurane sont ajoutés goutte-à-goutte à une solution de 1,8g de triphosgène dans 180mL de tétrahydrofurane a 0°C. 4,95mL de triéthylamine sont ensuite introduits et la réaction est agitée à cette température pendant 10min. On laisse remonter la température à 20°C et on agite pendant 1h15. On introduit ensuite goutte-à-goutte une solution de 3g de 2-amino-5-bromopyridine dans 30mL de THF. Apres 16h, on filtre la suspension, puis le filtrat est concentré sous pression réduite. Le résidu est repris dans un mélange d'acétate d'éthyle et d'eau. On obtient un précipité insoluble blanc qui est filtré, lavé à l'acétate d'éthyle puis séché sous vide. On obtient 3,3g de poudre blanche de 1-(5-bromo-pyridin-2-yl)-3-(2-méthoxy-5-trifluorométhyl-phényl)-urée.

SM : 390⁺ = MH⁺

### 1-(2-Méthoxy-5-trifluorométhyl-phényl)-3-[5-(4,4,5,5-tétraméthyl-1,3,2-dioxaborolan-2-yl)-pyridin-2-yl]-urée

A une solution de 51 mg de tricyclohexylphosphine dans 12mL de dioxane à 20°C sont ajoutés 30mg de bis(dibenzylidèneacétone)palladium. La solution brun-violette est agitée pendant 30min, puis, on ajoute 500mg de 1-(5-bromo-pyridin-2-yl)-3-(2-méthoxy-5-trifluorométhyl-phényl)-urée et 190mg d'acétate de potassium. La suspension est chauffée au reflux pendant 3h15 puis la solution rouge-violette est refroidie à 20°C et concentrée à sec sous pression réduite. On obtient un résidu gris-vert qui est lavé dans un mélange d'acétate d'éthyle et d'eau. La phase organique est filtrée, et le filtrat jaune est concentré sous pression réduite. Le résidu solide obtenu est repris dans du méthanol. Il se forme un précipité qui est filtré et lavé par du méthanol et de l'acétate d'éthyle. On obtient 244mg de poudre jaune pale de 1-(2-méthoxy-5-trifluorométhyl-phényl)-3-[5-(4,4,5,5-tétraméthyl-1,3,2-dioxaborolan-2-yl)-pyridin-2-yl]-urée.

SM : 437⁺ = M⁺°

Spectre de R.M.N. ¹H (300 MHz, (CD₃)₂SO d6, δ en ppm): 1,32 (s : 12H) ; 4,03 (s : 3H) ; 7,25 (d, J = 9,5 Hz : 1H); 7,34 (d large, J = 9 Hz : 1H) ; 7,39 (dd, J = 9,5 et 1 Hz : 1H); 7,96 (dd, J = 9 et 2 Hz : 1H); 8,52 (d, J = 1Hz : 1H) ; 8,63 (d, J = 2 Hz : 1H) ; 10,20 (s : 1H) ; 11,50 (s large : 1H).

### Derivés di-boc et tri-boc de la 1-(3'-cyano-2'-hydrazino-[3,4']bipyridinyl-6-yl)-3-(2-méthoxy-5-trifluorométhyl-phényl)-uréee

234mg de 1-(2-Méthoxy-5-trifluorométhyl-phényl)-3-[5-(4,4,5,5-tétraméthyl-1,3,2-dioxaborolan-2-yl)-pyridin-2-yl]-urée sont ajoutés à 20°C à une solution de 0,3g de N, N', N'-(3-cyano-4-iodo-pyridin-2-yl)-hydrazine-tricarboxylate de tri-tert-butyle dans 16,5mL de dioxane. On ajoute ensuite une solution de 126mg de bicarbonate de sodium dans 4,5mL d'eau, suivi de 57mg de tétrakis(triphénylphosphine)palladium. La suspension jaune obtenue est chauffée au reflux a 100°C pendant 2,5h. Après refroidissement, on ajoute de l'acétate d'éthyle. La phase organique est lavée avec de l'eau puis de la saumure. La phase organique est séchée sur sulfate de magnésium puis concentrée à sec sous pression réduite. La résine jaune-orange résiduelle est purifiée sur colonne biotage kp-sil de 60å SiO₂ 32-63µm pré-remplie (éluant : cyclohexane / acétate d'éthyle, 7 : 3, puis dichlorométhane / solution A, 95:5; solution A = dichlorométhane / méthanol / ammoniaque, 38:17:2). On obtient 265mg d'un mélange de_dérivés di-boc et tri-boc du 1-(3'-cyano-2'-hydrazino-[3,4']bipyridinyl-6-yl)-3-(2-méthoxy-5-trifluorométhyl-phényl)-urée sous forme d'une poudre jaune.

### 1-[5-(3-Amino-1h-pyrazolo[3,4-b]pyridin-4-yl)-pyridin-2-yl]-3-(2-méthoxy-5-trifluorométhyl-phényl)-urée

0,8mL d'acide trifluoroacétique contenant 10% d'anisole sont ajoutés à 20°C à une solution de 242mg d'un mélange de dérivés di-boc et tri-boc du 1-(3'-cyano-2'-hydrazino-[3,4']bipyridinyl-6-yl)-3-(2-méthoxy-5-trifluorométhyl-phényl)-urée dans 7mL de dichlorométhane. Après 2,5h d'agitation, le milieu réactionnel rouge-orange est concentré à sec sous pression réduite. L'huile rouge-orange résiduelle est purifiée sur colonne Biotage KP-Sil de 60Å SiO₂ 32-63µm pré-remplie (gradient dichlorométhane / solution A, 90:10 à 70:30; solution A = dichlorométhane / méthanol / ammoniaque, 38:17:2). On obtient 130mg d'une poudre jaune beige de 1-[5-(3-amino-1H-pyrazolo[3,4-b]pyridin-4-yl)-pyridin-2-yl]-3-(2-méthoxy-5-trifluorométhyl-phényl)-urée, dont les caractéristiques sont les suivantes :
SM : 444⁺ = MH⁺
Spectre IR (KBr): 3420; 3219; 1684; 1613; 1586; 1438; 1303; 1271; 1247; 1136; 834 et 622 cm⁻¹
Spectre de R.M.N. ¹H (300 MHz, (CD₃)₂SO d6, δ en ppm) : 4,02 (s, 3H); 4,72 (m très étalé, 2H) ; 7,01 (d, J = 5,0 Hz, 1H) ; 7,24 (d, J = 8,5 Hz, 1H) ; 7,37 (dd large, J = 2,0 et 8,5 Hz, 1H) ; 7,48 (d large, J = 8,5 Hz, 1H) ; 8,03 (dd, J = 2,5 et 8,5 Hz, 1H) ; 8,41 (d, J = 5,0 Hz, 1H) ; 8,59 (d, J = 2,5 Hz, 1H) ; 8,64 (d, J = 2,0 Hz, 1H) ; 10,2 (s, 1H) ; 11,5 (m très étalé, 1H) ; 12,35 (s large, 1H).
PF = >260°C. (Köfler).

### Exemple 7

### 1-[4-(3-Amino-1H-pyrazolo[3,4-b]pyridin-4-yl)-phényle]-3-(2-fluoro-5-trifluorométhyl-phényl)-urée

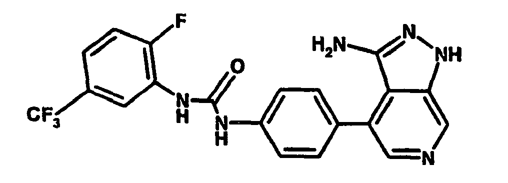

### 1-[4-(5-chloro-4-cyano-pyridin-3-yl)-phényle]-3-(2-fluoro-5-trifluorométhyl-phényl)-urée :

A une solution de 346mg de 3,5-dichloro-isonicotinonitrile et de 959mg de 1-(2-fluoro-5-trifluorométhyl-phényl)-3-[4-(4,4,5,5-tetraméthyl-1,3,2-dioxaborolan-2-yl)-phényl]-urée dans 24mL de dioxane sont ajoutés 462mg de NaHCO₃ dans 14,4mL d'eau. Puis, 462mg de tértakis(triphénylphosphine)palladium sont ajoutés et la réaction est chauffée au reflux 5h puis laissée une nuit à 20°C. Ensuite, 20mL d'eau sont ajoutés et le mélange extrait avec 2X60mL d'acétate d'éthyle. La phase organique est séchée sur sulfate de magnésium puis concentrée sous pression réduite. L'huile marron est purifiée sur une cartouche Merck de 30g de SiO₂ 15-40µm pré-remplie (dichlorométhane puis dichlorométhane / méthanol, 99:1). On obtient 290mg de cristaux jaunes de 1-[4-(5-chloro-4-cyano-pyridin-3-yl)-phényl]-3-(2-fluoro-5-trifluorométhyl-phényl)-urée.
Spectre MS (ES⁺) : m/z = 435 [MH⁺]
Spectre RMN 1H à 400 MHz sur spectromètre BRUKER AVANCE DRX-400 déplacements chimiques (□ en ppm) - solvant : (DMSO-d6) référencé à 2,50 ppm à la température de 303K :
   7,41 (m, 1H) ; 7,51 (dd, J = 8,5 et 10,5 Hz, 1H) ; 7,68 (s large, 4H) ; 8,62 (dd, J = 2,0 et 7,0 Hz, 1H) ; 8,85 (s, 1H) ; 8,94 (s, 1H) ; 9,04 (m étalé, 1H) ; 9,49 (m étalé, 1H).

### 1-[4-(3-Amino-1H-pyrazolo[3,4-c]pyridin-4-yl)-phényl]-3-(2-fluoro-5-trifluorométhyl-phényl)-urée

A une solution de 145mg de 1-[4-(5-chloro-4-cyano-pyridin-3-yl)-phényl]-3-(2-fluoro-5-trifluorométhyl-phényl)-urée dans 2mL de EtOH à 20°C sont ajoutés 0,05mL d'hydrate d'hydrazine. La réaction est portée au reflux pendant 5h30. La réaction est incomplète et on rajoute 1 mL d'EtOH et 0,05mL d'hydrate d'hydrazine et le reflux maintenu 18h30 supplémentaires. On laisse revenir à 20°C et on concentre sous courant d'azote. Le résidu est purifié par chromatographie sur gel de silice : cartouche Merck de 30g de SiO₂ 15-40µm pré-remplie (dichlorométhane ; puis dichlorométhane / méthanol, 95:5 ; puis dichlorométhane / méthanol, 1:1 ; puis dichlorométhane / méthanol / NH₄OH, 82:15:3). On récupère une huile beige impure que l'on chromatographie à nouveau dans les mêmes conditions. Le produit est toujours impur et il est purifié par HPLC preparative basic : conditions : colonne: Nucleodur Gravity C18 5µm (N° cat Macherey Nagel: 762101 ; série 4055902; batch 3044) ; débit : 20mUmin ; détection 254nm (UV118, Gilson) ; gradient de 10 à 95% d'acétonitrile dans de l'eau contenant du formiate d'ammonium à 10mM selon le protocole suivant : (t (min.) : acétonitrile (%)) : 0 : 10 ; 2 : 10 ; 25 : 95 ; 33 : 95 ; 34 : 10 . On obtient 19mg d'une poudre jaune de 1-[4-(3-Amino-1H-pyrazolo[3,4-c]pyridin-4-yl)-phényl]-3-(2-fluoro-5-trifluorométhyl-phényl)-urée.

PF = 196°C (Koffler)

Spectre MS (ES⁺) : m/z = 431 [MH⁺]

Spectre RMN 1H à 400 MHz sur spectromètre BRUKER AVANCE DRX-400, déplacements chimiques (□ en ppm) - solvant (DMSO-d6) référencé à 2,50 ppm à 303K : 4,61 (s, 2H); 7,40 (m, 1H) ; 7,50 (m, 3H) ; 7,66 (d large, J = 8,5 Hz, 2H) ; 7,94 (s, 1H) ; 8,63 (dd, J = 2,0 et 7,0 Hz, 1H) ; 8,74 (s, 1H) ; 9,10 (m étalé, 1H) ; 9,51 (s, 1H) ; 12,3 (m étalé, 1H).

IR : (KBr): 1610; 1531; 1443; 1340; 1314; 1265; 1195; 1166; 1118; 1069; 820 & 615 cm⁻¹

### Exemple 8

### 1-[4-(3-Amino-1H-pyrazolo[4,3-c]pyridin-4-yl]-phényl]-3-phényl-urée Préparé selon le schéma 2

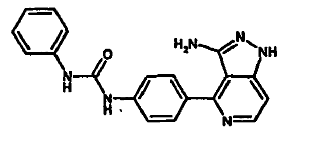

### 1-Phényl-3-[4-(4,4,5,5-tetraméthyl-1,3,2-dioxaborolan-2-yl)-phényl]-urée

A une solution de 490 mg de 2-(4-isocyanato-phenyl)-4, 4, 5, 5-tetraméthyl-1,3,2,-dioxaborolane et de 203 mg de triéthylamine dans 5 ml de tétrahydrofurane sont ajoutés 198 mg d'aniline. La solution est agitée 12 heures à 20°C sous argon. 10 ml de méthanol sont additionnés au mélange réactionnel. Puis, on agite à 20°C pendant 15 minutes. Après concentration sous pression réduite du mélange, on obtient 630 mg de 1-Phenyle-3-[4-(4,4,5,5-tétraméthyl-1,3,2-dioxaborolan-2-yl)-phényl]-urée, dont les caractéristiques sont les suivantes :
SM : 339 (+) = (M+H) (+)
   337 (-) = (M-H) (-)
Spectre IR (KBr): 3332; 2976; 1656; 1593; 1543; 1400; 1361; 1143; 1092; 963; 860 & 655 cm⁻¹
Spectre de R.M.N. ¹H (400 MHz, (CD₃)₂SO, δ en ppm) : 1,29 (s,12H) ; 6,98 (t large, J = 7,5 Hz, 1H) ; 7,28 (t large, J = 8,0 Hz, 2H) ; de 7,41 à 7,49 (m, 4H) ; 7,59 (d large, j = 8,0 Hz, 2H) ; 8,70 (s, 1H) ; 8,82 (s,1H).

### 1-[4-(3-Amino-1H-pyrazolo[4,3-c]pyridin-4-yl]-phényl]-3-phényl-urée

A une solution de 65 mg de 4-bromo-1H-pyrazolo[4,3-c]pyridin-3-ylamine, préparé comme décrit dans l'exemple 3, dans 3 ml de dioxane sont ajoutés 155 mg de 1-phényle-3-[4-(4,4,5,5,-tétraméthyl-1,3,2-dioxaborolan-2-yl)-phényl]-urée, 72 mg d'hydrogenocarbonate de sodium dans 1 ml d'eau et 53 mg de tétrakis(triphénylphosphine)palladium. La suspension est chauffée à 100°C sous argon pendant 2H. Après refroidissement, le mélange réactionnel est coulé sur 50 ml d'eau et extrait 3 fois à l'acétate d'éthyle. Les phases organiques réunies sont séchées sur sulfate de magnésium et concentrées sous pression réduite pour donner 302 mg de produit brut qui sont purifés sur colonne Biotage KP-Sil de 60Å SiO₂ 32-63 µM pré-packée ( gradient d'une solution A dans le dichloromethane de 5/95 à 30/70 ; solution A = dichloromethane/methanol/ammoniaque 38:17:2). On obtient 23 mg d'une poudre jaune de 1-[4-(3-amino-1H-pyrazolo[4,3-c]pyridin-4-yl]-phényl]-3-phényl-urée, dont les caractéristiques sont les suivantes :
SM : 344 (+) = M (+) (SM-EI)
Spectre IR (KBr) : 3388; 1672; 1601; 1528; 1498; 1442; 1313 ; 1233 ; 1180 ; 1045 ; 752 & 693 cm⁻¹
Spectre de R.M.N. ¹H (400 MHz, (CD₃)₂SO, δ en ppm) : 4,71(s large, 2H) ; 6,99 (t, J = 7,5 Hz, 1H) ; 7,19 (d, J = 5,0 Hz, 1H) ; 7,30 (t, J = 7,5 Hz, 2H) ; 7,48 (d, J = 7,5 Hz, 2H) ; 7,62 (m, 4H) ; 8,21 (d, J = 5,0 Hz, 1H); 8,73 (s, 1H) ; 8,89 (s, 1H) ; 12,1 (s large, 1H).
PF = 188°C (Koffler)

### Exemple 9

### 1-[5-(3-Amino-1H-pyrazolo[4,3-c]pyridin-4-yl)-pyridin-2-yl]-3-(2-méthoxy-5-trifluorométhyl-phényl)-urée

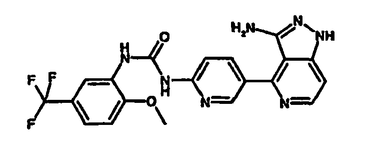

A une solution de 70 mg de 4-bromo-1H-pyrazolo[4,3-c]pyridin-3-ylamine dans 3 ml de dioxane sont ajoutés 215 mg de 1-(2-méthoxy-5-trifluorométhyl-phényl)-3-[5-(4,4,5,5-tétraméthyl-1,3,2-dioxaborolan-2-yl)-pyridin-2-yl]-urée, (préparée comme décrit dans l'exemple 6) 77 mg d'hydrogénocarbonate de sodium dans 1 ml d'eau et 38 mg de tétrakis(triphénylphosphine)palladium. La suspension est chauffée à 80°C sous argon pendant 2h. Après refroidissement, la réaction est coulée sur 50 ml d'eau et le mélange extrait 3 fois avec un mélange acétate d'éthyle/méthanol 90/10. Les phases organiques réunies sont séchées sur sulfate de magnésium et concentrées sous pression réduite. 393 mg de produit brut sont purifiés sur colonne Biotage KP-Sil de 60Å SiO₂ 32-63 δM pré-packée (gradient d'une solution A dans le dichlorométhane de 5/95 à 30/70 ; solution A = dichlorométhane/méthanol/ammoniaque 38/17/2). On obtient 16 mg d'une poudre jaune de 1-[5-(3-amino-1H-pyrazolo[4,3-c]pyridin-4-yl)-pyridin-2-yl]-3-(2-méthoxy-5-trifluorométhyl-phényl)-urée, dont les caractéristiques sont les suivantes :
LC-SM : 444 (+) = (M+H)(+)
Spectre IR (KBr): 3657; 3387; 3308; 3223; 2925; 1685; 1610; 1580; 1439; 1351; 1302; 1246; 1166; 1135; 1116; 1037; 807 & 542 cm⁻¹
Spectre de R.M.N. ¹H (400 MHz, (CD₃)₂SO, δ en ppm) : 4,02 (s, 3H) ; 4,89 (s large, 2H) ; de 7,21 à 7,28 (m, 2H) ; 7,38 (d, J = 8,5 Hz, 1H); 7,46 (d large, J = 8,5 Hz, 1H); 8,12 (dd, J = 2,0 et 8,5 Hz, 1H); 8,27 (d, J = 5,0 Hz, 1H); 8,63 (d, J = 2,0 Hz, 1H); 8,69 (d, J = 2,0 Hz, 1H); 10,15 (s, 1H); 11,65 (m étalé, 1H); 12,2 (s large, 1H).
PF = 228°C (Koffler)

### Exemple 10

### 1-[4-(3-Amino-1H-pyrazolo[4,3-c]pyridin-4-yl)-phényl]-3-(2-méthoxy-5-trifluorométhyl-phényl)-urée

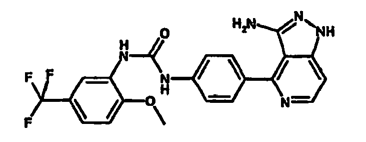

A une solution de 50 mg de 4-bromo-1H-pyrazolo[4,3-c]pyridin-3-ylamine dans 3 ml de dioxane sont ajoutés 122,8 mg de 1-(2-méthoxy-5-trifluorométhyl-phényl)-3-[4-(4,4,5,5,-tétraméthyl-1,3,2-dioxaborolan-2-yl)-phényl]-urée, 99,5 mg de carbonate de sodium dans 1 ml d'eau et 54,24 mg de térakis(triphénylphosphine)palladium. La suspension est chauffée à 80°C sous atmosphère d'argon pendant 22 heures. Après refroidissement le mélange réactionnel est dilué avec de l'acétate d'éthyle et lavé 2 fois à l'eau. La phase organique est séchée sur sulfate de magnésium et concentrée sous pression réduite. 438 mg de produit brut sont purifiés sur colonne Biotage KP-Sil de 60Å SiO₂ 32-63 µM pré-packée (gradient d'une solution A dans le dichlorométhane de 5/95 à 30/70 ; solution A = dichlorométhane / méthanol / ammoniaque 38/17/2). On obtient 32 mg d'une poudre jaune de 1-[4-(3-amino-1H-pyrazolo[4,3-c]pyridin-4-yl)-phényl]-3-(2-méthoxy-5-trifluorométhyl-phényl)-urée, dont les caractéristiques sont les suivantes :
LC-SM : 443 (+) = (M+H) (+)
Spectre IR (KBr): 3326; 1691; 1605; 1542; 1447; 1314; 1270; 1217; 1120; 1024; 812 & 623 cm⁻¹
Spectre R.M.N. ¹H (400 MHz, (CD₃)₂SO, δ en ppm): 3,99 (s, 3H); 4,73 (s large, 2H); 7,19 (d, J = 5,0 Hz, 1H); 7,22 (d, J = 8,5 Hz, 1H); 7,33 (d large, J = 8,5 Hz, 1H); 7,64 (m, 4H); 8,21 (d, J = 5,0 Hz, 1H); 8,59 (m, 2H); 9,63 (s, 1H); 12,1 (s, large, 1H)
PF = 200°C (Koffler)

### Exemple 11

### N-(2-{3-[4-(3-Amino-1H-pyrazolo[4,3-c]pyridin-4-yl)-phényl]-uréido}-4-trifluorométhyl-phényl)-acétamide

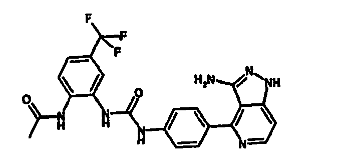

### N-(2-{3[4-(4,4,5,5-Tétraméthyl-1,3,2-dioxaborolan-2-yl)-phényl]-ureido}-4-trifluorométhyl-phényl)-acétamide

### Préparé selon le schéma 4

A une solution de 343mg de N1-[2-amino-4-(trifluorométhyl)phényl]acétamide et de 39mg de 4-diméthylaminopyridine dans 50mL de tétrahydrofurane et 0,45mL de triéthylamine sont ajouté, à 20°C, 385mg de 2-(4-isocyanato-phényl)-4,4,5,5-tétraméthyl-1,3,2-dioxaborolane. Après une nuit à 40°C la réaction est évaporée à sec sous pression réduite. Le résidu est repris par un mélange de dichlorométhane et d'eau. La phase organique est lavée par du HCl 1 N le précipité blanc est essoré et lavé avec du dichlorométhane, puis séché sous vide pour donner 356mg de poudre blanche de N-(2-{3-[4-(4,4,5,5-tétraméthyl-1,3,2-dioxaborolan-2-yl)-phényl]-ureido}-4-trifluorométhyl-phenyl)-acétamide, dont les caractéristiques sont les suivantes :
SM-ES⁺: 464(+)=(M+H)(+)
   SM-ES⁻: 462(-)=(M-H)(-)
Spectre de R.M.N. ¹H (300 MHz, (CD₃)₂SO d6, δ en ppm) : 1,28 (s, 12H) ; 2,15 (s large, 3H) ; 7,37 (d large, J = 8,5 Hz, 1H) ; 7,50 (d, J = 8,5 Hz, 2H) ; 7,54 (d partiellement masqué, J = 8,5 Hz, 1H) ; 7,61 (d, J = 8,5 Hz, 2H) ; 8,16 (s large, 1H) ; 8,28 (s large, 1H) ; 9,43 (s large, 1H) ; 9,79 (s large, 1H).

### N-(2-{3-[4-(3-Amino-1H-pyrazolo[4,3-c]pyridin-4-yl)-phényl]-uréido}-4-trifluorométhyl-phényl)-acetamide

Le 4-bromo-1H-pyrazolo[4,3-c]pyridin-3-ylamine et le N-(2-{3-[4-(4,4,5,5-tétraméthyl-1,3,2-dioxaborolan-2-yl)-phényl]-uréido}-4-trifluorométhyl-phényl)-acetamide, sont couplés par réaction de Suzuki selon un protocole similaire à la préparation de l'exemple 10. On obtient une poudre jaune de N-(2-{3-[4-(3-amino-1H-pyrazolo[4,3-c]pyridin-4-yl)-phényl]-uréido}-4-trifluorométhyl-phényl)-acetamide, dont les caractéristiques sont les suivantes :
SM : 470 (+) = (M+H) (+)
   468 (-) = (M-H) (-)
   514 (-) = (M + acide formique - H) (-)
Spectre IR (KBr): 3390; 1667; 1605; 1528; 1436; 1336; 1315; 1247; 1167; 1126; 1042; 813 & 684 cm⁻¹
Spectre de R.M.N. ¹H (400 MHz, (CD₃)₂SO, δ en ppm) : 2,16 (s, 3H) ; 4,72 (s, 2H) ; 7,19 (d, J = 5,0 Hz, 1H) ; 7,37 (d large, J = 8,5 Hz, 1H); 7,56 (d, J = 8,5 Hz, 1H); 7,64 (m, 4H); 8,21 (d, J = 5,0 Hz, 1H); 8,28 (m, 2H); 9,54 (s large, 1H); 9,82 (s large, 1H); 12,1 (s large, 1H).
PF = 182°C (Koffler)

### Exemple 12

### N-(2-{3-[4-(3-Amino-1H-pyrazolo[3,4-b]pyridin-4-yl)-phényl]-uréido}-4-trifluorométhyl-phényl)-acétamide

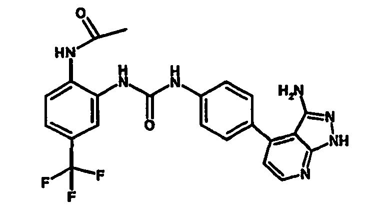

### Dérivé N, N', N' tri-boc du N-(2-{3-[4-(3-Cyano-2-hydrazino-pyridin-4-yl)-phényl]-uréido}-4-trifluorométhyl-phényl)-acétamide

### Préparé selon le schéma 1

A une solution de 278mg de N,N',N'-(3-cyano-4-iodo-pyridin-2-yl)-hydrazine-tricarboxylate de tri-tert-butyle (préparé selon l'exemple 1) dans 15mL de dioxane, à 20°C, sont ajoutés 282mg de N-(2-{3-[4-(4,4,5,5-tétraméthyl-1,3,2-dioxaborolan-2-yl)-phényl]-ureido}4-trifluorométhyl-phényl)-acetamide. A cette solution on ajoute une solution de 117mg de bicarbonate de sodium dans 4,2mL d'eau suivi de 53mg de tétrakis(triphénylphosphine)palladium. La réaction est chauffée au reflux à 100°C pendant 2,5h. La solution jaune-clair est refroidie, puis on ajoute de l'acétate d'éthyle. Le mélange est lavé avec de l'eau puis avec de la saumure. La phase organique est séchée sur sulfate de magnésium, puis concentrée sous pression réduite. La résine jaune orange obtenu est purifié sur colonne Biotage KP-Sil de 60Å SiO₂ 32-63µ pré-packée (éluant : cyclohexane / acétate d'éthyle, 7 : 3, puis dichlorométhane / solution A, 9:1; solution A = dichlorométhane / méthanol / ammoniaque, 38:17:2). On obtient 239mg de poudre jaune d'un mélange de dérivés di-boc et tri-boc du N-(2-{3-[4-(3-cyano-2-hydrazino-pyridin-4-yl)-phényl]-uréido}-4-trifluorométhyl-phényl)-acétamide.

### N-(2-{3-[4-(3-Amino-1H-pyrazolo[3,4-b]pyridin-4-yl)-phényl]-uréido}-4-trifluorométhyl-phényl)-acétamide

### Préparé selon le schéma 1

Les dérivés di-boc et tri-boc du N-(2-{3-[4-(3-cyano-2-hydrazino-pyridin-4-yl)-phényl]-ureido}-4-trifluorométhyl-phényl)-acetamide sont transformés selon la procédure pour la préparation de l'exemple 6. Le produit obtenu est une poudre jaune-clair dont les caractéristiques sont les suivantes :
SM-ES⁺: 470(+)=(M+H)(+)
Spectre IR (KBr) : 3363; 1672; 1596; 1525; 1435; 1336; 1316; 1126; 1078 & 825 cm⁻¹
Spectre de R.M.N. ¹H (400 MHz, (CD₃)₂SO d6, δ en ppm) :
   2,16 (s large, 3H) ; 4,58 (s, 2H) ; 6,91 (d, J = 5,0 Hz, 1H) ; 7,39 (d large, J = 9,0 Hz, 1H) ; de 7,52 à 7,58 (m, 3H) ; 7,67 (d, J = 8,5 Hz, 2H) ; 8,21 (s large, 1H) ; 8,29 (s large, 1H) ; 8,37 (d, J = 5,0 Hz, 1H) ; 9,52 (s large, 1H) ; 9,84 (s large, 1H) ; 12,25 (s large, 1H).

### Exemple 13

### N-(2-{3-[5-(3-Amino-1H-pyrazolo[3,4-b]pyridin-4-yl)-pyridin-2-yl]-uréido}-4-trifluorométhyl-phényl)-acétamide

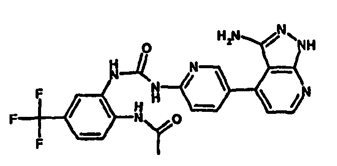

### N-(2-{3-[5-(4,4,5,5-Tetraméthyl-1,3,2-dioxaborolan-2-yl)-pyridin-2-yl]-uréido}-4-trifluorométhyl-phényl)-acétamide

### Préparé selon le schéma 4

A une solution de 10mg de tricyclohexylphosphine dans 2.5mL de dioxane à 20°C sont ajoutés 6mg de bis(dibenzylidèneacétone)palladium. La solution brun-violet est agitée pendant 30min. On ajoute ensuite 100mg de N-{2-[3-(5-bromo-pyridin-2-yl)-uréido]-4-trifluorométhyl-phényl}-acétamide et 36mg d'acétate de potassium et 84mg de bis-(pinacolato)-dibore. La suspension est chauffée au reflux pendant 3h15 puis la suspension est refroidie à 20°C et concentrée à sec sous pression réduite. Le résidu gris-vert est repris dans l'acétate d'éthyle et l'insoluble est éliminé par filtration. Le filtrat jaune est lavé à l'eau puis séché sur sulfate de magnésium et concentrée sous pression réduite. On obtient 88mg de poudre jaune-blanc de N-(2-{3-[5-(4,4,5,5-tétraméthyl-1,3,2-dioxaborolan-2-yl)-pyridin-2-yl]-uréido}-4-trifluorométhyl-phényl)-acétamide, dont les caractéristiques sont les suivantes :
SM-ES⁺: 465(+)=(M+H)(+)

### 4-Iodo-1H-pyrazolo[3,4-b]pyridin-3-ylamine

### Préparé selon le schéma 5

A une solution de 1,145 g de 2-fluoro-4-iodo-nicotinonitrile dans 27 ml d'éthanol est ajouté 2,5 ml d'hydrate d'hydrazine. Dès l'introduction de l'hydrate d'hydrazine, il se forme un précipité blanc. La suspension est agitée à 20°C pendant 20 minutes. Le précipité est filtré et lavé à l'éthanol. On obtient 882 mg de poudre beige de 2-hydrazino-4-iodo-nicotinonitrile. Cet intermédiaire est repris dans 35 ml de dichlorométhane. A cette suspension blanche est ajouté 5,8 ml d'acide trifluoroacétique contenant 10% d'anisole. La solution jaune orangée obtenue est agitée à 20°C pendant 30 minutes. Le mélange réactionnel est concentré à sec sous pression réduite. La poudre rouge obtenue est reprise dans de l'eau avec de l'ammoniaque à 28% (en quantité suffisante pour avoir le pH basique) : la suspension devient jaune pâle. L'insoluble est filtré et séché pour donner 862 mg de 4-iodo-1H-pyrazolo[3,4-b]pyridin-3-ylamine.

### N-(2-{3-[5-(3-Amino-1H-pyrazolo[3,4-b]pyridin-4-yl)-pyridin-2-yl]-uréido}-4-trifluorométhyl-phényl)-acétamide

### Préparé selon le schéma 1

A une solution de 70mg de 4-iodo-1H-pyrazolo[3,4-b]pyridin-3-ylamine dans 5.5mL de dioxane, à 20°C, sont ajoutés 246mg de N-(2-{3-[5-(4,4,5,5-tétraméthyl-1,3,2-dioxaborolan-2-yl)-pyridin-2-yl]-uréido}-4-trifluorométhyl-phényl)-acétamide. A cette suspension on ajoute une solution de 64mg de bicarbonate de sodium dans 1 mL d'eau, suivi de 32mg de tétrakis(triphénylphosphine)palladium. La réaction est chauffée au reflux à 85°C pendant 3,5h. La solution jaune-clair est refroidie, puis on ajoute de l'acétate d'éthyle. Le mélange est lavé avec de l'eau. Un insoluble est isolé par filtration puis le filtrat est lavé avec de la saumure. La phase organique est séchée sur sulfate de magnésium et concentrée à sec sous pression réduite. Le résidu jaune ainsi obtenu et l'insoluble précédent sont purifiés sur colonne Biotage KP-Sil de 60Å SiO₂ 32-63µ pré-packée (éluant: dichlorométhane / solution A, 9:1 puis 8:2 puis 7:3; solution A = dichlorométhane / méthanol / ammoniaque, 38:17:2). On obtient 52mg de poudre jaune de N-(2-{3-[5-(3-amino-1H-pyrazolo[3,4-b]pyridin-4-yl)-pyridin-2-yl]-uréido}-4 trifluorométhyl-phényl)-acétamide, dont les caractéristiques sont les suivantes :
SM-ES⁺: 471(+)=(M+H)(+)
   SM-ES⁻: 469(-)=(M-H)(-)
Spectre de R.M.N. ¹H (300 MHz, (CD₃)₂SO d6, δ en ppm) : 2,16 (s, 3H) ; 4,70 (s, 2H) ; 6,97 (d, J = 5,0 Hz, 1H) ; 7,40 (d, J = 9,0 Hz, 1H) ; de 7,47 à 7,54 (m, 2H) ; 8,04 (d, J = 9,0 Hz, 1H) ; 8,41 (d, J = 5,0 Hz, 1H) ; 8,48 à 8,54 (m, 2H) ; 9,86 (s, 1H) ; 10,15 (m étalé, 1H) ; 10,85 (m étalé, 1H); 12,35 (s, 1H).
Spectre IR (KBr) : 3440; 3213; 3043; 2925; 1706; 1609; 1515; 1432; 1332; 1249; 1164; 1108; 1080 & 822 cm⁻¹

### Exemple 14

### 1-[4-(3-Amino-1H-pyrazolo[3,4-b]pyridin-4-yl)-phényl]-3-(2-éthoxy-5-trifluorométhyl-phényl)-urée

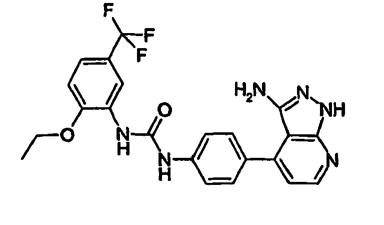

Le 2-éthoxy-5-trifluorométhyl-phénylamine a été préparé selon: European Journal of Pharmaceutical Sciences 22 (2004) p153-164

### 1-(2-Ethoxy-5-trifluorométhyl-phényl)-3-[4-(4,4,5,5-tétraméthyl-1,3,2-dioxaborolan-2-yl)-phényl]-urée

La 2-éthoxy-5-trifluorométhyl-phénylamine est mise en réaction avec le 2-(4-isocyanato-phényl)-4,4,5,5-tétraméthyl-1,3,2-dioxaborolane selon la préparation du 1-(2-méthoxy-4-trifluorométhyl-phényl)-3-[4-(4,4,5,5-tétraméthyl-1,3,2-dioxaborolan-2-yl)-phényl]-urée décrite dans l'exemple 5. On obtient, après purification, une poudre beige de 1-(2-éthoxy-5-trifluorométhyl-phényl)-3-[4-(4,4,5,5-tétraméthyl-1,3,2-dioxaborolan-2-yl)-phényl]-urée dont les caractéristiques sont les suivantes :
SM-ES⁺: 451(+)=(M+H)(+)
   SM-ES⁻: 449(-)=(M-H)(-)
Spectre de R.M.N. ¹H (300 MHz, (CD₃)₂SO d6, δ en ppm) :
   1,28 (s, 12H) ; 1,45 (t, J = 7,0 Hz, 3H) ; 4,25 (q, J = 7,0 Hz, 2H) ; 7,18 (d, J = 8,5 Hz, 1H) ; 7,30 (dd, J = 2,5 et 8,5 Hz, 1H) ; 7,50 (d, J = 8,5 Hz, 2H) ; 7,61 (d, J = 8,5 Hz, 2H) ; 8,37 (s, 1H) ; 8,55 (d, J = 2,5 Hz, 1H) ; 9,66 (s, 1H).

### 1-[4-(3-Amino-1H-pyrazolo[3,4-b]pyridin-4-yl)-phényl]-3-(2-éthoxy-5-trifluorométhyl-phényl)-urée

Le 1-(2-éthoxy-5-trifluorométhyl-phényl)-3-[4-(4,4,5,5-tétraméthyl-1,3,2-dioxaborolan-2-yl)-phényl]-urée est couplé, par réaction de Suzuki, au 4-iodo-1*H*-pyrazolo[3,4-b]pyridin-3-ylamine selon la préparation de l'exemple 6. On obtient une poudre jaune de 1-[4-(3-amino-1H-pyrazolo[3,4-b]pyridin-4-yl)-phényl]-3-(2-éthoxy-5-trifluorométhyl-phényl)-urée, dont les caractéristiques sont les suivantes :
SM-ES⁺: 457(+)=(M+H)(+)
Spectre IR (KBr) : 3369; 1672; 1601; 1542; 1445; 1315; 1271; 1210; 1134; 1043 & 822 cm⁻¹
Spectre de R.M.N. ¹H (400 MHz, (CD₃)₂SO d6, δ en ppm) : 1,47 (t, J = 7,0 Hz, 3H) ; 4,27 (q, J = 7,0 Hz, 2H) ; 4,59 (s large, 2H) ; 6,91 (d, J = 5,0 Hz, 1H) ; 7,21 (d, J = 8,5 Hz, 1H) ; 7,31 (d large, J = 8,5 Hz, 1H) ; 7,55 (d, J = 8,5 Hz, 2H) ; 7,68 (d, J = 8,5 Hz, 2H) ; 8,36 (d, J = 5,0 Hz, 1H) ; 8,44 (s, 1H) ; 8,58 (s large, 1H) ; 9,78 (s, 1H) ; 12,25 (s, 1H).

### Exemple 15

### 1-[4-(3-Amino-1H-pyrazolo[3,4-b]pyridin-4-yl)-phényl]-3-(2-fluoro-5-méthyl-phényl)-urée

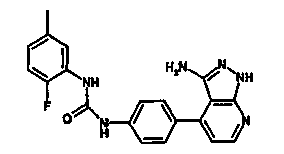

### 1-(2-Fluoro-5-méthyl-phényl)-3-[4-(4,4,5,5-tétraméthyl-1,3,2-dioxaborolan-2-yl)-phényl]-urée

A une solution de 1,45g de 4-(4,4,5,5-tétraméthyl-[1,3,2]dioxaborolan-2-yl)-phénylamine, 1,86mL de triéthylamine et 162mg de 4-diméthlamino pyridine dans 150mL de tétrahydrofurane à 20°C on ajoute 1g de 1-fluoro-2-isocyanato-4-méthyl-benzène. La réaction est agitée pendant 3,5h à 60°C, puis concentrée à sec sous pression réduite. Le résidu est repris dans un mélange d'eau et de dichlorométhane. La phase organique est lavée avec une solution de HCl 1 N puis sechée sur sulfate de magnésium et concentrée à sec sous pression réduite. On obtient 2,13g de poudre blanche de 1-(2-Fluoro-5-méthyl-phényl)-3-[4-(4,4,5,5-tetraméthyl-1,3,2-dioxaborolan-2-yl)-phényl]-urée, dont les caractéristiques sont les suivantes :
SM-EI: 370(+)=(M)(+); 125(+)=(C₇H₈NF)(+) pic de base
Spectre de R.M.N. ¹H (400 MHz, (CD₃)₂SO d6, δ en ppm) : 1,28 (s, 12H) ; 2,27 (s, 3H) ; 6,81 (m, 1H) ; 7,10 (dd, J = 8,5 et 11,5 Hz, 1H) ; 7,47 (d, J = 8,5 Hz, 2H) ; 7,60 (d, J = 8,5 Hz, 2H) ; 7,98 (dd, J = 2,0 et 8,0 Hz, 1H) ; 8,51 (d, J = 2,5 Hz, 1H) ; 9,21 (s, 1H).

### 1-[4-(3-Amino-1H-pyrazolo[3,4-b]pyridin-4-yl)-phényl]-3-(2-fluoro-5-méthyl-phényl)-urée

Le 1-(2-fluoro-5-méthyl-phényl)-3-[4-(4,4,5,5-tétraméthyl-1,3,2-dioxaborolan-2-yl)-phényl]-urée est couplé, par réaction de Suzuki, au 4-bromo-1H-pyrazolo[4,3-c]pyridin-3-ylamine selon un protocole similaire à la préparation de l'exemple 3. On obtient une poudre jaune de 1-[4-(3-amino-1H-pyrazolo[3,4-b]pyridin-4-yl)-phényl]-3-(2-fluoro-5-méthyl-phényl)-urée, dont les caractéristiques sont les suivantes :
SM-ES⁺: 377(+)=(M+H)(+)
   SM-ES⁻: 375(-)=(M-H)(-)
Spectre de R.M.N. ¹H (400 MHz, (CD₃)₂SO d6, δ en ppm) : 2,28 (s, 3H) ; 4,57 (s, 2H) ; 6,82 (m, 1H) ; 6,91 (d, J = 5,0 Hz, 1H) ; 7,11 (dd, J = 8,5 et 11,5 Hz, 1H) ; 7,54 (d, J = 8,5 Hz, 2H) ; 7,65 (d, J = 8,5 Hz, 2H) ; 7,99 (d large, J = 7,5 Hz, 1H) ; 8,37 (d, J = 5,0 Hz, 1H) ; 8,56 (s large, 1H) ; 9,29 (s, 1H) ; 12,25 (s, 1H).
Spectre IR (KBr): 3368; 1709; 1602; 1537; 1314; 1217; 1184; 1116 & 817 cm⁻¹

### Exemple 16

### 1-[4-(3-Amino-1H-pyrazolo[3,4-b]pyridin-4-yl)-phényl]-3-(3,4-diméthyl-phényl)-urée

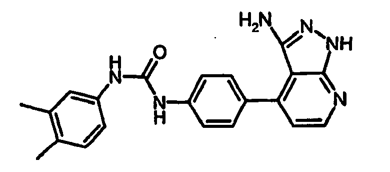

### 4-(4-Amino-phenyl)-1H-pyrazolo[3,4-b]pyridin-3-ylamine

### Préparé selon le schéma 6

A une solution de 190mg de 4-bromo-1H-pyrazolo[4,3-c]pyridin-3-ylamine et de 236mg de 4-(4,4,5,5-tétraméthyl-[1,3,2]dioxaborolan-2-yl)-phénylamine dans 20mL de dioxane est ajouté une solution de 215mg de bicarbonate de sodium dans 5mL d'eau, puis 103mg de tétrakis(triphénylphosphine)palladium. La réaction est chauffée à 85°C pendant 3,5h. La solution jaune foncée est refroidi à température ambiante et on rajoute 30mL d'acétate d'éthyle, puis on lave le mélange avec de l'eau et de la saumure. La phase organique est séchée sur sulfate de magnésium et évaporée à sec sous pression réduite. Le résidu solide jaune est purifié sur colonne Biotage KP-Sil de 60Å SiO₂ 32-63µ pré-packée (éluant : dichlorométhane / solution A, de 95:5 puis 90:10 puis 80:20; solution A = dichlorométhane / méthanol / ammoniaque, 38:17:2). On obtient 93mg de poudre jaune de 4-(4-amino-phényl)-1H-pyrazolo[3,4-b]pyridin-3-ylamine, dont les caractéristiques sont les suivantes :
SM-EI: 225(+)=(M)(+)pic de base
Spectre de R.M.N. ¹H (300 MHz, (CD₃)₂SO d6, δ en ppm) : 4,57 (s large, 2H) ; 5,47 (s large, 2H) ; 6,71 (d, J = 8,5 Hz, 2H) ; 6,80 (d, J = 5,0 Hz, 1H) ; 7,28 (d, J = 8,5 Hz, 2H) ; 8,28 (d, J = 5,0 Hz, 1H) ; 12,1 (s large, 1H)

### 1-[4-(3-Amino-1H-pyrazolo[3,4-b]pyridin-4-yl)-phényl]-3-(3,4-diméthyl-phényl)-urée

### Préparé selon le schéma 6

A une solution de 100mg de 4-(4-amino-phényl)-1H-pyrazolo[3,4-b]pyridin-3-ylamine, de 125µL de triéthylamine et de 10mg de 4-diméthlamino pyridine dans 17mL de tétrahydrofurane à 20°C on ajoute 66mg de 3,4-diméthylphényl isocyanate. La réaction est agitée pendant 6h à 80°C, puis concentrée à sec sous pression réduite. Le résidu est repris dans un mélange d'eau et d'acétate d'éthyle. La phase organique est sechée sur sulfate de magnésium puis concentrée à sec sous pression réduite. Le résidu jaune est purifié sur colonne Biotage KP-Sil de 60Å SiO₂ 32-63µ pré-packée (éluant : dichlorométhane / solution A, de 95:5 puis 90:10 puis 80:20; solution A = dichlorométhane / méthanol / ammoniaque, 38:17:2). On obtient 29mg de poudre jaune de 1-[4-(3-amino-1*H*-pyrazolo[3,4-b]pyridin-4-yl)-phényl]-3-(3,4-diméthyl-phényl)-urée, dont les caractéristiques sont les suivantes :
LC-MS : tr= 3.44mn : m/z 373 : [M+H]⁺, m/z 371 : [M-H]⁻, m/z 417 : [M-H]⁻ +HCO2H , m/z 224 : [M-H]⁻-C9H10NO
   SM-IE : m/z 372 : [M^{+.}], m/z 121 : C8H10N⁺(pic de base).
Spectre de R.M.N. ¹H (400 MHz, (CD₃)₂SO d6, δ en ppm) : 2,16 (s, 3H) ; 2,20 (s, 3H) ; 4,59 (s, 2H) ; 6,90 (d, J = 5,5 Hz, 1H) ; 7,04 (d, J = 8,5 Hz, 1H) ; 7,20 (dd, J = 2,5 et 8,5 Hz, 1H) ; 7,26 (d, J = 2,5 Hz, 1H) ; 7,52 (d, J = 8,5 Hz, 2H) ; 7,65 (d, J = 8,5 Hz, 2H) ; 8,36 (d, J = 5,5 Hz, 1H) ; 8,68 (s, 1H) ; 8,97 (s, 1H) ; 12,25 (s, 1H)

Les produits ci-dessous on été préparés selon l'exemple 16 (schéma 6):

### Exemple 17

### 1-[4-(3-Amino-1H-pyrazolo[3,4-b]pyridin-4-yl)-phényl]-3-(5-tert-butyl-2-méthoxy-phényl)-urée

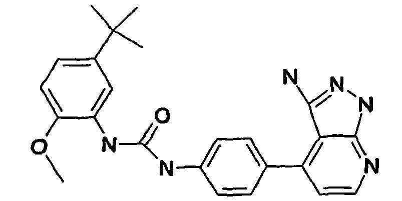

LC-MS sur ZQ : tr= 3.74mn : m/z 431 : [M+H]⁺, m/z 429 : [M-H]⁻
Spectre de R.M.N. ¹H (400 MHz, (CD₃)₂SO d6, δ en ppm) : 1,28 (s, 9H) ; 3,87 (s, 3H) ; 4,59 (s, 2H) ; 6,90 (d, J = 5,5 Hz, 1H) ; 6,92 (d, J = 8,5 Hz, 1H) ; 6,97 (dd, J = 2,5 et 8,5 Hz, 1H) ; 7,53 (d, J = 8,5 Hz, 2H) ; 7,68 (d, J = 8,5 Hz, 2H) ; 8,28 (m, 2H) ; 8,37 (d, J = 5,5 Hz, 1H) ; 9,58 (s, 1H) ; 12,25 (s large, 1H) PF = 220°C (Koffler)

### Exemple 18

### 1-[4-(3-Amino-1H-pyrazolo[3,4-b]pyridin-4-yl)-phényl]-3-(4-méthyl-3-trifluorométhyl-phényl)-urée

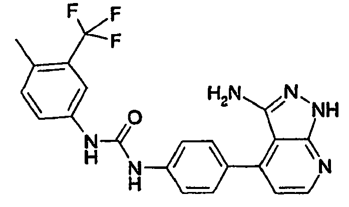

LC-MS : tr= 8.84mn : m/z 427 : [M+H]⁺, m/z 425 : [M-H]⁻
Spectre de R.M.N. ¹H (400 MHz, (CD₃)₂SO d6, δ en ppm) : 2,38 (s, 3H) ; 4,58 (s large, 2H) ; 6,90 (d, J = 5,5 Hz, 1H) ; 7,35 (d, J = 8,5 Hz, 1H) ; 7,53 (m, 3H) ; 7,68 (d, J = 8,5 Hz, 2H) ; 7,96 (d, J = 2,5 Hz, 1H) ; 8,38 (d, J = 5,5 Hz, 1H) ; 9,06 (s, 1H) ; 9,09 (s, 1H) ; 12,25 (s large, 1H).
PF = 166°C (Koffler)

### Exemple 19

### 1-[4-(3-Amino-1H-pyrazolo[3,4-b]pyridin-4-yl)-phényl]-3-(3-chloro-4-méthyl-phényl)-urée

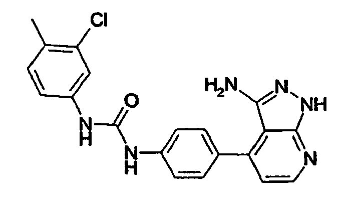

LCMS: tr= 3.30mn : m/z 393 : [M+H]⁺(pic de base), m/z 391 : [M-H]⁻
m/z 437 : [M-H]⁻+HCO2H , m/z 224 : [M-H]⁻-C8H7CINO
Spectre de R.M.N. ¹H (300 MHz, (CD₃)₂SO d6, δ en ppm) : 2,28 (s, 3H) ; 4,58 (s large, 2H) ; 6,90 (d, J = 5,5 Hz, 1H) ; de 7,18 à 7,29 (m, 2H) ; 7,53 (d, J = 8,5 Hz, 2H) ; 7,66 (d, J = 8,5 Hz, 2H) ; 7,72 (s large, 1H) ; 8,38 (d, J = 5,5 Hz, 1H) ; 8,92 (s, 1H) ; 9,02 (s, 1H) ; 12,25 (s large, 1H)
PF = 206°C (Koffler)

### Exemple 20

### 1-[4-(3-Amino-1H-pyrazolo[3,4-b]pyridin-4-yl)-phényl]-3-(3-éthyl-phényl)-urée

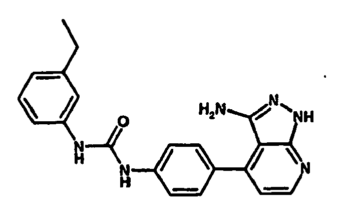

LC-MS-DAD-ELSD : 373 (+) = (M + H) (+)
371 (-) = (M - H)(-)
Spectre IR (KBr) : 3372 ; 3266 ; 1688 ; 1591 ; 1539 ; 1524 ; 1435 ; 1317 ; 1215 ; 1020 ; 822 ; 695 & 598 cm⁻¹
Spectre de R.M.N. ¹H (400 MHz, (CD₃)₂SO, δ en ppm) : 1,19 (t, J = 7,5 Hz, 3H) ; 2,58 (q, J = 7,5 Hz, 2H) ; 4,59 (s, large, 2H) ; 6,84 (d large, J = 8,0 Hz, 1H) ; 6,90 (d, J = 5,0 Hz, 1H) ; 7,19 (t, J = 8,0 Hz, 1H) ; 7,28 (d large, J = 8,0 Hz, 1H) ; 7,35 (s large, 1H) ; 7,53 (d, J = 8,5 Hz , 2H) ; 7,65 (d, J =8,5 Hz, 2H) ; 8,36 (d, J = 5,0 Hz, 1H) ; (8,74 (s, 1H) ; 8,94 (s, 1H) ; 12.25 (s, 1H). PF = 162°C (Koffler)

### Exemple 21

### 1-[4-(3-Amino-1H-pyrazolo[3,4-b]pyridin-4-yl)-phényl]-3-1,3-benzodioxol-5-yl-urée

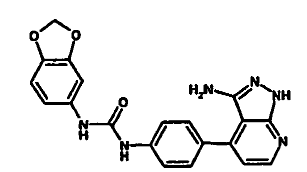

LC-MS-DAD-ELSD: 389 (+) = (M + H) (+)
387 (-) = (M - H) (-)
433 (-) = (M + Ac Form - H) (-)
Spectre IR (KBr) : 3301 ; 1639 ; 1585 ; 1503 ; 1490 ; 1246 ; 1209 ; 1035 ; 929 ; 838 & 816 cm⁻¹
Spectre de R.M.N. ¹H (400 MHz, (CD₃)₂SO, δ en ppm) : 4,58 (s large, 2H) ; 5,98 (s, 2H) ; 6,79 (dd, J = 2,0 et 8,5 Hz, 1H) ; 6,84 (d, J = 8,5 Hz, 1H) ; 6,90 (d, J = 5,0 Hz, 1H) ; 7,23 (d, J = 2,0 Hz, 1H) ; 7,52 (d, J = 9,0 Hz, 2H) ; 7,63 (d, J = 9,0 Hz, 2H) ; 8,36 (d, J = 5,0 Hz, 1H) ; 8,69 (s, 1H) ; 8,99 (s, 1H) ; 12,25 (s, 1H)
PF = 242 °C (Koffler)

### Exemple 22

### 1-[4-(3-Amino-1H-pyrazolo[3,4-b]pyridin-4-yl)-phényl]-3-(3-chloro-4-méthoxy-phényl)-urée

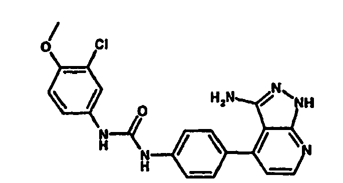

LC-MS-DAD-ELSD : 409 (+) = (M + H) (+)
407 (-) = (M - H) (-)
Spectre IR (KBr) : 3370 ; 3301 ; 1711 ; 1680 ; 1594 ; 1539 ; 1524 ; 1502 ; 1396 ; 1319 ; 1280 ; 1218 ; 1060 & 822 cm⁻¹
Spectre R.M.N. ¹H (400 MHz, (CD₃)₂SO, δ en ppm): 3,82 (s, 3H); 4,58 (s large, 2H); 6,90 (d, J = 5,0 Hz, 1H); 7,10 (d, J = 9,0 Hz, 1H); 7,30 (dd, J = 2,5 et 9,0 Hz, 1H); 7,53 (d, J = 9,0 Hz, 2H); 7,64 (d, J = 9,0 Hz, 2H) ; 7,68 (d, J = 2,5 Hz, 1H) ; 8,36 (d, J = 5,0 Hz, 1H) ; 8,80 (s, 1H) ; 8,98 (s, 1H) ; 12,25 (s, 1H).
PF = 261 °C (Koffler)

### Exemple 23

### 1-[4-(3-Amino-1H-pyrazolo[3,4-b]pyridin-4-yl)-phényl]-3-(5-chloro-2-méthoxy-phényl)-urée

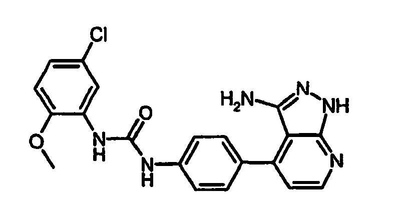

LC-MS-DAD-ELSD: 409 (+) = (M + H) (+)
407 (-) = (M - H) (-)
Spectre IR (KBr) : 3365 ; 1707 ; 1596 ; 1522 ; 1417 ; 1316 ; 1213 ; 1175 ; 1131 ; 1025 & 820 cm⁻¹
Spectre R.M.N. ¹H (400 MHz, (CD₃)₂SO, δ en ppm) : 3,91 (s, 3H); 4,59 (s large, 2H); 6,91 (d, J = 5,0 Hz, 1H); 7,00 (dd, J = 2,5 et 9,0 Hz, 1H); 7,05 (d, J = 9,0 Hz, 1H); 7,55 (d, J = 9,0 Hz, 2H) ; 7,65 (d, J = 9,0 Hz, 2H) ; 8,26 (d, J = 2,5 Hz, 1H) ; 8,37 (d , J = 5,0 Hz, 1H) ; 8,49 (s, 1H) ; 9,64 (s, 1H) ; 12,25 (s, 1H)
PF =171 °C (Koffler)

### Exemple 24

### 1-[4-(3-Amino-1H-pyrazolo[3,4-b]pyridin-4-yl)-phényl]-3-(2-éthoxy-phényl)-urée

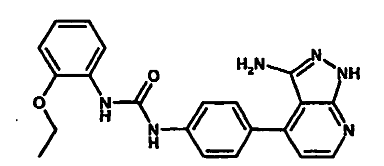

LC-MS-DAD-ELSD: 389 (+) = (M+H) (+)
387 (-) = (M - H) (-)
Spectre IR (KBr) : 2924 ; 2854 ; 1618 ; 1437 ; 1384 ; 1121 ; 1005 ; 826 ; 722 ; 695 & 544 cm⁻¹
Spectre de R.M.N. ¹H (400 MHz, (CD₃)₂SO, δ en ppm) : 1,43 (t, J = 7,0 Hz, 3H) ; 4,16 (q, J = 7,0 Hz, 2H) ; 4,59 (s large, 2H) ; de 6,85 à 6,97 (m, 3H) ; 7,02 (dd, J = 2,0 et 8,0 Hz, 1H) ; 7,54 (d, J = 9,0 Hz, 2H) ; 7,67 (d, J = 9,0 Hz, 2H) ; 8,14 (dd, J = 2,0 et 8,0 Hz, 1H) ; 8,18 (s, 1H) ; 8,36 (d, J = 5,0 Hz, 1H) ; 9,65 (s, 1H) ; 12,25 (s, 1H)
PF = 152°C (Koffler)

### Exemple 25

### 1-[4-(3-Amino-1H-pyrazolo[3,4-b]pyridin-4-yl)-phényl]-3-(2-méthoxy-5-méthyl-phényl)-urée

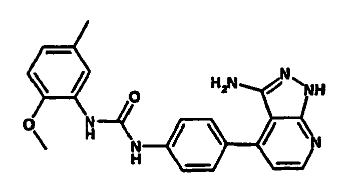

LC-MS-DAD-ELSD : 389 (+) = (M + H) (+)
387 (-) = (M - H) (-)
Spectre IR (KBr) : 3346 ; 1673 ; 1594 ; 1538 ; 1316 ; 1279 ; 1211 ; 1181 ; 1135 ; 1028 & 823 cm⁻¹
Spectre de R.M.N. ¹H (400 MHz, (CD₃)₂SO, δ en ppm) : 2,24 (s, 3H) ; 3,85 (s, 3H) ; 4,59 (s large, 2H); 6,76 (dd, J = 2,0 et 8,0 Hz, 1H); de 6,89 à 6,93 (m, 2H) ; 7,53 (d, J = 8,5 Hz, 2H) ; 7,65 (d, J = 8,5 Hz, 2H) ; 8,01 (d, J = 2,5Hz, 1H) ; 8,26 (s, 1H) ; 8,36 (d, J = 5,0 Hz, 1H) ; 9,56 (s, 1H) ; 12,25 (s, 1H) PF = 170 °C (Koffler).

### Exemple 26

### N-[4-(3-Amino-1H-pyrazolo[3,4-b]pyridin-4-yl)-phenyl]-2,3-dichloro-benzenesulfonamide

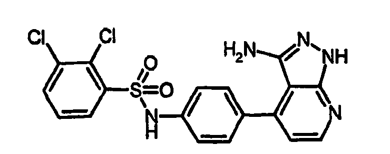

SM-ES⁺: 434 (MH⁺)
Temps de rétention DAD-TIC : 3.31min

### Exemple 27

### 1-[4-(3-Amino-1H-pyrazolo[4,3-c]pyridin-4-yl)-phényl]-3-(2-fluoro-5-méthyl-phényl)-urée

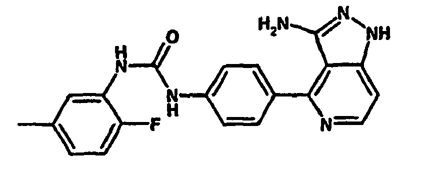

### 2,4-Dichloro-nicotinonitrile

Une solution de 5.0 g de 4-méthoxy-2-oxo-1,2-dihydro-pyridine-3-carbonitrile commercial dans 50 ml d'oxychlorure de phosphore est chauffée au reflux pendant 19H. Après refroidissement, le milieu réactionnel est coulé sur un mélange d'eau et de glace. Le précipité formé est filtré et le filtrat est extrait 2 fois avec une solution d'acétate d'éthyle / méthanol 90/10. Les phases organiques rassemblées et le précipité sont séchés sur sulfate de magnésium puis concentrés sous pression réduite pour donner 6,76 g de poudre jaunâtre. Le produit brut est purifié sur colonne Biotage KP-Sil de 60Å SiO₂ 32-63µM pré-packée (gradient d'acétate d'éthyle dans le cyclohexane de 5/95 à 10/90) pour donner 2,08 g d'une poudre blanche de 2,4-dichloro-nicotinonitrile.
SM-IE : 172 = [M^{+.}] (pic de base) , 137 = [M^{+.}] - Cl
Spectre IR (KBr) : 3072; 2236; 1559; 1539; 1445; 1368; 1220; 1197; 1069; 859; 818; 791 & 416 cm⁻¹
Spectre de R.M.N. ¹H (400 MHz, (CD₃)₂SO, δ en ppm): 7,92 (d, J = 5,5 Hz, 1H) ; 8,67 (d, J = 5,5 Hz, 1H)

### [4-(4-Chloro-3-cyano-pyridin-2-yl)-phenyl]-carbamic acid tert-butyl ester

A une solution de 519 mg de 2,4-dichloro-nicotinonitrile dans 25,5 ml de dioxane sont ajoutés 782 mg de (4-boc-aminophényl)boronic acid, 693 mg de bicarbonate de sodium dans 8,5 ml d'eau et 347 mg de tétrakis(triphenylphosphine)palladium. La suspension est agitée à 100°C pendant 2 heures sous argon. Après refroidissement, le mélange réactionnel est coulé sur de l'eau et extrait 3 fois avec une solution d'acétate d'éthyle /méthanol 90/10. Les phases organiques réunies sont séchées sur sulfate de magnésium et concentrées sous pression réduite. 1,58 g de produit brut sont chromatographiés sur colonne Biotage KP-Sil de 60Å SiO2 32-63 µM pré-packée (gradient d'une solution A dans le dichloromethane de 0.5/99.5 à 1/99 ; solution A = dichloromethanelmethanol/ammoniaque 38/17/2). On obtient 797 mg de [4-(4-chloro-3-cyano-pyridin-2-yl)-phényl]-carbamic acid tert-butyl ester, dont les caractéristiques sont les suivantes :
SM-EI: 329 (+)
Spectre IR (CCl₄) : 3343; 2981; 2230; 1741; 1524; 1501; 1411; 1392; 1368; 1316; 1220; 1155; 1050 & 844 cm⁻¹
Spectre de R.M.N. ¹H (400 MHz, (CD₃)₂SO, δ en ppm): 1,50 (s, 9H) ; 7,62 (d, J = 9,0 Hz, 2H) ; de 7,78 à 7,84 (m, 3H) ; 8,83 (d, J = 5,5 Hz, 1H) ; 9,67 (s, 1H).

### 2-(4-Amino-phényl)-4-chloro-nicotinonitrile

A une solution de 3,40 g de [4-(4-chloro-3-cyano-pyridin-2-yl)-phényl]-carbamic acid tert-butyl ester dans 20 ml de dichloromethane est ajouté 5,5 ml d'acide trifluoroacétique. La solution est agitée à 20°C pendant 26 heures. La solution est concentrée à sec sous pression réduite. Le résidu est repris dans une solution aqueuse de bicarbonate de sodium et extrait 3 fois au dichlorométhane. Les phases organiques réunies sont séchées sur sulfate de magnésium et concentrées sous pression réduite. Le résidu est lavé à l'éther diéthylique. On obtient 1,678 g d'une poudre jaune de 2-(4-amino-phényl)-4-chloro-nicotinonitrile, dont les caractéristiques sont les suivantes :
SM-ES⁺ : 230 (+) = (M + H) (+)
Spectre IR (KBr): 3402; 3339; 3230; 2223; 1608; 1555; 1538; 1520; 1432; 1388; 1180; 1066; 825 & 607 cm⁻¹
Spectre de R.M.N. ¹H (400 MHz, (CD₃)₂SO, δ en ppm) : 5,76 (s large, 2H); 6,67 (d, J = 8,5 Hz, 2H) ; 7,63 (d, J = 5,5 Hz, 1H); 7,68 (d, J = 8,5 Hz, 2H); 8,73 (d, J = 5,5 Hz, 1H).

### 1-[4-(4-Chloro-3-cyano-pyridin-2-yl)-phényl]-3-(2-fluoro-5-méthyl-phényl)-urée Préparé selon le schéma 7

A une solution de 115mg de 2-(4-amino-phényl)-4-chloro-nicotinonitrile et de 70µL de triéthylamine dans 5mL de tétrahydrofuranne à 20°C on ajoute 65µL de 2-fluoro-5-méthylphényl isocyanate. Après 3h à 20°C on ajoute de l'eau et on extrait le mélange avec de l'acétate d'éthyle. La phase organique est lavée à l'eau puis à la saumure et séchée sur sulfate de magnésium, puis concentrée à sec sous pression réduite. Le résidu est repris dans de l'éther éthylique et l'insoluble est isolé par filtration pour donner 118mg d'une poudre blanche de 1-[4-(4-chloro-3-cyano-pyridin-2-yl)-phényl]-3-(2-fluoro-5-méthyl-phényl)-urée, dont les caractéristiques sont les suivantes :
SM-EI: 380(+)=(M)(+); 125(+)=(C₇H₈NF)(+) pic de base
Spectre IR (KBr) : 3379; 2231; 1687; 1598; 1550; 1413; 1315; 1219; 1185; 1116 & 810 cm⁻¹
Spectre de R.M.N. ¹H (400 MHz, (CD₃)₂SO d6, δ en ppm) : 2,28 (s, 3H) ; 6,82 (m, 1H) ; 7,12 (dd, J = 8,5 et 11,5 Hz, 1H) ; 7,64 (d, J = 8,5 Hz, 2H) ; 7,81 (d, J = 5,5 Hz, 1H) ; 7,87 (d, J = 8,5 Hz, 2H) ; 7,99 (d large, J = 8,0 Hz, 1H) ; 8,60 (s large, 1H) ; 8,84 (d, J = 5,5 Hz, 1H) ; 9,38 (s, 1H).

### 1-[4-(3-Cyano-4-hydrazino-pyridin-2-yl)-phényl]-3-(2-fluoro-5-méthyl-phényl)-urée

### Préparé selon le schéma 7

A un mélange de 50mg de 1-[4-(4-chloro-3-cyano-pyridin-2-yl)-phényl]-3-(2-fluoro-5-méthyl-phényl)-urée dans 0,5mL d'éthanol à 20°C on ajoute 45µL d'hydrate d'hydrazine. La suspension blanche est agitée 2h à 20°C puis chauffée à 80°C pendant 4h. On laisse revenir à 20°C. L'insoluble est isolé par filtration et lavé avec de l'éther éthylique pour donner 33mg d'un solide beige de 1-[4-(3-cyano-4-hydrazino-pyridin-2-yl)-phényl]-3-(2-fluoro-5-méthyl-phényl)-urée dont les caractéristiques sont les suivantes :
SM-ES⁺: 377(+)=(M+H)(+)
SM-ES⁻: 375(-)=(M-H)(-)
Spectre IR (KBr) : 3379; 2212; 1700; 1599; 1551; 1440; 1314; 1257; 1221; 1187 & 816 cm⁻¹
Spectre de R.M.N. ¹H (400 MHz, (CD₃)₂SO d6, δ en ppm) : 2,28 (s, 3H) ; 4,52 (s, 2H) ; 6,81 (m, 1H) ; 7,06 (d, J = 6,0 Hz, 1H) ; 7,11 (dd, J = 8,5 et 11,5 Hz, 1H) ; 7,57 (d, J = 8,5 Hz, 2H) ; 7,71 (d, J = 8,5 Hz, 2H) ; 8,00 (d large, J = 8,0 Hz, 1H) ; 8,24 (s large, 1H) ; 8,27 (d, J = 6,0 Hz, 1H) ; 8,57 (s large, 1H) ; 9,32 (s, 1H).

### 1-[4-(3-Amino-1H-pyrazolo[4,3-c]pyridin-4-yl)-phényl]-3-(2-fluoro-5-méthyl-phényl)-urée

### Préparé selon le schéma 7

A une solution de 55mg de 1-[4-(3-cyano-4-hydrazino-pyridin-2-yl)-phényl]-3-(2-fluoro-5-méthyl-phényl)-urée dans 2,2mL de dichlorométhane à 20°C est ajouté 0,25mL d'acide trifluoroacétique contenant 10% d'anisole. Après 40min la solution est évaporée à sec sous pression réduite. Le résidu est repris dans de l'eau et le milieu est rendu alcalin. Le précipité formé est isolé par filtration. Ce solide beige est chromatographié sur une cartouche AIT de 2 g de silice 15-40 µm après dépôt solide (éluant avec un gradient de 100% CH₂Cl₂ à 60% CH₂Cl₂ / 40% (CH₂C₂ 38 / MeOH 17 / NH₄OH 3). On obtient 26mg d'un solide beige de 1-[4-(3-amino-1H-pyrazolo[4,3-c]pyridin-4-yl)-phényl]-3-(2-fluoro-5-méthyl-phényl)-urée dont les caractéristiques sont les suivantes :
PF : 195°C
SM-ES⁺: 377(+)=(M+H)(+)
Spectre IR (KBr) : 3351; 1696; 1605; 1544; 1313; 1257; 1221; 1181; 1045 & 808 cm⁻¹
Spectre de R.M.N. ¹H (300 MHz, (CD₃)₂SO d6, δ en ppm) : 2,28 (s, 3H) ; 4,73 (s, 2H) ; 6,82 (m, 1H) ; 7,12 (dd, J = 8,5 et 11,5 Hz, 1H) ; 7,19 (d, J = 6,0 Hz, 1H) ; de 7,59 à 7,67 (m, 4H) ; 8,01 (dd, J = 2,5 et 8,0 Hz, 1H) ; 8,21 (d, J = 6,0 Hz, 1H) ; 8,55 (d, J = 2,5 Hz, 1H) ; 9,26 (s, 1H) ; 12,1 (s large, 1H).

Les produits ci-dessous ont été préparés selon un protocole similaire à celui de l'exemple 27, (schéma 7)

### Exemple 28

### 1-[4-(3-amino-1H-pyrazolo[4,3-c]pyridin-4-yl)-phényl]-3-(2,4-diméthoxyphényl)-urée

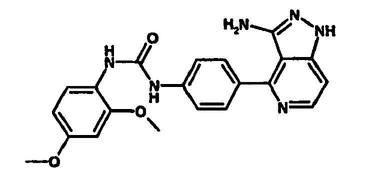

### 1-[4-(4-Chloro-3-cyano-pyridin-2-yl)-phényl]-3-(2,4-diméthoxy-phényl)-urée Préparé selon le schéma 7

Le 2-(4-amino-phényl)-4-chloro-nicotinonitrile est mise en réaction avec le 2-,4 diméthoxyphényl isocyanate dans des conditions similaires à celles décrites dans l'exemple 27. On obtient le 1-[4-(4-chloro-3-cyano-pyridin-2-yl)-phényl]-3-(2,4-diméthoxy-phényl)-urée sous forme d'un solide blanc dont les caractéristiques sont les suivantes :
SM-ES⁺: 409(+)=(M+H)(+)
Spectre de R.M.N. ¹H (400 MHz, (CD₃)₂SO d6, δ en ppm) : 3,74 (s, 3H) ; 3,88 (s, 3H) ; 6,50 (dd, J = 2,5 et 9,0 Hz, 1H) ; 6,63 (d, J = 2,5 Hz, 1H) ; 7,62 (d, J = 8,5 Hz, 2H) ; 7,80 (d, J = 5,5 Hz, 1H) ; 7,85 (d, J = 8,5 Hz, 2H) ; 7,94 (d, J = 9,0 Hz, 1H) ; 8,12 (s, 1H) ; 8,84 (d, J = 5,5 Hz, 1H) ; 9,49 (s, 1H).

### 1-[4-(3-Amino-1H-pyrazolo[4,3-c]pyridin-4-yl)-phényl]-3-(2,4-diméthoxyphényl)-urée

### Préparé selon le schéma 7

Le 1-[4-(4-chloro-3-cyano-pyridin-2-yl)-phényl]-3-(2,4-diméthoxy-phényl)-urée est traitée avec l'hydrate d'hydrazine puis avec l'acide trifluoroacétique dans des conditions similaires à celles décrites dans l'exemple 27. On obtient le 1-[4-(3-amino-1H-pyrazolo[4,3-c]pyridin-4-yl)-phényl]-3-(2,4-diméthoxy-phényl)-urée sous forme d'un solide beige, dont les caractéristiques sont les suivantes :
SM-ES⁺: 405(+)=(M+H)(+)
PF = 196°C (Koffler)
Spectre IR (KBr) : 1603; 1526; 1452; 1210; 1180; 1157; 1035 & 824 cm⁻¹
Spectre de R.M.N. ¹H (300 MHz, (CD₃)₂SO d6, δ en ppm) : 3,74 (s, 3H) ; 3,87 (s, 3H) ; 4,74 (s large, 2H) ; 6,50 (dd, J = 2,5 et 9,0 Hz, 1H) ; 6,63 (d, J = 2,5 Hz, 1H) ; 7,18 (d, J = 6,0 Hz, 1H) ; 7,61 (s large, 4H) ; 7,95 (d, J = 9,0 Hz, 1H) ; 8,09 (s, 1H) ; 8,20 (d, J = 6,0 Hz, 1H) ; 9,39 (s large, 1H) ; 12,1 (m étalé, 1H).

### Exemple 29

### 1-[4-(3-Amino-1H-pyrazolo[4,3-c]pyridin-4-yl)-phényl]-3-(5-tert-butyl-2-méthoxy-phenyl)-urée

### JK33913-110-1

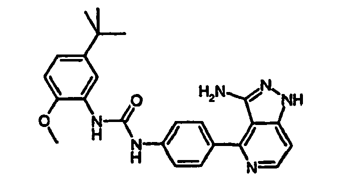

Le produit est un solide jaune dont les caractéristiques sont les suivantes:
LC-MS : 431(+)=(M+H)(+)
PF = 181°C (Koffler)
Spectre IR (KBr) : 3333; 2958; 1678; 1604; 1525; 1487; 1421; 1315; 1249; 1216; 1177; 1143; 1042; 842 & 807 cm⁻¹
Spectre de R.M.N. ¹H (300 MHz, (CD₃)₂SO d6, δ en ppm) :1,27 (s, 9H) ; 3,87 (s, 3H) ; 4,73 (s large, 2H) ; 6,92 (d, J = 8,5 Hz, 1H) ; 6,97 (dd, J = 2,0 et 8,5 Hz, 1H) ; 7,19 (d, J = 6,0 Hz, 1H) ; 7,63 (s, 4H) ; 8,21 (d, J = 6,0 Hz, 1H) ; 8,25 (s, 1H) ; 8,29 (d, J = 2,5 Hz, 1H) ; 9,51 (s, 1H) ; 12,1 (s large, 1H).

### Exemple 30

### 1-[4-(3-Amino-1H-pyrazolo[4,3-c]pyridin-4-yl)-phényl]-3-(3,4-diméthyl-phényl)-urée

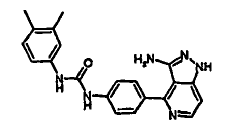

Le produit est un solide jaune dont les caractéristiques sont les suivantes:
SM-ES⁺: 373 : [M+H]⁺
PF = 182°C (Koffler)
Spectre IR (KBr) : 3413; 1679; 1621; 1549; 1210; 1136; 842; 803 & 724 cm⁻¹ contient de l'acide trifluoroacétique
Spectre de R.M.N. ¹H (400 MHz, (CD₃)₂SO d6, δ en ppm) : 2,16 (s, 3H) ; 2,20 (s, 3H) ; 4,74 (s large, 2H) ; 7,04 (d, J = 8,0 Hz, 1H) ; 7,18 (d, J = 6,0 Hz, 1H) ; 7,20 (dd partiellement masqué, J = 2,5 et 8,5 Hz, 1H) ; 7,26 (d, J = 2,5 Hz, 1H) ; 7,62 (s, 4H) ; 8,21 (d, J = 6,0 Hz, 1H) ; 8,60 (s, 1H) ; 8,87 (s, 1H) ; 12,15 (s, 1H)

### Exemple 31

### 1-[4-(3-Amino-1H-pyrazolo[4,3-c]pyridin-4-yl)-phényl]-3-(4-méthyl-3-trifluorométhyl-phényl)-urée

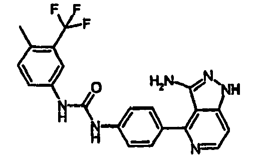

Le produit est un solide jaune dont les caractéristiques sont les suivantes:
SM-ES⁺: 427 : [M+H]⁺
PF = 191 °C (Koffler)
Spectre IR (KBr) : 3393; 3319; 3226; 3112; 1678; 1621; 1552; 1529; 1505; 1319; 1206; 1186; 1134; 1054; 839; 802 & 723 cm⁻¹ contient de l'acide trifluoroacétique
Spectre de R.M.N. ¹H (400 MHz, (CD₃)₂SO d6, δ en ppm) : 2,38 (q, J = 2,0 Hz, 3H) ; 4,74 (s large, 2H) ; 7,19 (d, J = 6,0 Hz, 1H) ; 7,35 (d, J = 8,5 Hz, 1H) ; 7,53 (dd, J = 2,5 et 8,5 Hz, 1H) ; 7,64 (s, 4H) ; 7,96 (d, J = 2,5 Hz, 1H) ; 8,21 (d, J = 6,0 Hz, 1H) ; 8,96 (s, 1H) ; 9,00 (s, 1H) ; 12,15 (s, 1H)

### Exemple 32

### 1-[4-(3-Amino-1H-pyrazolo[4,3-c]pyridin-4-yl)-phényl]-3-(2,5-difluoro-phényl)-urée

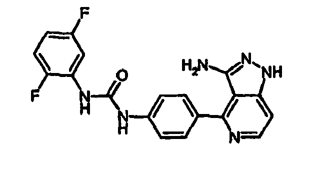

Le produit est un solide jaune dont les caractéristiques sont les suivantes:
LC-MS : 381(+)=(M+H)(+)
PF =187°C (Koffler)
Spectre IR (KBr) : 3372; 1718; 1606; 1534; 1442; 1313; 1208; 1179; 862; 796 & 727 cm⁻¹
Spectre de R.M.N. ¹H (300 MHz, (CD₃)₂SO d6, δ en ppm) : 4,74 (s large, 2H) ; 6,84 (m, 1H) ; 7,20 (d, J = 6,0 Hz, 1H) ; 7,31 (ddd, J = 5,5 - 9,0 et 11,0 Hz, 1H) ; de 7,61 à 7,68 (m, 4H) ; 8,07 (ddd, J = 3,0 - 6,5 et 11,0 Hz, 1H) ; 8,21 (d, J = 6,0 Hz, 1H) ; 8,87 (s large, 1H) ; 9,36 (s, 1H) ; 12,15 (s large, 1H) .

### Exemple 33

### 1-[4-(3-Amino-1H-pyrazolo[4,3-c]pyridin-4-yl)-phényl]-3-(3-chloro-4-méthyl-phényl)-urée

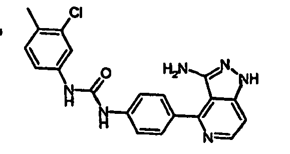

Le produit est un solide jaune-pâle dont les caractéristiques sont les suivantes:
SM-ES⁺ : 393 [M+H]⁺
PF =196°C (Koffler)
Spectre IR (KBr) :3406; 3323; 3110; 1675; 1621; 1592; 1528; 1498; 1210; 1185; 1139; 839; 803 & 724 cm⁻¹ contient de l'acide trifluoroacétique
Spectre de R.M.N. ¹H (400 MHz, (CD₃)₂SO d6, δ en ppm) : 2,27 (s, 3H) ; 4,74 (s large, 2H) ; 7,19 (d, J = 5,5 Hz, 1H) ; 7,21 (dd, J = 2,0 et 8,5 Hz, 1H) ; 7,25 (d, J = 2,0 Hz, 1H) ; 7,63 (s, 4H) ; 7,72 (d, J = 2,5 Hz, 1H) ; 8,21 (d, J = 5,5 Hz, 1H) ; 8,88 (s, 1H) ; 8,96 (s, 1H) ; 12,15 (s, 1H).

### Exemple 34

### 1-[4-(3-Amino-1H-pyrazolo[4,3-c]pyridin-4-yl)-phényl]-3-(5-fluoro-2-méthyl-phényl)-urée

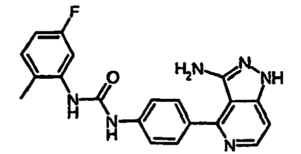

Le produit est un solide jaune dont les caractéristiques sont les suivantes:
LC-MS : 377(+)=(M+H)(+)
PF = >265°C (Koffler)
Spectre IR (KBr) : 3287; 1639; 1604; 1539; 1452; 1312; 1217; 1156; 1109; 1046; 846 & 809 cm⁻¹
Spectre de R.M.N. ¹H (300 MHz, (CD₃)₂SO d6, δ en ppm) : 2,24 (s, 3H) ; 4,74 (s large, 2H) ; 6,76 dt, J = 3,0 et 8,5 Hz, 1H) ; 7,19 (d, J = 6,0 Hz, 1H) ; 7,20 (m partiellement masqué, 1H) ; 7,64 (s, 4H) ; 7,86 (dd, J = 3,0 et 12,0 Hz, 1H) ; 8,15 (s large, 1H) ; 8,21 (d, J = 6,0 Hz, 1H) ; 9,40 (s large, 1H) ; 12,15 (s large, 1H).

### Exemple 35

### N-[4-(3-Amino-1H-pyrazolo[4,3-c]pyridin-4-yl)-phényl]-2,3-dichloro-benzenesulfonamide

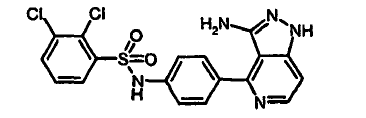

SM-ES⁺ : 434 (MH+)

### Exemple 36

### 1-[4-(3-Amino-1H-pyrazolo[4,3-c]pyridin-4-yl)-phényl].3-(5-chloro-2,4-diméthoxy-phényl)-urée

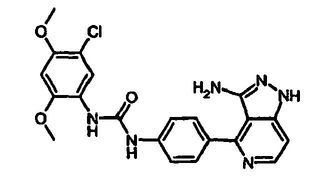

SM-ES⁺ : 439 (MH+)

### Exemple 37

### 1-[4-(3-Amino-1H-pyrazolo[4,3-c]pyridin-4-yl)-phényl]-3-(3-trifluorométhylsulfanyl-phényl)-urée

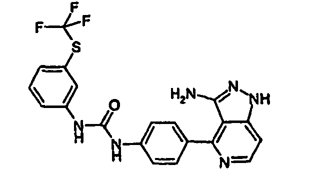

SM-ES⁺ : 445 (MH+)

### Exemple 38

### 1-[4-(3-Amino-1H-pyrazolo[3,4-c] pyridin-4-yl)-phényl]-3-(2-méthoxy-5-trifluorométhyl-phényl]-urée

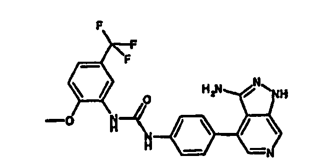

### 3-Chloro-5-(4-nitrophényl)-isonicotinonitrile

### Préparé selon le schéma 8

A une solution de 1.73 g de 3,5-dichloro-isonicotinonitrile commercial dans 120 ml de dioxane sont ajoutés 2,74 g de 4,4,5,5-tétraméthyl-2-(4-nitrophényl)-1,3,2-dioxaborolane, 2,31 g de bicarbonate de sodium dans 70 ml d'eau et 1,16 g de tétrakis(triphénylphosphine)palladium (0). La suspension est chauffée à 100°C pendant 1 h30. Après refroidissement le mélange réactionnel est coulé sur 40 ml d'eau et extrait avec 3 fois 100 ml d'acétate d'éthyle. Les phases organiques réunies sont lavées avec 50 ml d'eau, 50 ml de saumure, séchées sur sulfate de magnésium et concentrées à sec sous pression réduite. La gomme marron résiduelle est purifiée par chromatographie sur cartouche Merck pré-packée de 70 g de silice 15-40 µM en éluant au dichlorométhane. On obtient 1,58 g de solide jaune de 3-chloro-5-(4-nitro-phenyl)-isonicofinonitrile, dont les caractéristiques sont les suivantes :
SM-EI : 259 (+)
Spectre de R.M.N. ¹H (400MHz, (CD₃)₂SO, δ en ppm) : 8,02 (d, J = 9,0 Hz, 2H) ; 8,43 (d, J = 9,0 Hz, 2H) ; 8,91 (s, 1H) ; 9,09 (s, 1H)

### Dérivé N,N' di-BOC de 3-Hydrazino-5-(4-nitro-phenyl)-isonicotinonitrile

### Préparé selon le schéma 8

A une solution de 4,43 g de di-tert-butyl hydrazinodiformate dans 19 ml de diméthylformamide sont ajoutés 2,62 g de carbonate de potassium et 990 mg de 3-chloro-5-(4-nitrophenyl)-isonicotinonitrile. La suspension est chauffée à 75°C pendant 6 heures puis agitée 18 heures à 20°C. Le mélange réactionnel est extrait avec 3 fois 80 ml d'acétate d'éthyle. Les phases organiques sont lavé avec 2 fois 50 ml d'eau puis avec 50 ml de saumure. Les phases organiques réunies sont séchées sur sulfate de magnésium et concentrées sous pression réduite. Le résidu marron est purifié par chromatographie sur une cartouche Merck de 25 g de silice pré-packée 15-40 µM (gradient d'élution : cyclohexane/acétate d'éthyle 9/1 à 7/3). On obtient 2,38 g de solide jaune du dérivé N,N'-di-boc de 3-hydrazino-5-(4-nitrophényl)-isonicotinonitrile, isolés impurs et utilisés tels quels.

LC-MS: 456 (+) = (M + H) (+)
454 (-) = (M - H) (-)

### Dérivé N.N'-di-Boc de 3-(4-amino-phenyl)-5-hydrazino-isonicotinonitrile

### Préparé selon le schéma 8

A une solution de 1,74 g de dérivé N,N'-di-boc du 3-hydrazino-5-(4-nitro-phényl)-isonicotinonitrile dans 8 ml d'éthanol sont ajoutés 3 ml de cyclohexène et 200 mg d'hydroxyde de palladium. La suspension est agitée au reflux pendant 2h15, filtrée à chaud sur célite et concentrée à sec sous pression réduite. Le résidu beige est purifié par chromatographie sur cartouche Merck de 70 g de silice 15-40 µM en éluant avec une solution acétate d'éthyle / dichlorométhane 8/2. On obtient 309 mg de solide jaune de dérivé N,N'-di-boc de 3.(4-Amino-phenyl)-5-hydrazino-isonicotinonitrile, dont les caractéristiques sont les suivantes :
LC-MS : 426 (+) = (M + H) (+)
Spectre de R.M.N. ¹H (400 Mhz, (CD₃)₂SO, δ en ppm) : 1,45 (s large, 18H) ; 5,61 (s,2H) ; 6,71 (d, J = 9,0 Hz, 2H) ; 7,31 (d, J = 9,0 Hz, 2H); 8,55 (s large, 1H); 8,64 (s, 1H); 10,05 (m étalé, 1H)

### Dérivé N N' di-BOC de 1-[4-(4-Cyano-5-hydrazino-pyridin-3-yl)-phenyl]-3-(2-méthoxy-5-trifluorométhyl-phenyl]-urée

### Préparé selon le schéma 8

A une solution de 41 mg de triphosgène dans 4 ml de tétrahydrofurane sont ajoutés à 0°C sous argon 78 mg de 2-méthoxy-5-trifluorométhyl aniline dans 2 ml de tétrahydrofurane et 110 µl de triéthylamine. La suspension est agitée à 0°C pendant 10 minutes puis à 20°C pendant 1h15. Une solution de 174 mg de dérivé N,N'-di-boc de 3-(4-amino-phényl)-5-hydrazino-isonicotinonitrile dans 2ml de tétrahydrofurane est alors additionnée. Le mélange réactionnel est chauffé au reflux pendant 8 heures puis concentré à sec sous pression réduite. Le résidu est purifié par chromatographie sur une cartouche Analogix de 12 g de silice 50 µM (gradient d'élution de l'acétate d'éthyle dans le cyclohexane de 1/9 à 5/5). On obtient 195 mg de solide jaune de dérivé N,N'-di-boc de 1-[4-(4-cyano-5-hydrazino-pyridin-3-yl)-phényl]-3-(2-méthoxy-5-trifluorométhyl-phényl]-urée, dont les caractéristiques sont les suivantes :
LC-MS: 643 (+) = (M + H) (+)
Spectre de R.M.N. ¹H (400 MHz, (CD₃)₂SO, δ en ppm) : De 1,4 à 1,55 (s large, 18H) ; 3,99 (s, 3H) ; 7,22 (d, J = 8,5 Hz, 1H) ; 7,34 (dd, J = 2,0 et 8,5 Hz, 1H) ; 7,58 (d, J = 9,0 Hz, 2H) ; 7,69 (d, J = 9,0 Hz, 2H) ; 8,57 (d, J = 2,0 Hz, 1H); 8,65 (s, 1H); 8,69 (s large, 1H); 8,72 (s, 1H); 9,78 (s, 1H); 10,08 (m étalé, 1H)

### 1-[4-(3-Amino-1H-pyrazolo[3,4-c]pyridin-4-yl)-phenyl]-3-(2-méthoxy-5-trifluorométhyl-phenyl]-urée

### Préparé selon le schéma 8

Une solution de 282 mg de dérivé N,N'-di-boc de 1-[4-(4-cyano-5-hydrazino-pyridin-3-yl)-phényl]-3-(2-méthoxy-5-trifluorométhyl-phényl]-urée et de 600 µl d'acide trifluoroacétique à 10% d'anisole dans 8 ml de dichlorométhane est chauffée au reflux pendant 2h30. Après refroidissement le mélange réactionnel est coulé sur une solution saturée en bicarbonate de sodium et extrait avec 40 ml d'acétate d'éthyle. La phase organique est séchée sur sulfate de magnésium et concentrée à sec sous pression réduite. Le résidu jaune est empâté dans l'éther éthylique. Après filtration et séchage sous vide de l'insoluble on obtient 103 mg de solide jaune de 1-[4-(3-amino-1H-pyrazolo[3,4-c] pyridin-4-yl)-phényl]-3-(2-méthoxy-5-trifluorométhyl-phényl]-urée, dont les caractéristiques sont les suivantes :
LC-MS: tr= 2.83mn : 443 = [M + H]⁺ ; 441 = [M - H]⁻; 487 = [M - H]⁻ + HCO₂H SM-EI: 176 (pic de base) = C₈H₆F₃O⁺ ; 442 = [M⁺]
Spectre IR (KBr) : 3342 ; 1696 ; 1609 ; 1538 ; 1491 ; 1447 ; 1314 ; 1270 ; 1215 ; 1177 ; 1135 ; 1024 ; 837 & 622 cm⁻¹
Spectre de R.M.N. ¹H (400 MHz, (CD₃)₂SO, δ en ppm) : 3,99 (s, 3H) ; 4,61 (s, 2H) ; 7,22 (d, J = 8,5 Hz, 1H) ; 7,33 (d large, J = 8,5 Hz, 1H) ; 7,50 (d, J = 8,5 Hz, 2H) ; 7,64 (d, J = 8,5 Hz, 2H) ; 7,93 (s, 1H) ; 8,58 (s large, 2H) ; 8,74 (s, 1H) ; 9,61 (s, 1H) ; 12 ,25 (s, 1H)
PF = 210°C (Koffler)

### Exemple 39

### N-[4-(3-Amino-1H-pyrazolo[3,4-c]pyridin-4-yl)-phényl]-2,3-dichloro-benzenesulfonamide

### Préparé selon les schémas 6 et 8

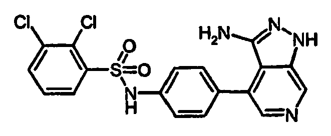

SM-ES⁺ = 434 (MH+)

### Exemple 40

### Acide 3-{3-[4-(3-amino-1H-pyrazolo[3,4-b]pyridin-4-yl)-phényl]-uréido}-4-méthoxy-benzoic

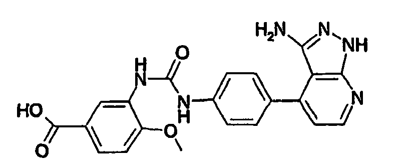

A une solution de 100mg de 1-[4-(3-amino-1H-pyrazoto[3,4-b]pyridin-4-yl)-phényl]-3-(2-méthoxy-5-trifluorométhyl-phényl)-urée, décrit dans l'exemple 5, dans 5mL de dichlorométhane à -40°C on ajoute goutte à goutte 1,6mL d'une solution 1 M de tribromure de bore et on laisse remonter à 20°C. on concentre à sec sous courrant d'argon une nuit. Le résidu est purifié sur colonne Biotage KP-Sil de 60Å SiO₂ 32-63_{N} pré-packée (éluant : dichlorométhane / solution A, de 95:5 puis 90:10 puis 80:20 puis 73:30; solution A = dichlorométhane / méthanol / ammoniaque, 38:17:2). On obtient 41 mg de poudre jaune d'acide 3-{3-[4-(3-amino-1*H*-pyrazolo[3,4-*b*]pyridin-4-yl)-phényl]-uréido}-4-méthoxy-benzoique, dont les caractéristiques sont les suivantes :
LCMS sur Quattro Premier : tr= 5.04mn
m/z 419 : [M+H]⁺
Spectre IR (KBr) :3354; 1697; 1597; 1542; 1431; 1319; 1279; 1205; 1122; 821 & 767 cm⁻¹
Spectre de R.M.N. ¹H (400 MHz, (CD₃)₂SO, δ en ppm) :3,80 (s, 3H) ; 4,59 (s, 2H) ; 6,90 (d, J = 5,5 Hz, 1H) ; 6,92 (d, J = 8,5 Hz, 1H) ; 7,51 (dd, J = 2,5 et 8,5 Hz, 1H) ; 7,54 (d, J = 8,5 Hz, 2H) ; 7,67 (d, J = 8,5 Hz, 2H) ; 8,38 (d, J = 5,5 Hz, 1H) ; 8,40 (s, 1H) ; 8,79 (d, J = 2,5 Hz, 1H) ; 9,60 (s, 1H) ; 10,6 (m très étalé, 1H) ; 12,25 (s large, 1H).

### Exemple 41

### 1-[4-(3-Amino-1H-pyrazolo[3,4-b]pyridin-4-yl)-phényl]-3-(2-hydroxy-5-trifluorométhyl-phényl)-urée

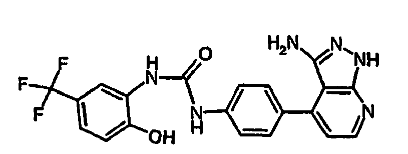

A une solution de 1-[4-(3-amino-1H-pyrazolo[3,4-b]pyridin-4-yl)-phényl]-3-(2-méthoxy-5-trifluorométhyl-phényl)-urée, décrit dans l'exemple 5, dans 6mL de dichlorométhane à -40°C on ajoute goutte à goutte 1,6mL d'une solution 1 M de tribromure de bore et on laisse remonter à 20°C. Après 30min, la réaction est refroidi dans un bain d'eau glacée puis on ajoute de l'eau. Après 15min d'agitation le mélange est filtré et l'insoluble orange lavé à l'eau et au dichlorométhane. Le solide est purifié sur colonne Biotage KP-Sil de 60Å SiO₂ 32-63µ pré-packée (éluant : dichlorométhane / solution A, de 95:5 puis 90:10 puis 80:20 puis 73 :30; solution A = dichlorométhane / méthanol / ammoniaque, 38:17:2). On obtient 29mg de poudre jaune de 1-[4-(3-Amino-1*H*-pyrazolo[3,4-b]pyridin-4-yl)-phényl]-3-(2-hydroxy-5-trifluorométhyl-phényl)-urée, dont les caractéristiques sont les suivantes :
LCMS sur ZQ : tr= 3.22mn
m/z 429 [M+H]⁺(pic de basé)
Spectre de R.M.N. ¹H (300 MHz, (CD₃)₂SO d6, δ en ppm) : 4,59 (s, 2H) ; 6,90 (d, J = 5,5 Hz, 1H) ; 7,00 (d, J = 8,5 Hz, 1H) ; 7,18 (dd, J = 2,5 et 8,5 Hz, 1H) ; 7,54 (d, J = 8,5 Hz, 2H) ; 7,66 (d, J = 8,5 Hz, 2H) ; 8,37 (d, J = 5,5 Hz, 1H) ; 8,51 (m, 2H) ; 9,64 (s, 1H) ; 10,7 (m très étalé, 1H) ; 12,25 (s large, 1H).

### Détermination de l'activité des composés - Protocoles expérimentaux

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70% d'homogénéité.

L'activité kinase est déterminée en incubant l'enzyme (6.6 µg/mL) avec différentes concentrations de composé à tester dans un tampon 50 mM Hepes pH = 7,2, 10 mM MgCl₂, 100 µM Na₃VO₄ ,15 µM d'ATP pendant 1 heure à 37°C. La réaction enzymatique est stoppée par l'addition de tampon Hepes pH = 7,0 contenant 0.4 mM KF, 133 mM EDTA, 0.1 % BSA et le marquage est effectuée, pendant 1 à 2 heures à température ambiante, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK. Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).
Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLCγ exprimée sous forme de protéine de fusion GST), 2 µCi γ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).
Le bruit de fond est déterminé par la mesure de la radioactivité dans quatre puits différents contenant l'ATP radioactif et le substrat seul.
Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.
Le composé SU5614 (Calbiochem) (1 µM) est inclus dans chaque plaque comme contrôle d'inhibition.

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.
L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.
L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FIashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µl de PBS. La radioactivité est mesurée sur un MicroBeta1450 Wallac.
L'inhibition de l'activité Tie2 est calculée et exprimée en pourcentage d'inhibition par rapport à l'activité contrôle déterminée en l'absence de composé.

### Résultats : Tableau 1 :

| Structure | exemple | FAK | KDR | TIE2 |
|---|---|---|---|---|
| | | IC 50 (nM | IC 50 (nM) | IC 50 (nM) |
| 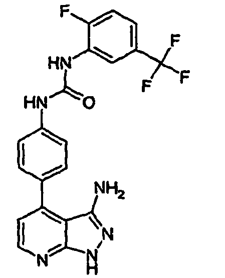 | **1** | 264 | 50 | 8 |
| 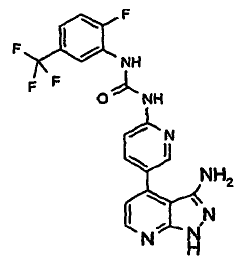 | **2** | 150 | 940 | 23 |
| 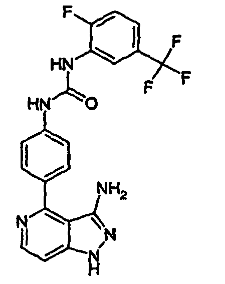 | **3** | 73 | 33 | 5 |
| 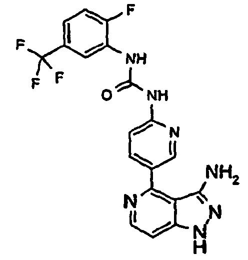 | **4** | 80 | 218 | 4 |
| 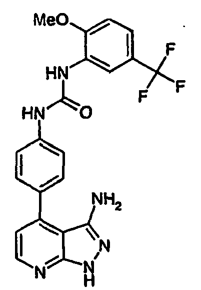 | **5** | 286 | 49 | 15 |
| 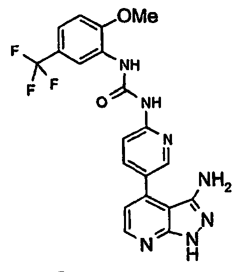 | **6** | 190 | 1855 | 27 |
| 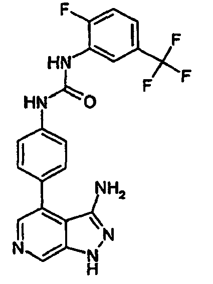 | **7** | - | 36 | 9 |
| 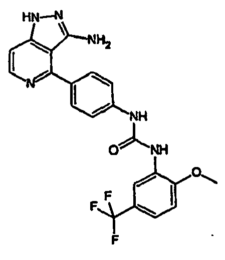 | **10** | - | 60 | 10 |
| 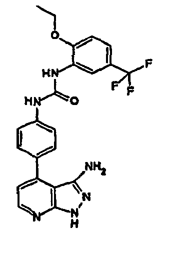 | **14** | - | 24 | 21 |

## Revendications

1. Produit répondant à la formule (I) suivante: 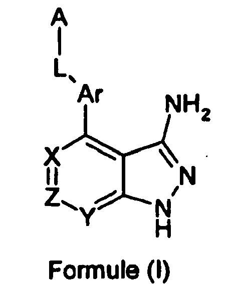 dans laquelle :
1) Ar-L-A est 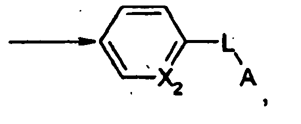 dans lequel X2 est choisi parmi N, C-CH₃, CF et CH.
2) A est est choisi parmi phényle, pyrazolyle et isoxazolyle ; éventuellement substitué par un ou plusieurs substituant choisi parmi halogène; (C1-C12)alkyle; (C6-C14)aryle; (C1-C13)hétéroaryle avec 1 à 4 hétéroatomes, (C3-C12)cycloalkyle ; (C1-C13)hétérocyclyle avec 1 à 4 hétéroatomes; (C2-C12)alkylène; (C2-C12)alkynyle; OH ; O-(C1-C12)alkyle; O-(C2-C12)alkylèrie ; O-(C6-C14)aryle; O-(C1-C13)hétéroaryle avec 1 à 4 hétéroatomes; NH₂ ; NH-(C1-C12)alkyle; NH-(C6-C14)aryle; NH-(C1-C13)hétéroaryle; N-(C1-C12)alkyle-(C1-C12)alkyle' ; SH ; S-(C1-C12)alkyle; S-(C6-C14)aryle; S(O₂)H ; S(O₂)-(C1-C12)alkyle; S(O₂)-(C6-C14)aryle; SO₃H ; SO₃-(C1-C12)alkyle; SO3-(C6-C14)aryle; CHO; C(O)-(C1-C12)alkyle; C(O)-(C6-C14)aryle; C(O)OH ; C(O)O-(C1-C12)alkyle; C(O)O-(C6-C14)aryle; OC(O)-(C1-C12)alkyle; OC(O)-(C6-C14)aryle ; C(O)NH₂; C(O)NH-(C1-C12)alkyle; C(O)NH-(C6-C14)aryle ; NHCHO; NHC(O)-(C1-C12)alkyle; NHC(O)-(C6-C14)aryle ; NH-(C3-C12)cycloalkyle; NH-(C1-C13)hétérocyclyle avec 1 à 4 hétéroatomes, CONH-(C1-C13)Hétérocyclyle avec 1 à 4 hétéroatomes, CO-(C1-C13)Hétéroaryle avec 1 à 4 hétéroatomes, CO-(C1-C13)Hétérocyclyle avec 1 à 4 hétéroatomes, NHCO-(C1-C13)Héteroaryle avec 1 à 4 hétéroatomes, NHCO-(C1-C13)Hétérocyclyle avec 1 à 4 hétéroatomes , NHCONH-(C1-C12)alkyle.
3) L est sélectionné dans le groupe constitué par: NH-SO₂, - NH-CO-NH,;
4) L'un de X, Y et Z est choisi parmi N et NO, et deux autres de Z, Y et X sont C(R5) et C(R6);
5) R5 et R6 sont sélectionnés parmi H et F.

2. Produit selon la revendication 1, **caractérisé en ce que** Ar-L-A est : 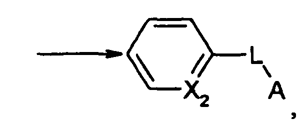 dans lequel X2 est N.

3. Produit selon la revendication 1 **caractérisé en ce que** L-A est NH-CO-NH-A.

4. Produit selon l'une quelconque des revendications 1 à 3, **caractérisé en ce que** A est phényle ou isoxazolyle substitué par halogène, (C1-C4)alkyle, (C1-C3)alkyle halogéné, O-(C1-C4)alkyle, S-(C1-C4)alkyle, O-(C1-C4)alkyle halogéné, S-(C1-C4)alkyle halogéné et lorsque A est disubsitué, les deux substituants de A peuvent former un cycle de 5 à 7 chainons contenant de 0 à 3 hétéroatomes.

5. Produit selon l'une quelconque des revendications 1 à 3, **caractérisé en ce que** A est substitué par un ou plusieurs substituants, identiques ou différents, indépendemment sélectionnés dans le groupe constitué par F, CI, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9), (C1-C6)alkyle, (C2-C6)alkylène, (C2-C6)alkynyle, aryle, hétéroaryle, O-(C1-C6)alkyle, O-Aryle, O-hétéroaryle, S-(C1-C6)alkyle, S-Aryle, S-hétéroaryle, chacun étant éventuellement substitué par un ou plusieurs substituants choisis parmi (C1-C3)alkyle, halogène, O-(C1-C3)alkyle; dans lequel R8 et R9 sont indépendamment choisis parmi H, (C1-C3)alkyle, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et
K ; et dans lequel R10 est H ou un hétérocycle non aromatique éventuellement substitué, comprenant 2 à 7 atomes de carbone, et 1 à 3 hétéroatomes choisis parmi N, O et S

6. Produit selon la revendication 1, **caractérisé en ce que** A est 2-fluoro-5-trifluorométhyl-phényle ou 2-méthoxy-5-trifluorométhyl-phényle.

7. Produit selon la revendication 1, **caractérisé en ce qu'**il est choisi
parmi:
1-[4-(3-Amino-1H-pyrazolo[3,4-b]pyridin-4-yl)-phényl]-3-(2-fluoro-5-trifluorométhyl-phényl)-urée,
1-[5-(3-Amino-1H-pyrazolo[3,4-b]pyridin-4-yl)-pyridin-2-yl]-3-(2-fluoro-5-trifluorométhyl-phényl)-urée,
1-[4-(3-Amino-1H-pyrazolo[3,4-b]pyridin-4-yl)-phényl]-3-(2-méthoxy-5-trifluorométhyl-phényl)-urée,
1-[5-(3-Amino-1H-pyrazolo[3,4-b]pyridin-4-yl)-pyridin-2-yl]-3-(2-méthoxy-5-trifluorométhyl-phényl)-urée,
1-[4-(3-Amino-1H-pyrazolo[3,4-b]pyridin-4-yl)-phényl]-3-(2-méthylcarbonylamino-5-trifluorométhyl-phényl)-urée,
1-[5-(3-Amino-1H-pyrazolo[3,4-b]pyridin-4-yl)-pyridin-2-yl]-3-(2-méthylcarbonylamino-5-trifluorométhyl-phényl)-urée,
1-[4-(3-Amino-1H-pyrazolo[3,4-b]pyridin-4-yl)-phényl]-3-(2-éthoxy-5-trifluorométhyl-phényl)-urée,
1-[4-(3-Amino-1H-pyrazolo[3,4-b]pyridin-4-yl)-phényl]-3-(2-fluoro-5-méthyl-phényl)-urée,
Acide 3-{3-[4-(3-amino-1*H*-pyrazolo[3,4-*b*]pyridin-4-yl)-phényl]-uréido}-4-méthoxy-benzoique,
1-[4-(3-Amino-1H-pyrazolo[3,4-b]pyridin-4-yl)-phényl]-3-(2-hydroxy-5-trifluorométhyl-phényl)-urée,
1-[4-(3-Amino-1H-pyrazolo[3,4-b]pyridin-4-yl)-phényl]-3-(3,4-diméthyl-phényl)-urée,
1-[4-(3-Amino-1H-pyrazolo[3,4-b]pyridin-4-yl)-phényl]-3-(2-méthoxy-5-terbutyl-phényl)-urée,
1-[4-(3-Amino-1H-pyrazolo[3,4-b]pyridin-4-yl)-phényl]-3-(3-trifluorométhyl-4-méthyl-phényl)-urée,
1-[4-(3-Amino-1H-pyrazolo[3,4-b]pyridin-4-yl)-phényl]-3-(3-chloro-4-méthyl-phényl)-urée,
1-[4-(3-Amino-1H-pyrazolo[3,4-b]pyridin-4-yl)-phényl]-3-(3-éthyl-phényl)-urée,
1-[4-(3-Amino-1H-pyrazolo[3,4-b]pyridin-4-yi)-phényl]-3-1,3-benzodioxol-5-yl-urée,
1-[4-(3-Amino-1H-pyrazolo[3,4-b]pyridin-4-yl)-phényl]-3-(3-chloro-4-méthoxy-phényl)-urée,
1-[4-(3-Amino-1H-pyrazolo[3,4-b]pyridin-4-yl)-phényl]-3-(2-méthoxy-5-chloro-phényl)-urée,
1-[4-(3-Amino-1H-pyrazolo[3,4-b]pyridin-4-yl)-phényl]-3-(2-éthoxy-phényl)-urée,
1-[4-(3-Amino-1H-pyrazolo[3,4-b]pyridin-4-yl)-phényl]-3-(2-méthoxy-5-méthyl-phényl)-urée,
N-[4-(3-Amino-1H-pyrazolo[3,4-b]pyridin-4-yl)-phenyl]-2,3-dichloro-benzenesulfonamide.

8. Produit selon la revendication 1, **caractérisé en ce qu'**il est choisi
parmi:
1-[4-(3-Amino-1H-pyrazolo[4,3-c]pyridin-4-yl)-phényl]-3-(2-fluoro-5-trifluorométhyl-phényl)-urée,
1-[5-(3-Amino-1H-pyrazolo[4,3-c]pyridin-4-yl)-pyridin-2-yl]-3-(2-fluoro-5-trifluorométhyl-phényl)-urée,
1-[4-(3-Amino-1H-pyrazolo[4,3-c]pyridin-4-yl)-phényl]-3-(phényl)-urée,
1-[5-(3-Amino-1H-pyrazolo[4,3-c]pyridin-4-yl)-pyridin-2-yl]-3-(2-méthoxy-5-trifluorométhyl-phényl)-urée,
1-[4-(3-Amino-1H-pyrazolo[4,3-c]pyridin-4-yl)-phényl]-3-(2-méthoxy-5-trifluorométhyl-phényl)-urée,
1-[4-(3-Amino-1H-pyrazolo[4,3-c]pyridin-4-yl)-phényl]-3-(2-méthylcarbonylamino-5-trifluorométhyl-phényl)-urée,
1-[4-(3-Amino-1H-pyrazolo[4,3-c]pyridin-4-yl)-phényl]-3-(2-fluoro-5-méthyl-phényl)-urée,
1-[4-(3-Amino-1H-pyrazolo[4,3-c]pyridin-4-yl)-phényl]-3-(2,4-diméthoxy-phényl)-urée,
N-[4-(3-Amino-1H-pyrazolo[4,3-c]pyridin-4-yl)-phényl]-2,3-dichloro-benzenesulfonamide,
1-[4-(3-Amino-1H-pyrazolo[4,3-c]pyridin-4-yl)-phényl]-3-(2-méthoxy-5-terbutyl-phényl)-urée,
1-[4-(3-Amino-1H-pyrazolo[4,3-c]pyridin-4-yl)-phényll-3-(3,4-diméthyl-phényl)-urée,
1-[4-(3-Amino-1H-pyrazolo[4,3-c]pyridin-4-yl)-phényl]-3-(3-trifluorométhyl-4-méthyl-phényl)-urée,
1-[4-(3-Amino-1H-pyrazolo[4,3-c]pyridin-4-yl)-phényl]-3-(2,5-difluoro-phényl)-urée,
1-[4-(3-Amino-1H-pyrazolo[4,3-c]pyridin-4-yl)-phényl]-3-(3-chloro-4-méthyl-phényl)-urée,
1-[4-(3-Amino-1H-pyrazolo[4,3-c]pyridin-4-yl)-phényl]-3-(2-méthyl-5-fluoro-phényl)-urée,
1-[4-(3-Amino-1H-pyrazolo[4,3-c]pyridin-4-yl)-phényl]-3-(2,4-diméthoxy-5-chloro-phényl)-urée,
1-[4-(3-Amino-1H-pyrazolo[4,3-c]pyridin-4-yl)-phényl]-3-(3-trifluorométhylsulfanyl-phényl)-urée.

9. Produit selon la revendication 1, **caractérisé en ce qu'**il est choisi
parmi :
1-[4-(3-Amino-1H-pyrazolo[3,4-c]pyridin-4-yl)-phényl]-3-(2-fluoro-5-trifluorométhyl-phényl)-urée,
1-[4-(3-Amino-1H-pyrazolo[3,4-c]pyridin-4-yl)-phényl]-3-(2-méthoxy-5-trifluorométhyl-phényl)-urée,
N-[4-(3-Amino-1H-pyrazolo[3,4-c]pyridin-4-yl)-phényl]-2,3-dichloro-benzenesulfonamide.

10. produit selon l'une quelconque des revendications précédentes, **caractérisé en ce qu'**il est sous forme :
a. non chirale, ou
b. racémique, ou
c. enrichie en un stéréo-isomère, ou
d. enrichie en un énantiomère ;
et **en ce qu'**il est éventuellement salifié.

11. Médicament, **caractérisé en ce qu'**il comprend un produit de formule (I) selon l'une quelconque des revendication 1 à 9, ou un sel d'addition de ce composé à un acide pharmaceutiquement acceptable, ou encore un hydrate ou un solvate du produit de formule (I).

12. Composition pharmaceutique comprenant un produit selon l'une quelconque des revendications précédentes, en combinaison avec un excipient pharmaceutiquement acceptable.

13. Utilisation d'un produit selon l'une quelconque des revendications 1 à 9 pour la préparation d'un médicament comme agent inhibiteur d'une réaction catalysée par une kinase.

14. Utilisation d'un produit selon la revendication 13, **caractérisée en ce que** la kinase est choisie parmi FAK, KDR et Tie2.

15. Utilisation d'un produit selon l'une quelconque des revendications 1 à 9, pour la fabrication d'un médicament utile pour traiter un état pathologique.

16. Utilisation selon la revendication 15, **caractérisée en ce que** l'état pathologique est le cancer.

## Claims

1. Product of the following formula (I): 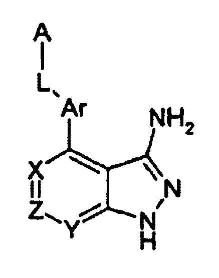 in which:
1) Ar-L-A is 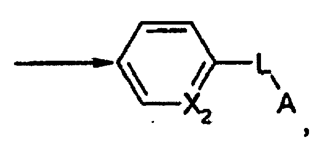 in which X2 is selected from N, C-CH₃, CF and CH.
2) A is selected from phenyl, pyrazolyl and isoxazolyl; optionally substituted with one or more substituents selected from halogen; (C1-C12)alkyl; (C6-C14)aryl; (C1-C13)heteroaryl having 1 to 4 heteroatoms; (C3-C12)cycloalkyl; (C1-C13)heterocyclyl having 1 to 4 heteroatoms; (C2-C12)alkylene; (C2-C12)alkynyl; OH; O-(C1-C12)alkyl; O-(C2-C12)alkylene; O-(C6-C14)aryl; O-(C1-C13)heteroaryl having 1 to 4 heteroatoms; NH₂; NH-(C1-C12)alkyl; NH-(C6-C14)aryl; NH-(C1-C13)heteroaryl; N-(C1-C12)alkyl-(C1-C12)alkyl; SH; S-(C1-C12)alkyl; S-(C6-C14)aryl; S(O₂)H; S(O₂)-(C1-C12)alkyl; S(O₂)-(C6-C14)aryl; SO₃H; SO₃-(C1-C12)alkyl; SO₃-(C6-C14)aryl; CHO; C(O)-(C1-C12)alkyl; C(O)-(C6-C14)aryl; C(O)OH; C(O)O-(C1-C12)alkyl; C(O)O-(C6-C14)aryl; OC(O)-(C1-C12)alkyl; OC(O)-(C6-C14)aryl; C(O)NH₂; C(O)NH-(C1-C12)alkyl; C(O)NH-(C6-C14)aryl; NHCHO; NHC(O)-(C1-C12)alkyl; NHC(O)-(C6-C14)aryl; NH-(C3-C12)cycloalkyl; NH-(C1-C13)heterocyclyl having 1 to 4 heteroatoms; CONH-(C1-C13)heterocyclyl having 1 to 4 heteroatoms; CO-(C1-C13)heteroaryl having 1 to 4 heteroatoms; CO-(C1-C13)heterocyclyl having 1 to 4 heteroatoms; NHCO-(C1-C13)heteroaryl having 1 to 4 heteroatoms; NHCO-(C1-C13)heterocyclyl having 1 to 4 heteroatoms; NHCONH-(C1-C12)alkyl.
3) L is selected from the group consisting of: NH-SO₂, -NH-CO-NH;
4) one of X, Y and Z is selected from N and NO, and the other two of Z, Y and X are C(R5) and C(R6)
5) R5 and R6 are selected from H and F.

2. Product according to claim 1, **characterised in that** Ar-L-A is: 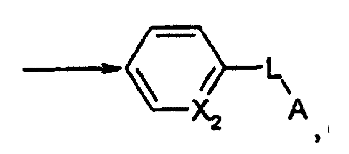 in which X2 is N.

3. Product according to claim 1, **characterised in that** L-A is NH-CO-NH-A.

4. Product according to any one of claims 1 to 3, **characterised in that** A is phenyl or isoxazolyl substituted by halogen, (C1-C4)alkyl, halogenated (C1-C3)alkyl, O-(C1-C4)alkyl, S-(C1-C4)alkyl, halogenated O-(C1-C4)alkyl, halogenated S-(C1-C4) alkyl, and when A is disubstituted, the two substituents of A can form a cycle of 5 to 7 links containing 0 to 3 heteroatoms.

5. Product according to any one of claims 1 to 3, **characterised in that** A is substituted by one or more identical or different substituents selected independently from the group consisting of F, Cl, Br, I, OH, SH, SO₃M, COOM, CN, NO₂, CON(R8)(R9), N(R8)CO(R9), (C1-C3)alkyl-OH, (C1-C3)alkyl-N(R8)(R9), (C1-C3)alkyl-(R10), (C1-C3)alkyl-COOH, N(R8)(R9), (C1-C6)alkyl, (C2-C6)alkylene, (C2-C6)alkynyl, aryl, heteroaryl, O-(C1-C6)alkyl, O-aryl, O-heteroaryl, S-(C1-C6)alkyl, S-aryl, S-heteroaryl, each optionally being substituted by one or more substituents selected from (C1-C3)alkyl, halogen, O-(C1-C3)alkyl; in which R8 and R9 are independently selected from H, (C1-C3)alkyl, (C1-C3)alkylOH, (C1-C3)alkylNH₂, (C1-C3)alkylCOOM, (C1-C3)alkylSO₃M; in which when R8 and R9 are simultaneously different from H, they can be linked to form a cycle of 5 to 7 links comprising 0 to 3 heteroatoms selected from 0, N and S; in which M is H or an alkali metal cation selected from Li, Na and K; and in which R10 is H or an optionally substituted non-aromatic heterocycle, comprising 2 to 7 carbon atoms and 1 to 3 heteroatoms selected from N, O and S.

6. Product according to claim 1, **characterised in that** A is 2-fluoro-5-trimethyl-phenyl or 2-methoxy-5-trifluoromethyl-phenyl.

7. Product according to claim 1, **characterised in that** it is selected from:
1-[4-(3-amino-1H-pyrazolo[3,4-b]pyridin-4-yl)-phenyl]-3-(2-fluoro-5-trifluoromethyl-phenyl)-urea,
1-[5-(3-amino-1H-pyrazolo[3,4-b]pyridin-4-yl)-pyridin-2-yl]-3-(2-fluoro-5-trifluoromethylphenyl)-urea,
1-[4-(3-amino-1H-pyrazolo[3,4-b]pyridin-4-yl)-phenyl]-3-(2-methoxy-5-trifluoromethyl-phenyl)-urea,
1-[5-(3-amino-1H-pyrazolo[3,4-b]pyridin-4-yl)-pyridin-2-yl]-3-(2-methoxy-5-trifluoromethylphenyl)-urea,
1-[4-(3-amino-1H-pyrazolo[3,4-b]pyridin-4-yl)-phenyl]-3-(2-methylcarbonylamino-5-trifluoromethyl-phenyl)-urea,
1-[5-(3-amino-1H-pyrazolo[3,4-b]pyridin-4-yl)-pyridin-2-yl]-3-(2-methylcarbonylamino-5-trifluoromethyl-phenyl)-urea,
1-[4-(3-amino-1H-pyrazolo[3,4-b]pyridin-4-yl)-phenyl]-3-(2-ethoxy-5-trifluoromethyl-phenyl)-urea,
1-[4-(3-amino-1H-pyrazolo[3,4-b]pyridin-4-yl)-phenyl]-3-(2-fluoro-5-methyl-phenyl)-urea,
3-{3-[4-(3-amino-1*H*-pyrazolo[3,4-b]pyridin-4-yl)-phenyl]-ureido}-4-methoxy-benzoic acid,
1-[4-(3-amino-1H-pyrazolo[3,4-b]pyridin-4-yl)-phenyl]-3-(2-hydroxy-5-trifluoromethyl-phenyl)-urea,
1-[4-(3-amino-1H-pyrazolo[3,4-b]pyridin-4-yl)-phenyl]-3-(3,4-dimethyl-phenyl)-urea,
1-[4-(3-amino-1H-pyrazolo[3,4-b]pyridin-4-yl)-phenyl]-3-(2-methoxy-5-terbutyl-phenyl)-urea,
1-[4-(3-amino-1H-pyrazolo[3,4-b]pyridin-4-yl)-phenyl]-3-(3-trifluoromethyl-4-methyl-phenyl)-urea,
1-[4-(3-amino-1H-pyrazolo[3,4-b]pyridin-4-yl)-phenyl]-3-(3-chloro-4-methyl-phenyl)-urea,
1-[4-(3-amino-1H-pyrazolo[3,4-b]pyridin-4-yl)-phenyl]-3-(3-ethyl-phenyl)-urea,
1-[4-(3-amino-1H-pyrazolo[3,4-b]pyridin-4-yl)-phenyl]-3-1,3-benzodioxol-5-yl-urea,
1-[4-(3-amino-1H-pyrazolo[3,4-b]pyridin-4-yl)-phenyl]-3-(3-chloro-4-methoxy-phenyl)-urea,
1-[4-(3-amino-1H-pyrazolo[3,4-b]pyridin-4-yl)-phenyl]-3-(2-methoxy-5-chloro-phenyl)-urea,
1-[4-(3-amino-1H-pyrazolo[3,4-b]pyridin-4-yl)-phenyl]-3-(2-ethoxy-phenyl)-urea,
1-[4-(3-amino-1H-pyrazolo[3,4-b]pyridin-4-yl)-phenyl]-3-(2-methoxy-5-methyl-phenyl)-urea,
N-[4-(3-amino-1H-pyrazolo[3,4-b]pyridin-4-yl)-phenyl]-2,3-dichloro-benzenesulphonamide.

8. Product according to claim 1, **characterised in that** it is selected from:
1-[4-(3-amino-1H-pyrazolo[4,3-c]pyridin-4-yl)-phenyl]-3-(2-fluoro-5-trifluoromethyl-phenyl)-urea,
1-[5-(3-amino-1H-pyrazolo[4,3-c]pyridin-4-yl)-pyridin-2-yl]-3-(2-fluoro-5-trifluoromethylphenyl)-urea,
1-[4-(3-amino-1H-pyrazolo[4,3-c]pyridin-4-yl)-phenyl]-3-(phenyl)-urea,
1-[5-(3-amino-1H-pyrazolo[4,3-c]pyridin-4-yl)-pyridin-2-yl]-3-(2-methoxy-5-trifluoromethylphenyl)-urea,
1-[4-(3-amino-1H-pyrazolo[4,3-c]pyridin-4-yl)-phenyl]-3-(2-methoxy-5-trifluoromethyl-phenyl)-urea,
1-[4-(3-amino-1H-pyrazolo[4,3-c]pyridin-4-yl)-phenyl]-3-(2-methylcarbonylamino-5-trifluoromethyl-phenyl)-urea,
1-[4-(3-amino-1H-pyrazolo[4,3-c]pyridin-4-yl)-phenyl]-3-(2-fluoro-5-methyl-phenyl)-urea,
1-[4-(3-amino-1H-pyrazolo[4,3-c]pyridin-4-yl)-phenyl]-3-(2,4-dimethoxy-phenyl)-urea,
N-[4-(3-amino-1H-pyrazolo[4,3-c]pyridin-4-yl)-phenyl]-2,3-dichloro-benzenesulphonamide,
1-[4-(3-amino-1H-pyrazolo[4,3-c]pyridin-4-yl)-phenyl]-3-(2-methoxy-5-terbutyl-phenyl)-urea,
1-[4-(3-amino-1H-pyrazolo[4,3-c]pyridin-4-yl)-phenyl]-3-(3,4-dimethyl-phenyl)-urea,
1-[4-(3-amino-1H-pyrazolo[4,3-c]pyridin-4-yl)-phenyl]-3-(3-trifluoromethyl-4-methyl-phenyl)-urea,
1-[4-(3-amino-1H-pyrazolo[4, 3-c]pyridin-4-yl)-phenyl]-3-(2, 5-difluoro-phenyl)-urea,
1-[4-(3-amino-1H-pyrazolo[4,3-c]pyridin-4-yl)-phenyl]-3-(3-chloro-4-methyl-phenyl)-urea,
1-[4-(3-amino-1H-pyrazolo[4,3-c]pyridin-4-yl)-phenyl]-3-(2-methyl-5-fluoro-phenyl)-urea,
1-[4-(3-amino-1H-pyrazolo[4,3-c]pyridin-4-yl)-phenyl]-3-(2,4-dimethoxy-5-chloro-phenyl)-urea,
1-[4-(3-amino-1H-pyrazolo[4,3-c]pyridin-4-yl)-phenyl]-3-(3-trifluoromethylsulphanyl-phenyl)-urea,

9. Product according to claim 1, **characterised in that** it is selected from:
1-[4-(3-amino-1H-pyrazolo[3,4-c]pyridin-4-yl)-phenyl]-3-(2-fluoro-5-trifluoromethyl-phenyl)-urea,
1-[4-(3-amino-1H-pyrazolo[3,4-c]pyridin-4-yl)-phenyl]-3-(2-methoxy-5-trifluoromethyl-phenyl)-urea,
N-[4-(3-amino-1H-pyrazolo[3,4-c]pyridin-4-yl)-phenyl]-2,3-dichloro-benzenesulphonamide.

10. Product according to any one of the preceding claims, **characterised in that** it is in:
a. a non-chiral form, or
b. a racemic form, or
c. a form enriched in one stereoisomer, or
d. a form enriched in one enantiomer;
and **in that** it is optionally salified.

11. Drug, **characterised in that** it comprises a product of formula (I) according to any one of claims 1 to 9, or an addition salt of said compound with a pharmaceutically acceptable acid, or alternatively a hydrate or solvate of the product of formula (I).

12. Pharmaceutical composition comprising a product according to any one of the preceding claims, in combination with a pharmaceutically acceptable excipient.

13. Use of a product according to any one of claims 1 to 9 for the preparation of a drug as an agent inhibiting a reaction catalysed by a kinase.

14. Use of a product according to claim 13, **characterised in that** the kinase is selected from FAK, KDR and Tie2.

15. Use of a product according to any one of claims 1 to 9, for manufacturing a drug which can be used to treat a pathological condition.

16. Use according to 15, **characterised in that** the pathological condition is cancer.

## Patentansprüche

1. Produkt entsprechend der folgenden Formel (I): 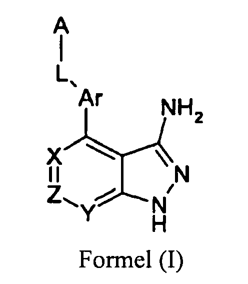 in der:
1) Ar-L-A 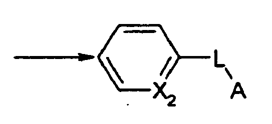 bedeutet, in der X₂ aus N, C-CH₃, CF und CH ausgewählt ist,
2) A ausgewählt ist aus Phenyl, Pyrazolyl und Isoxazolyl;
die gegebenenfalls durch einen oder mehrere Substituenten substituiert sind, ausgewählt aus Halogen; (C₁-C₁₂)-Alkyl; (C₆-C₁₄)-Aryl; (C₁-C₁₃)-Heteroaryl mit 1 bis 4 Heteroatomen, (C₃-C₁₂)-Cycloalkyl; (C₁-C₁₃)-Heterocyclyl mit 1 bis 4 Heteroatomen; (C₂-C₁₂)-Alkylen; (C₂-C₁₂)-Alkinyl; OH; O-(C₁-C₁₂)-Alkyl; O-(C₂-C₁₂)-Alkylen; O-(C₆-C₁₄)-Aryl; O-(C₁-C₁₃)-Heteroaryl mit 1 bis 4 Heteroatomen; NH₂, NH-(C₁-C₁₂)-Alkyl; NH-(C₆-C₁₄)-Aryl; NH-(C₁-C₁₃)-Heteroaryl; N-(C₁-C₁₂)-Alkyl-(C₁-C₁₂)-alkyl'; SH; S-(C1₋C₁₂)-Alkyl; S-(C₆-C₁₄)-Aryl; S(O₂)H; S(O₂)-(C₁-C₁₂)-Alkyl; S(O₂)-(C₆-C₁₄)-Aryl; SO₃H; SO₃-(C₁-C₁₂)-Alkyl; SO₃-(C₆-C₁₄)-Aryl; CHO; C(O)-(C₁-C₁₂)-Alkyl; C(O)-(C₆-C₁₄)-Aryl; C(O)OH; C(O)O-(C₁-C₁₂)-Alkyl, C(O)O-(C₆-C₁₄)-Aryl; OC(O)-(C₁-C₁₂)-Alkyl; OC(O)-(C₆-C₁₄)-Aryl; C(O)NH₂; C(O)NH-(C₁-C₁₂)-Alkyl; C(O)NH-(C₆-C₁₄)-Aryl; NHCHO; NHC(O)-(C₁-C₁₂)-Alkyl; NHC(O)-(C₆-C₁₄)-Aryl; NH-(C₃-C₁₂)-Cycloalkyl; NH-(C₁-C₁₃)-Heterocyclyl mit 1 bis 4 Heteroatomen, CONH-(C₁-C₁₃)-Heterocyclyl mit 1 bis 4 Heteroatomen, CO-(C₁-C₁₃)-Heteroaryl mit 1 bis 4 Heteroatomen, CO-(C₁-C₁₃)-Heterocyclyl mit 1 bis 4 Heteroatomen, NHCO-(C₁-C₁₃)-Heteroaryl mit 1 bis 4 Heteroatomen, NHCO-(C₁-C₁₃)-Heterocyclyl mit 1 bis 4 Heteroatomen, NHCONH-(C₁-C₁₂)-Alkyl;
3) L aus der Gruppe ausgewählt ist, die durch : NH-SO₂ -NH-CO-NH gebildet ist;
4) einer der Reste X, Y und Z ausgewählt ist aus N und NO und die beiden anderen Reste Z, Y und X C(R₅) und C(R₆) bedeuten;
5) R₅ und R₆ aus H und F ausgewählt sind.

2. Produkt nach Anspruch 1, **dadurch gekennzeichnet, dass** Ar-L-A: 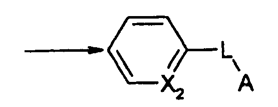 bedeutet, in der X₂ N darstellt.

3. Produkt nach Anspruch 1, **dadurch gekennzeichnet, dass** L-A NH-CO-NH-A bedeutet.

4. Produkt nach einem Ansprüche 1 bis 3, **dadurch gekennzeichnet, dass** A Phenyl oder Isoxazolyl bedeutet, substituiert durch Halogen (C₁-C₄)-Alkyl, halogeniertes (C₁-C₃)-Alkyl, O-(C₁-C₄)-Alkyl, S-(C₁-C₄)-Alkyl, halogeniertes O-(C₁-C₄)-Alkyl, halogeniertes S-(C₁-C₄)-Alkyl, wobei dann, wenn A disubstituiert ist, die beiden Substituenten von A einen Ring mit 5 bis 7 Kettengliedern bilden können, der 0 bis 3 Heteroatome enthält.

5. Produkt nach einem der Ansprüche 1 bis 3, **dadurch gekennzeichnet, dass** A durch einen oder mehrere identische oder verschiedenartige Substituenten substituiert ist, die unabhängig voneinander aus der Gruppe ausgewählt sind, die gebildet wird durch F, Cl, Br, I, OH, SH, SO₃M, COOM, CN, NO₂, CON(R₈)(R₉), N(R₈)CO(R₉), (C₁-C₃)-Alkyl-OH, (C₁-C₃)-Alkyl-N(R₈)(R₉), (C₁-C₃)-Alkyl-(R₁₀), (C₁-C₃)-Alkyl-COOH, N(R₈)(R₉), (C₁-C₆)-Alkyl, (C₂-C₆)-Alkylen, (C₂-C₆)-Alkinyl, Aryl, Heteroaryl, O-(C₁-C₆)-Alkyl, O-Aryl, O-Heteroaryl, S-(C₁-C₆)-Alkyl, S-Aryl, S-Heteroaryl, jeweils gegebenenfalls substituiert durch einen oder mehrere Substituenten ausgewählt aus (C₁-C₃)-Alkyl, Halogen, O-(C₁-C₃)-Alkyl; worin R₈ und R₉ unabhängig voneinander ausgewählt sind aus H, (C₁-C₃)-Alkyl, (C₁-C₃)-Alkyl-OH, (C₁-C₃)-Alkyl-NH₂, (C₁-C₃)-Alkyl-COOM, (C₁-C₃)-Alkyl-SO₃M; wobei dann, wenn R₈ und R₉ gleichzeitig von H verschieden sind, sie verbunden sein können zur Bildung eines Rings mit 5 bis 7 Kettengliedern, der 0 bis 3 Heteroatome ausgewählt aus O, N und S aufweist; worin M H oder ein Alkalimetallkation ausgewählt aus Li, Na und K bedeutet; und worin R₁₀ H bedeutet oder einen nichtaromatischen, gegebenenfalls substituierten Heterocyclus bedeutet, der 2 bis 7 Kohlenstoffatome und 1 bis 3 Heteroatome ausgewählt aus N, O und S aufweist.

6. Produkt nach Anspruch 1, **dadurch gekennzeichnet, dass** A 2-Fluor-5-trifluormethyl-phenyl oder 2-Methoxy-5-trifluormethyl-phenyl bedeutet.

7. Produkt nach Anspruch 1, **dadurch gekennzeichnet, dass** es ausgewählt ist aus:
1-[4-(3-Amino-1H-pyrazolo[3,4-b]pyridin-4-yl)-phenyl]-3-(2-fluor-5-trifluormethylphenyl)-harnstoff,
1-[S-(3-Amino-1H-pyrazolo[3,4-b]pyridin-4-yl)-pyridin-2-yl]-3-(2-fluor-5-trifluormethylphenyl)-harnstoff,
1-[4-(3-Amino-1H-pyrazolo[3,4-b]pyridin-4-yl)-phenyl]-3-(2-methoxy-5-trifluormethylphenyl)-harnstoff,
1-[5-(3-Amino-1H-pyrazolo [3,4-b]pyridin-4-yl)-pyridin-2-yl]-3-(2-methoxy-5-trifluormethyl-phenyl)-harnstoff,
1-[4-(3-Amino-1H-pyrazolo[3,4-b]pyridin-4-yl)-phenyl]-3-(2-methylcarbonylamino-5-trifluormethyl-phenyl)-harnstoff,
1-[5-(3-Amino-1H-pyrazolo[3,4-b]pyridin-4-yl)-pyridin-2-yl]-3-(2-methylcarbonylamino-5-trifluormethyl-phenyl)-harnstoff,
1-[4-(3-Amino-1H-pyrazolo[3,4-b]pyridin-4-yl)-phenyl]-3-(2-ethoxy-5-trifluormethylphenyl)-harnstoff,
1-[4-(3-Amino-1H-pyrazolo[3,4-b]pyridin-4-yl)-phenyl]-3-(2-fluor-5-methyl-phenyl)-harnstoff,
3-{3-[4-(3-Amino-1*H*-pyrazolo[3,4-b]pyridin-4-yl)-phenyl}-ureido}-4-methoxybenzoesäure,
1-[4-(3-Amino-1H-pyrazolo[3,4-b]pyridin-4-yl)-phenyl]-3-(2-hydroxy-5-trifluormethylphenyl)-harnstoff,
1-[4-(3-Amino-1H-pyrazolo[3,4-b]pyridin-4-yl)-phenyl]-3-(3,4-dimethyl-phenyl)-harnstoff,
1-[4-(3-Amino-1H-pyrazolo[3,4-b]pyridin-4-yl)-phenyl]-3-(2-methoxy-5-tert.-butylphenyl)-harnstoff,
1-[4-(3-Amino-1H-pyrazolo[3,4-b]pyridin-4-yl)-phenyl]-3-(3-trifluormethyl-4-methylphenyl)-harnstoff,
1-[4-(3-Amino-1H-pyrazolo[3,4-b]pyridin-4-yl)-phenyl]-3-(3-chlor-4-methyl-phenyl)-harnstoff,
1-[4-(3-Amino-1H-pyrazolo[3,4-b]pyridin-4-yl)-phenyl]-3-(3-ethyl-phenyl)-harnstoff,
1-[4-(3-Amino-1H-pyrazolo[3,4-b]pyridin-4-yl)-phenyl]-3-1,3-benzodioxol-5-yl-harnstoff,
1-[4-(3-Amino-1H-pyrazolo[3,4-b]pyridin-4-yl)-phenyl]-3-(3-chlor-4-methoxy-phenyl)-harnstoff,
1-[4-(3-Amino-1H-pyrazolo[3,4-b]pyridin-4-yl)-phenyl]-3-(2-methoxy-5-chlor-phenyl)-harnstoff,
1-[4-(3-Amino-1H-pyrazolo[3,4-b]pyridin-4-yl)-phenyl]-3-(2-ethoxy-phenyl)-harnstoff,
1-[4-(3-Amino-1H-pyrazolo[3,4-b]pyridin-4-yl)-phenyl]-3-(2-methoxy-5-methyl-phenyl)-harnstoff,
N-[4-(3-Amino-1H-pyrazolo[3,4-b]pyridin-4-yl)-phenyl]-2,3-dichlor-benzolsulfonamid.

8. Produkt nach Anspruch 1, **dadurch gekennzeichnet, dass** es ausgewählt ist aus:
1-[4-(3-Amino-1H-pyrazolo[4,3-c]pyridin-4-yl)-phenyl]-3-(2-fluor-5-trifluormethylphenyl)-harnstoff,
1-[5-(3-Amino-1H-pyrazolo[4,3-c]pyridin-4-yl)-pyridin-2-yl]-3-(2-fluor-5-trifluormethylphenyl)-harnstoff,
1-[4-(3-Amino-1H-pyrazolo[4,3-c]pyridin-4-yl)-phenyl]-3-(phenyl)-harnstoff,
1-[5-(3-Amino-1H-pyrazolo[4,3-c]pyridin-4-yl)-pyridin-2-yl]-3-(2-methoxy-5-trifluormethyl-phenyl)-harnstoff,
1-[4-(3-Amino-1H-pyrazolo[4,3-c]pyridin-4-yl)-phenyl]-3-(2-methoxy-5-trifluormethylphenyl)-harnstoff,
1-[4-(3-Amino-1H-pyrazolo[4,3-c]pyridin-4-yl)-phenyl]-3-(2-methylcarbonylamino-5-trifluormethyl-phenyl)-harnstoff,
1-[4-(3-Amino-1H-pyrazolo[4,3-c]pyridin-4-yl)-phenyl]-3-(2-fluor-5-methyl-phenyl)-harnstoff,
1-[4-(3-Amino-1H-pyrazolo[4,3-c]pyridin-4-yl)-phenyl]-3-(2,4-dimethoxy-phenyl)-harnstoff,
1-[4-(3-Amino-1H-pyrazolo[4,3-c]pyridin-4-yl)-phenyl]-2,3-dichlorbenzolsulfonamid,
1-[4-(3-Amino-1H-pyrazolo[4,3-c]pyridin-4-yl)-phenyl]-3-(2-methoxy-5-tert.-butylphenyl)-harnstoff,
1-[4-(3-Amino-1H-pyrazolo[4,3-c]pyridin-4-yl)-phenyl]-3-(3,4-dimethyl-phenyl)-harnstoff,
1-[4-(3-Amino-1H-pyrazolo[4,3-c]pyridin-4-yl)-phenyl]-3-(3-trifluormethyl-4-methylphenyl)-harnstoff,
1-[4-(3-Amino-1H-pyrazolo[4,3-c]pyridin-4-yl)-phenyl]-3-(2,5-difluor-phenyl)-harnstoff,
1-[4-(3-Amino-1H-pyrazolo[4,3-c]pyridin-4-yl)-phenyl]-3-(3-chlor-4-methyl-phenyl)-harnstoff,
1-[4-(3-Amino-1H-pyrazolo[4,3-c]pyridin-4-yl)-phenyl]-3-(2-methyl-5-fluor-phenyl)-harnstoff,
1-[4-(3-Amino-1H-pyrazolo[4,3-c]pyridin-4-yl)-phenyl]-3-(2,4-dimethoxy-5-chlorphenyl)-harnstoff,
1-[4-(3-Amino-1H-pyrazolo[4,3-c]pyridin-4-yl)-phenyl]-3-(3-trifluormethylsulfanyl-phenyl)-harnstoff.

9. Produkt nach Anspruch 1, **dadurch gekennzeichnet, dass** es ausgewählt ist aus:
1-[4-(3-Amino-1H-pyrazolo[3,4-c]pyridin-4-yl)-phenyl]-3-(2-fluor-5-trifluormethylphenyl)-harnstoff,
1-[4-(3-Amino-1H-pyrazolo[3,4-c]pyridin-4-yl)-phenyl]-3-(2-methoxy-5-trifluormethylphenyl)-harnstoff,
N-[4-(3-Amino-1H-pyrazolo[3,4-c]pyridin-4-yl)-phenyl]-2,3-dichlorbenzolsulfonamid.

10. Produkt nach einem der vorhergehenden Ansprüche, **dadurch gekennzeichnet, dass** es:
a. in nichtchiraler Form oder
b. in racemischer Form oder
c. in an einem Stereoisomeren angereicherter Form oder
d. in an einem Enantiomeren angereicherter Form
vorliegt;
und dass es gegebenenfalls in Form eines Salzes vorliegt.

11. Arzneimittel, **dadurch gekennzeichnet, dass** es ein Produkt der Formel (I) gemäß einem der Ansprüche 1 bis 9 oder ein Additionssalz dieser Verbindung mit einer pharmazeutisch annehmbaren Säure oder ein Hydrat oder ein Solvat des Produkts der Formel (I) umfasst.

12. Pharmazeutische Zubereitung, umfassend ein Produkt nach einem der vorhergehenden Ansprüche in Kombination mit einem pharmazeutisch annehmbaren Hilfsstoff.

13. Verwendung eines Produkts nach einem der Ansprüche 1 bis 9 für die Herstellung eines Arzneimittels, das als Inhibitor einer durch eine Kinase katalysierten Reaktion wirkt.

14. Verwendung eines Produkts nach Anspruch 13, **dadurch gekennzeichnet, dass** die Kinase aus FAK, KDR und Tie2 ausgewählt ist.

15. Verwendung eines Produkts nach einem der Ansprüche 1 bis 9 für die Herstellung eines Arzneimittels zur Behandlung eines pathologischen Zustands.

16. Verwendung nach Anspruch 15, **dadurch gekennzeichnet, dass** der pathologische Zustand Krebs ist.
